(19) Europäisches Patentamt
European Patent Office
Office européen des brevets

(11) EP 2 809 440 B1

(12) **FASCICULE DE BREVET EUROPEEN**

(45) Date de publication et mention de la délivrance du brevet:
**04.04.2018 Bulletin 2018/14**

(21) Numéro de dépôt: **13701673.9**

(22) Date de dépôt: **31.01.2013**

(51) Int Cl.:
***B01J 13/22*** *(2006.01)* ***B01J 13/08*** *(2006.01)*
***B01J 13/10*** *(2006.01)*

(86) Numéro de dépôt international:
**PCT/EP2013/051976**

(87) Numéro de publication internationale:
**WO 2013/113855 (08.08.2013 Gazette 2013/32)**

(54) **CAPSULES CONTENANT DES CELLULES DE MAMMIFÈRES**

KAPSELN MIT SÄUGETIERZELLEN

CAPSULES CONTAINING MAMMALIAN CELLS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.01.2012 FR 1250875**

(43) Date de publication de la demande:
**10.12.2014 Bulletin 2014/50**

(73) Titulaire: **Capsum**
**13013 Marseille (FR)**

(72) Inventeurs:
- **BIBETTE, Jérôme**
**F-75005 Paris (FR)**
- **ATRUX-TALLAU, Nicolas**
**F-06600 Antibes (FR)**
- **DOMÉJEAN, Hugo**
**F-75013 Paris (FR)**
- **FUNFAK, Annette**
**F-75005 Paris (FR)**
- **BREMOND, Nicolas**
**F-75005 Paris (FR)**
- **NASSOY, Pierre**
**F-33140 Villenave d'Ornon (FR)**
- **ALLESSANDRI, Kévin**
**CH-1204 Genève (CH)**

(74) Mandataire: **Lavoix**
**2, place d'Estienne d'Orves**
**75441 Paris Cedex 09 (FR)**

(56) Documents cités:
**WO-A1-92/05778** **FR-A1- 2 955 257**
**GB-A- 2 145 992** **US-A- 4 409 331**

- **DATABASE WPI Week 201145 Thomson Scientific, London, GB; AN 2011-H11466 XP002712352, & WO 2011/072557 A1 (CAS DALIAN CHEM&PHYSICAL INST) 23 juin 2011 (2011-06-23)**
- **NICOLAS BREMOND ET AL: "Formation of liquid-core capsules having a thin hydrogel membrane: liquid pearls", SOFT MATTER, vol. 6, no. 11, 1 janvier 2010 (2010-01-01), page 2484, XP055072164, ISSN: 1744-683X, DOI: 10.1039/b923783f**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 809 440 B1

## Description

La présente invention concerne une capsule permettant la croissance d'au moins une cellule de mammifères ladite capsule étant formée d'un coeur liquide et d'au moins une enveloppe externe encapsulant totalement le coeur liquide à sa périphérie, le procédé de préparation d'une telle capsule, une méthode de criblage de principes actifs ainsi qu'une méthode de culture utilisant de telles capsules.

Chez tous les organismes multicellulaires, les cellules se trouvent de manière intrinsèque dans un environnement tridimensionnel (3D) représenté par les cellules avoisinantes et la matrice extracellulaire (MEC). Même si la vaste majorité des études de biologie cellulaire *in vitro* sont toujours réalisées en utilisant des monocouches cellulaires cultivées sur des substrats plats, de plus en plus de données démontrent que les cultures cellulaires bidimensionnelles (2D) ne parviennent pas à reproduire l'architecture des tissus vivants et, en conséquence, peuvent biaiser la réponse cellulaire aux signaux externes ou aux signaux intégrés dans la cellule. En particulier, on pense que la mauvaise prédictibilité de la réponse tissulaire aux nouveaux agents cosmétiques ou thérapeutiques qui ont été évalués sur des monocouches cellulaires est due à l'absence de propriétés spécifiques aux tissus des cultures en 2D. Depuis les années 80, les sphéroïdes multicellulaires (MCS) ont été proposés en tant que modèles de microtumeurs solides avasculaires *in vitro* en 3D, et des MCS incorporés dans ou déposés sur des gels de type MEC ont en outre été utilisés pour étudier les mécanismes de l'invasion cellulaire. En recherche biomédicale, ces essais à base de MCS représentent aujourd'hui une alternative prometteuse qui surmonte les limites des cultures cellulaires en 2D et évite ainsi de recourir à des tests systématiques chez l'animal, à condition que des protocoles de préparation standardisés soient disponibles et compatibles avec des plates-formes de tests thérapeutiques. En parallèle, les progrès réalisés en génie biologique et particulièrement en génie tissulaire, permettent de reconstruire, *in vitro,* une grande variété de tissus biologiques normaux, contenant un ou plusieurs types de cellules. A titre d'exemple, la reconstruction de tissu cutané est réalisée couramment et avec succès à des fins thérapeutiques notamment pour la greffe de peau dans le traitement des grands brûlés ou de plaies chroniques. Ces peaux reconstruites sont également utilisées dans la recherche cosmétique comme méthode alternative à l'expérimentation animale, mais aussi en recherche dermo-pharmaceutique. L'utilisation de tissus reconstruits représente un modèle de choix car les cellules sont dans un environnement similaire à l'environnement rencontré *in vivo,* les réponses biologiques seront donc cohérentes avec les réponses *in situ.* Cependant, la préparation de ces tissus biologiques ainsi que leur manipulation restent chronophage et complexe. Des avancées significatives ont déjà permis de concevoir une MEC reconstituée ou synthétique basée sur l'auto-assemblage de réseaux fibrillaires constitués de polymères fabriqués sur mesure. La formation de MCS est toujours généralement réalisée en utilisant des méthodes conventionnelles comme la technique de la goutte suspendue, la rotation giratoire ou les cultures sur couche liquide, dont les principaux inconvénients sont leur faible rendement et la difficulté à contrôler la taille des agrégats cellulaires. L'avènement de la photolithographie à l'échelle microscopique a récemment conduit à diverses tentatives sophistiquées en matière de production de MCS automatisée à l'aide de microréseaux, micropuits ou dispositifs microfluidiques. En revanche, ces techniques émergentes n'ont pas évolué au-delà de la preuve de concept, probablement en raison des problèmes de praticabilité et de la difficulté d'obtention de conditions de culture cellulaire délicates. Néanmoins, ces techniques concernent des capsules de très petites tailles de l'ordre de la dizaine de microns et restent couteuses. De plus, les capsules décrites par l'art antérieur ne permettent généralement pas une production rapide et massive (de type industriel) de coques creuses, perméables et élastiques, contenant des cellules et ayant une taille contrôlée.

Par ailleurs, l'art antérieur décrit des billes contenant des cellules, il s'agit de structures sphériques constituées d'un coeur solide (sphère pleine). L'absence de coeur liquide réduit le champ d'application de ces billes puisque les cellules sont dans un enchevêtrement de polymère et sont séparées les unes des autres par le polymère. Ainsi, ces cellules ne peuvent que coloniser l'enchevêtrement et ne peuvent donc s'organiser sous une autre forme, par exemple, former une structure de type épithéliale pluristratifiée.

Le demandeur a décrit des capsules susceptibles de contenir des cellules (FR 2 939 012). Néanmoins, la demande ne mentionne pas que les capsules sont adaptées pour l'encapsulation et la culture de cellules eucaryotes de mammifère qui du fait de leur plus grande fragilité, sont des cellules difficiles à cultiver. US 4 409 331 A décrit un procédé de préparation d'une capsule caractérisé en ce qu'il comprend les étapes de 1) formation d'une goutte liquide comprenant une solution aqueuse comprenant une cellule mammifère et une gomme polysaccharidique ; 2) gélification par immersion de ladite goutte liquide dans une solution comprenant des cations multivalents ; 3) réticulation des couches de surface et resolubilisation du gel interne. Ce document décrit aussi le résultat de ce procédé, c'est-à-dire une capsule comprenant un coeur liquide et une enveloppe externe comprenant une gomme polysaccharidique gélifiée, ladite capsule étant caractérisée en ce qu'elle comprend une cellule mammifère. Dans ce contexte, l'objectif principal de l'invention consiste à développer une nouvelle méthode, simple et reproductible, pour obtenir une préparation à haut débit de capsules ayant une taille contrôlée, dont la paroi périphérique soit biocompatible, et suffisamment perméable pour permettre le passage de nutriments et dont les propriétés élastiques ne provoquent pas de stress cellulaire susceptible d'inhiber la croissance des cellules eucaryotes de mammifère. L'objectif de l'invention consiste à développer une nouvelle méthode, simple et reproductible, pour obtenir une préparation à haut débit de capsules compartimentées ou non

permettant un choix de culture de cellules eucaryotes de mammifère en suspension et/ou en sphéroïde et/ou en tissu et notamment, permettant une culture tridimensionnelle des cellules eucaryotes de mammifère et donc la mise en place de modèles de tissus, des modèles d'interaction entre plusieurs types cellulaires, des modèles de barrières biologiques telles que la jonction dermo-épidermique.

L'invention concerne une capsule comprenant un coeur liquide, et au moins une enveloppe externe encapsulant totalement le coeur liquide à sa périphérie, ladite enveloppe externe étant propre à retenir le coeur liquide lorsque la capsule est plongée dans un gaz et comprenant au moins un polyélectrolyte gélifié et/ou un biopolymère rigidifié, ladite capsule comprenant au moins une cellule eucaryote de mammifère.

Les capsules selon l'invention sont de préparation simple et efficace, et permettent la culture à long terme de cellules eucaryotes ainsi que l'obtention de modèles de culture tridimensionnelle pour notamment, le criblage à haut débit dans des environnements 3D physiologiquement pertinents.

En effet, les inventeurs ont démontré que les capsules selon l'invention permettent une bonne croissance des cellules de mammifères en dépit du confinement lié à l'encapsulation. Ainsi, les caractéristiques élastiques de la capsule sont suffisantes pour permettre la déformation de l'enveloppe pour que la culture cellulaire arrive à confluence tout en conservant son intégrité, évitant ainsi l'éclatement de la capsule.

De plus les inventeurs ont montré que la capsule permet une adhésion des cellules améliorant ainsi leur croissance et leur survie.

Les inventeurs ont démontrés par ailleurs, que ces capsules permettent de créer des modèles de tissus tels que des modèles de peau, d'épiderme ou de derme.

L'invention concerne également un procédé de préparation d'une capsule comprenant les étapes suivantes de :

a) formation d'une goutte liquide multi-composante comprenant :

- un coeur liquide,
- une enveloppe intermédiaire liquide formée d'une composition aqueuse comprenant au moins un biopolymère, encapsulant totalement à sa périphérie le coeur liquide, et
- une enveloppe externe liquide formée d'une composition aqueuse, différente de la composition intermédiaire, ladite composition aqueuse comprenant au moins un polyélectrolyte et au moins un tensio-actif, ladite enveloppe externe liquide encapsulant totalement à sa périphérie l'enveloppe intermédiaire,

le coeur liquide et/ou l'enveloppe intermédiaire liquide comprenant au moins une cellule eucaryote, préférentiellement de mammifère

b) gélification par immersion de ladite goutte liquide multi-composante dans une solution gélifiante contenant un réactif propre à gélifier le polyélectrolyte de l'enveloppe externe liquide, pour obtenir une capsule gélifiée comprenant une enveloppe externe gélifiée,
c) rigidification de la composition intermédiaire de l'enveloppe intermédiaire liquide, pour obtenir une capsule gélifiée et rigidifiée comprenant une enveloppe intermédiaire rigidifiée, et
d) récupération desdites capsules gélifiée et rigidifiée.

L'invention porte également sur une méthode de criblage de principes actifs cosmétiques comprenant :

a) la mise en culture d'une capsule selon l'invention en présence et en absence d'une substance candidate,
b) la détection d'un phénotype d'intérêt dans les cellules de la capsule cultivée en présence de la substance candidate par rapport aux cellules de la capsule cultivée en absence de la substance candidate, et
c) l'identification de la substance en tant que principe actif si un phénotype d'intérêt a été détecté.

L'invention concerne de plus, l'utilisation d'une capsule selon l'invention pour la culture *in vitro* de cellules eucaryotes de mammifère.

L'invention concerne en outre, un procédé *in vitro* de culture de cellules eucaryotes de mammifère comprenant les étapes suivantes de :

a) mise en culture d'une capsule selon l'invention dans des conditions suffisantes pour la croissance cellulaire, et
b) récolte de ladite capsule.

La présente invention concerne enfin, un procédé de stockage de cellules eucaryotes de mammifère comprenant la fabrication d'une capsule selon le procédé de l'invention, et une étape de stockage desdites capsules obtenues.

*Procédé de préparation d'une capsule*

La présente invention concerne un procédé de préparation d'une capsule comprenant les étapes suivantes de :

a) formation d'une goutte liquide multi-composante comprenant :

- un coeur liquide,
- une enveloppe intermédiaire liquide formée d'une composition aqueuse ou le cas échant huileuse comprenant au moins un biopolymère, encapsulant totalement à sa périphérie le coeur liquide, et
- une enveloppe externe liquide formée d'une composition aqueuse, différente de la composition intermédiaire, ladite composition aqueuse ou le cas échant huileuse comprenant au moins un polyélectrolyte et au moins un tensio-actif, ladite enveloppe externe liquide encapsulant totalement à sa périphérie l'enveloppe intermédiaire,

le coeur liquide et/ou l'enveloppe intermédiaire liquide comprenant au moins une cellule eucaryote, préférentiellement de mammifère
b) gélification par immersion de ladite goutte liquide multi-composante dans une solution gélifiante contenant un réactif propre à gélifier le polyélectrolyte de l'enveloppe externe liquide, pour obtenir une capsule gélifiée comprenant une enveloppe externe gélifiée,
c) rigidification de la composition intermédiaire de l'enveloppe intermédiaire liquide, pour obtenir une capsule gélifiée et rigidifiée comprenant une enveloppe intermédiaire rigidifiée, et
d) récupération desdites capsules gélifiée et rigidifiée.

Dans le cadre de la présente description, on entend par « **capsule gélifiée** » une capsule comprenant un coeur liquide et une enveloppe gélifiée. Avantageusement, une capsule gélifiée ne comprend pas d'enveloppe rigidifiée, mais peut comprendre une enveloppe intermédiaire liquide. Une capsule gélifiée selon l'invention peut comprendre au moins une cellule eucaryote, préférentiellement de mammifère dans le coeur liquide. Lorsque l'enveloppe gélifiée et le cas échéant l'enveloppe liquide comprennent un biopolymère, elles peuvent inclure indépendamment l'une de l'autre, au moins une cellule eucaryote de mammifère.

Dans le cadre de la présente description, on entend par « **capsule gélifiée et rigidifiée** » une capsule comprenant un coeur liquide, une enveloppe gélifiée et une enveloppe rigidifiée. Avantageusement, l'enveloppe gélifiée encapsule totalement à sa périphérie l'enveloppe rigidifiée, qui encapsule elle-même totalement à sa périphérie le coeur liquide. Une capsule gélifiée et rigidifiée selon l'invention peut comprendre au moins une cellule eucaryote de mammifère dans le coeur liquide et/ou dans l'enveloppe rigidifiée, lorsque l'enveloppe gélifiée comprend un biopolymère.

Dans le cadre de la présente description, on entend par « **composition aqueuse** » une composition ayant la propriété de solubiliser des composés polaires.

Dans le cadre de la présente description, on entend par « **composition huileuse** » une composition ayant la propriété de solubiliser des composés apolaires, tels que des corps gras, des huiles ou des lipides.

Une composition huileuse, encore appelée hydrophobe, est insoluble dans l'eau. Elle comprend de préférence, un corps gras, une huile ou un mélange d'huiles d'origine végétale, animale ou minérale.

A titre d'huile végétale, on peut mentionner par exemple, l'huile d'amande douce, l'huile de jojoba, l'huile de palme ou le phytosqualane.

A titre de corps gras, on peut indiquer par exemple, les esters d'alcools gras et/ou d'acides gras, typiquement en $C_1$-$C_{20}$, tels que le myristate d'isopropyle, le myristate de glycérol, l'isononanoate d'isononyle, les triglycérides d'acide caprylique ou d'acide caprique, le palmitate d'isopropyle et le palmitate d'éthyle. On peut mentionner également les huiles de silicone ou polysiloxanes, tels que les polydiméthylsiloxanes (PDMS).

A titre d'huile animale, on peut indiquer par exemple, le squalène.

A titre d'huile minérale, on peut mentionner par exemple, le polyisobutylène hydrogéné, l'isododécane ou les huiles de paraffine.

Le coeur liquide est généralement constitué d'une composition interne, généralement liquide ou légèrement visqueuse, qui peut être aqueuse ou huileuseLe coeur liquide, destiné à être encapsulé et pouvant contenir au moins une cellule eucaryote de mammifère a préférentiellement une composition permettant la survie cellulaire, la régularisation du pH tel qu'un tampon. Le coeur est généralement liquide ou légèrement visqueux, il est préférentiellement isoosmotique et ne contient pas de composés non compatibles avec la gélification et/ou la rigidification.

L'enveloppe intermédiaire est formée d'une composition intermédiaire aqueuse ou le cas échant huileuse typiquement composée d'un latex de polymères, tel qu'un latex naturel notamment un biopolymère. On entend par « **biopolymère** », un polymère d'origine naturelle et/ou un polymère biocompatible ou un polymère comprenant un fragment d'un tel polymère. Un polymère d'origine naturelle est préférentiellement un polymère naturellement présent chez les eucaryotes mammifères. Un polymère biocompatible est un polymère synthétique identique à un polymère

d'origine naturelle ou un polymère synthétique qui n'interagit pas de façon négative avec les cellules et permettent leur survie. Un biopolymère peut comprendre des protéines et/ou des polysaccharides et/ou des acides gras et/ou des acides nucléiques ou leurs fragments. Un biopolymère convenant à l'invention peut être choisi parmi les protéines notamment de la matrice extracellulaire, les protéoglycannes, les glycosaminoglycanes (GAGs), les polysaccharides et leur forme non hydrolysée ou partiellement hydrolysée. Les protéines notamment de la matrice extracellulaire et leur forme non hydrolysée ou partiellement hydrolysée sont choisies parmi les collagènes, la gélatine, la fibronectine, l'élastine, la poly-L-Lysine, la laminine et leurs dérivés. Les protéoglycannes sont choisis parmi la décorine (chondroïtine-sulfate/dermatane-sulfate), le perlecan (héparane-sulfate) et l'aggrécane et leurs dérivés. Les glycosaminoglycanes sont choisis parmi l'héparine, l'acide hyaluronique, le kératane sulfate, l'héparane sulfate, la chondroïtine sulfate, le dermatane sulfate et leurs dérivés. Les polysaccharides sont choisis parmi l'inuline, le graminane, le lévane, l'amidon, l'amylopectine, l'amylose, la cellulose, le curdlan, les dextrines, le glycogène, le pullulane, le béta-glucane, l'agar-agar, les carraghénanes, la chitine, le chitosane, les mannanes, les xylanes. Préférentiellement, le biopolymère est choisi parmi le collagène, la gélatine, la laminine, l'entractine, au moins un GAG tel que l'héparane sulfate ou leur mélange.

L'enveloppe externe est formée d'une composition aqueuse externe comprend au moins un polyélectrolyte et au moins un tensio-actif. Ledit polyélectrolyte peut être différent ou identique au biopolymère de l'enveloppe intermédiaire

Lorsque le coeur liquide ou l'enveloppe intermédiaire est destiné à contenir des cellules eucaryotes de mammifère, la composition interne, la composition intermédiaire ou la composition externe est adaptée pour permettre la survie cellulaire. Par exemple, elle contient un tampon physiologique tel qu'un tampon Hepes ou un milieu de culture tel que le RPMI, le DMEM ou le MEM. Préférentiellement, la composition interne, la composition intermédiaire ou la composition externe ont un pH entre 7,2 et 7,4. Préférentiellement, le coeur liquide et/ou l'enveloppe intermédiaire comprend $10^3$ à $10^9$ cellules eucaryotes de mammifères/mL. Typiquement, le coeur liquide comprend $10^6$ cellules/mL, la composition intermédiaire $0,75.10^6$ cellules /mL.

En outre, le coeur liquide et/ou l'enveloppe intermédiaire comprennent, 1 à $10^7$ cellules, préférentiellement, 5 à $10^6$, 30 à $5.10^5$, 50 à $10^5$,35 à $5.10^4$, 100 à $10^4$, 150 à $10^4$, 200 à $10^3$ cellules eucaryotes de mammifères.

Selon l'invention, le coeur liquide et/ou l'enveloppe intermédiaire comprend ou ne comprend pas de chélatants ou de phosphates.

Dans le cadre de la présente invention, on entend par « **goutte multi-composante** » une goutte liquide constituée d'au moins un coeur central liquide et d'une enveloppe externe liquide encapsulant totalement à sa périphérie le coeur central liquide. Préférentiellement, la « goutte multi-composante » est une goutte liquide constituée d'un coeur central liquide, d'une enveloppe intermédiaire liquide, encapsulant totalement à sa périphérie le coeur liquide, et d'une enveloppe externe liquide encapsulant totalement à sa périphérie l'enveloppe intermédiaire liquide. Dans cette seconde variante, l'enveloppe intermédiaire est au contact du coeur et de l'enveloppe externe et maintient le coeur hors de contact de l'enveloppe externe. Selon une autre variante, la « goutte multi-composante » peut comprendre plus de deux enveloppes.

Préférentiellement, la composition intermédiaire, à l'interface entre la composition externe et la composition interne, a une viscosité inférieure à celle de la composition externe et supérieure à celle de la composition interne. L'homme du métier sait aisément en fonction de la composition de chacune des couches équilibrer leur viscosité afin d'obtenir de telles variations.

La viscosité peut être mesurée selon l'invention, à l'aide d'un viscosimètre de Brookfield RVT à 20 °C en suivant les indications du constructeur.

*Etape de formation d'une goutte liquide multi-composante*

La production de ce type de goutte est généralement effectuée par co-extrusion de différentes compositions, à savoir la composition interne, le cas échéant, la composition intermédiaire, et la composition externe, telles que définies dans le procédé précité.

Préférentiellement, la composition interne et/ou la composition intermédiaire sont aqueuses ou le cas échant huileuses.

La production de gouttes multi-composante par co-extrusion peut se faire par exemple, par convoyage séparé dans une double ou une triple enveloppe. Le procédé sera expliqué ci-après pour une goutte multi-composante par co-extrusion de trois compositions, néanmoins, le principe reste applicable pour deux compositions ou plus.

Dans le cas d'une triple enveloppe de trois flux : un premier flux constitué de la composition interne, un deuxième flux constitué de la composition intermédiaire et un troisième flux constitué de la composition externe, tel que décrit dans la demande FR 1061404 (figure 6).

A la sortie de la triple enveloppe, les trois flux entrent en contact et se forme alors une goutte multi-composante, selon un mode hydrodynamique dit de « dripping » (goutte-à-goutte, décrit notamment dans WO 2010/063937) ou de « jetting » (formation d'un jet liquide à la sortie de la triple enveloppe, décrit notamment dans FR 10 56925). Le premier flux constitue le coeur liquide, le deuxième flux constitue l'enveloppe intermédiaire liquide et le troisième flux constitue

l'enveloppe externe liquide.

Selon le mode de production, chaque goutte multi-composante se détache de la triple enveloppe et chute dans un volume d'air, avant d'être immergée dans une solution gélifiante S1 contenant un réactif propre à gélifier le polyélectrolyte de l'enveloppe externe liquide, pour former l'enveloppe externe gélifiée des capsules selon l'invention (figure 6).

Selon certaines variantes, les gouttes multi-composantes peuvent comprendre des couches supplémentaires entre l'enveloppe externe et le coeur liquide, autre que l'enveloppe intermédiaire. Ce type de goutte peut être préparé par convoyage séparé de multiples compositions dans des dispositifs à multiples enveloppes.

*Etape de gélification*

Lorsque la goutte multi-composante entre en contact de la solution gélifiante, le réactif propre à gélifier le polyélectrolyte présent dans la solution gélifiante forme alors des liaisons entre les différentes chaînes de polyélectrolyte présentes dans l'enveloppe externe liquide, passant alors à l'état gélifié, provoquant ainsi la gélification de l'enveloppe externe liquide.

Sans vouloir être lié à une théorie particulière, lors du passage à l'état gélifié du polyélectrolyte, les chaînes individuelles de polyélectrolyte présentes dans l'enveloppe externe liquide se raccordent les unes aux autres pour former un réseau réticulé, aussi appelé hydrogel.

Dans le cadre de la présente description, le polyélectrolyte présent dans l'enveloppe externe gélifiée est à l'état gélifié et est également appelé polyélectrolyte à l'état gélifié ou encore polyélectrolyte gélifié.

Une enveloppe externe gélifiée, propre à retenir le coeur de la capsule et le cas échéant, l'ensemble constitué par le coeur et l'enveloppe intermédiaire est ainsi formée. Cette enveloppe externe gélifiée présente une tenue mécanique propre, c'est-à-dire qu'elle est capable d'entourer totalement l'enveloppe intermédiaire et de retenir le coeur encapsulé par cette enveloppe intermédiaire. Ceci a pour effet de maintenir la structure interne du coeur liquide et de l'enveloppe intermédiaire.

De manière générale, l'enveloppe externe gélifiée se présente sous la forme d'une enveloppe monocouche, encapsulant totalement l'enveloppe intermédiaire sa périphérie.

Les capsules selon l'invention séjournent dans la solution gélifiante le temps que l'enveloppe externe soit complètement gélifiée.

Les capsules gélifiées peuvent ensuite éventuellement être collectées et plongées dans une solution aqueuse de rinçage, généralement essentiellement constituée d'eau, d'un tampon physiologique et/ou de milieu de culture afin de rincer les capsules gélifiées formées. Cette étape de rinçage permet d'extraire de l'enveloppe externe gélifiée, un éventuel excès du réactif de la solution gélifiante propre à gélifier, et tout ou partie du tensio-actif (ou d'autres espèces) contenu initialement dans la composition aqueuse externe.

La présence d'un tensio-actif dans la composition aqueuse externe permet d'améliorer la formation et la gélification des gouttes multi-composantes selon le procédé tel que décrit précédemment.

Le polyélectrolyte de l'enveloppe externe gélifiée des capsules selon l'invention est avantageusement choisi parmi les polyélectrolytes réactifs aux ions multivalents.

Dans le cadre de la présente description, on entend par « **polyélectrolyte réactif aux ions multivalents** » un polyélectrolyte susceptible de passer d'un état liquide dans une solution aqueuse à un état gélifié sous l'effet d'un contact avec une solution gélifiante contenant des ions multivalents tels que des ions d'un métal alcalino-terreux choisis par exemple, parmi les ions calcium, baryum ou magnésium.

A l'état liquide, les chaînes individuelles de polyélectrolyte sont sensiblement libres de s'écouler les unes par rapport aux autres. Une solution aqueuse de 2% en masse de polyélectrolyte présente alors un comportement purement visqueux aux gradients de cisaillement caractéristiques du procédé de mise en forme. La viscosité de cette solution à cisaillement nul est entre 50 mPa.s et 10000 mPa.s, avantageusement entre 3000 mPa.s et 7000 mPa.s.

Les chaînes individuelles de polyélectrolyte à l'état liquide présentent avantageusement une masse molaire supérieure à 65 000 g/moles.

Ladite solution gélifiante S1 est par exemple, une solution aqueuse d'un sel de type $X_nM_m$ où X est par exemple, un ion halogénure tel qu'un ion chlorure, bromure, iodure ou fluorure, ou bien encore un ion tartrate, et M est avantageusement un cation multivalent d'un alcalino-terreux tel que le calcium, le magnésium ou le baryum, et n et m sont supérieurs ou égaux à 1.

La concentration en sel de type $X_nM_m$ dans la solution gélifiante est avantageusement comprise de 5 % à 20 % en masse.

A l'état gélifié, les chaînes individuelles de polyélectrolyte forment, avec les ions multivalents, un réseau tridimensionnel cohérent qui retient le coeur et l'enveloppe intermédiaire et empêche son écoulement. Les chaînes individuelles sont retenues les unes par rapport aux autres et ne peuvent pas s'écouler librement les unes par rapport aux autres. De plus le gel a un seuil de contrainte à l'écoulement. Ce seuil de contrainte est supérieur à 0,05 Pa. Le gel possède également un module d'élasticité non-nul et supérieur à 35 kPa.

Le polyélectrolyte est de préférence un polymère biocompatible choisi parmi les polysaccharides, les polyélectrolytes de synthèse à base d'acrylates (polyacrylate de sodium, de lithium, de potassium ou d'ammonium, ou polyacrylamide), ou les polyélectrolytes de synthèse à base de sulfonates (poly(styrène sulfonate) de sodium, par exemple).
De préférence, le polyélectrolyte est choisi parmi les polysaccharides réactifs aux ions multivalents préférentiellement les polysaccharides alimentaires.
Plus particulièrement, le polyélectrolyte est choisi parmi les alginates d'alcalin, tel qu'un alginate de sodium ou un alginate de potassium, les géllanes et les pectines.
Dans le cas où le polyélectrolyte est un alginate de sodium (NaAlg), et où le réactif est le chlorure de calcium, la réaction qui se produit lors de la gélification est la suivante :

$$2NaAlg + CaCl_2 \rightarrow Ca(Alg)_2 + 2NaCl$$

Les alginates sont produits à partir d'algues brunes appelées « laminaires », désignées par le terme anglais « sea weed ».
De préférence, le polyélectrolyte est un alginate d'alcalin ayant avantageusement une teneur en bloc α-L-guluronate supérieure à 50%, notamment supérieure à 55%, voire supérieure à 60%.
Le polyélectrolyte est par exemple, un alginate de sodium.
Selon un mode de réalisation préféré, le pourcentage massique total en polyélectrolyte dans la phase externe gélifiée est compris de 0,5% à 5%, de préférence inférieur à 3%.
Le pourcentage massique total en polyélectrolyte dans la phase externe gélifiée est par exemple, égal à 2%.

*Etape de rigidification*

Au cours du procédé selon l'invention, lorsque la capsule comprend une enveloppe intermédiaire, le procédé comprend en outre, une étape de rigidification de la composition intermédiaire. Les capsules gélifiées obtenues à l'issue de l'étape de gélification, éventuellement rincées, subissent ensuite une étape de rigidification de la composition intermédiaire. Préférablement, l'étape de rigidification est concomitante à celle de gélification. La concomitance de ces deux étapes est d'intérêt afin d'éviter l'écoulement de la composition intermédiaire et son accumulation dans une partie de la capsule et donc la formation d'un enveloppe intermédiaire non uniforme.
Par l'étape de rigidification du procédé de l'invention, le module élastique de l'enveloppe intermédiaire devient non nul.
La composition intermédiaire comprend au moins un biopolymère seul ou en mélange avec un polymère ou un mélange de polymères et/ou un monomère ou un mélange de monomères, éventuellement présents sous forme de dispersion colloïdale.
Dans le cadre de la présente invention, l'enveloppe intermédiaire liquide peut être rigidifiée selon tout mode de rigidification envisageable, comme par exemple, par polymérisation, par précipitation, par agrégation colloïdale ou bien par transition vitreuse généralement provoquée par une variation de température.
Afin d'effectuer cette étape, les capsules gélifiées sont généralement plongées dans un bain de rigidification.
Selon une première variante, le bain de rigidification correspond à la solution gélifiante utilisée lors de l'étape de gélification. Avantageusement, les capsules sont gélifiées puis rigidifiées dans la solution de gélification.
Selon une autre variante, le bain de rigidification est différent de la solution gélifiante, et il est donc généralement nécessaire de collecter les capsules gélifiées, éventuellement de les rincer, puis de les immerger dans le bain de rigidification pour effectuer l'étape de rigidification.
L'étape de rigidification est typiquement effectuée par coacervation de la composition intermédiaire de l'enveloppe intermédiaire liquide.
La rigidification, notamment par coacervation est réalisée en présence de l'enveloppe externe et à travers celle-ci, après sa gélification. L'enveloppe externe joue donc le rôle d'un moule externe pour la réalisation de l'enveloppe intermédiaire rigidifiée.
Selon ce mode de réalisation, la coacervation de la composition intermédiaire de l'enveloppe intermédiaire provoque la rigidification de ladite enveloppe liquide, ce qui présente pour avantage de conférer une résistance mécanique supérieure auxdites capsules.
Au sein d'un coacervat comprenant au moins un biopolymère, les liaisons liant les chaînes de polymères entre elles sont généralement de type ionique, et sont généralement plus fortes que des liaisons présentes au sein d'une membrane de type tensioactif.
Plusieurs modes de coacervation de la composition intermédiaire peuvent être utilisés.
Selon un premier mode de réalisation, la coacervation de la composition intermédiaire est provoquée par une variation de la température ou du pH, ou par un rayonnement électromagnétique.
Selon ce mode de réalisation, le bain de rigidification ne comprend généralement pas d'agent de rigidification,

mais induit la coacervation par une variation des conditions réactionnelles, qui peuvent correspondre à une variation de la température, du pH ou des conditions de concentration ou de dilution, ou à l'application d'un rayonnement UV ou IR, de préférence par une variation de la température.

Le bioplolymère adapté à ce premier mode peut être choisi parmi les biopolymères dont la viscosité varie en fonction des températures telles que les glycanes, les glycoprotéines ou les protéines, telles que celles destinées à former des matrices biologiques extracellulaires. Un biopolymère convenant à l'invention peut être choisi parmi les protéines notamment de la matrice extracellulaire, les protéoglycannes, les glycosaminoglycanes, les polysaccharides et leur forme non hydrolysée ou partiellement hydrolysée. Préférentiellement, le biopolymère est choisi parmi le collagène, la gélatine, la laminine, l'entractine, au moins un GAG tel que l'héparane sulfate ou leur mélange. A titre d'exemple de biopolymère adapté à l'invention, on peut citer le Geltrex™ qui est liquide à basse température et devient élastique à 37°C après 30 minutes d'incubation ou le Matrigel™, qui est liquide à basse température et devient élastique à température ambiante, ou bien le collagène qui gélifie à basse température.

Selon un autre mode de réalisation, la coacervation du biopolymère est effectuée par coacervation avec un cation multivalent.

Selon une première variante, les cations multivalents sont contenus dans le bain de rigidification (ou éventuellement dans la solution gélifiante) et diffusent à travers l'enveloppe externe gélifiée pour réagir avec le biopolymère et former un coacervat.

L'hydrogel constituant l'enveloppe externe gélifiée est généralement suffisamment perméable pour permettre la perméation de cations multivalents.

A titre de biopolymère adapté à cette première variante, on peut noter par exemple, un polyélectrolyte hydrophile, plus particulièrement un polysaccharide (toutefois différent ou identique au polyélectrolyte de l'enveloppe externe gélifiée).

A titre de biopolymère également adapté à cette première variante, on peut évoquer le latex naturel, sous forme d'une dispersion colloïdale de polymères. Un cation multivalent adapté à ce mode de réalisation est par exemple, un cation d'un alcalino-terreux tel que le calcium, le magnésium ou le baryum.

Selon un autre mode de réalisation, la coacervation de la composition intermédiaire est effectuée par coacervation avec un deuxième réactif R2, différent du biopolymère R1.

Selon une première variante, le deuxième réactif R2 est contenu dans le bain de rigidification (ou éventuellement dans la solution gélifiante) et diffuse à travers l'enveloppe externe gélifiée pour réagir avec le biopolymère R1 et former un coacervat.

L'hydrogel constituant l'enveloppe externe gélifiée est généralement suffisamment perméable pour permettre la perméation de tels polymères.

Selon une autre variante, le deuxième réactif R2 est contenu dans la composition intermédiaire formant l'enveloppe intermédiaire liquide avant même l'immersion dans le bain de rigidification, et, lors de l'immersion dans le bain de rigidification, une variation des conditions de température ou de pH provoque la coacervation du biopolymère R1 avec ledit deuxième réactif R2.

La formation du coacervat entre le biopolymère R1 et le deuxième réactif R2 est généralement provoquée par une variation des conditions du milieu réactionnel (température, pH, concentration en réactifs, etc.), généralement occasionnée par l'immersion dans le bain de rigidification.

Typiquement, le biopolymère R1 et le deuxième réactif R2 sont des polymères chargés de charges opposées.

Dans ce cas, la réaction de coacervation résulte de la neutralisation du biopolymère R1 et du deuxième réactif R2 chargés de polarités opposées, et permet la formation d'une structure membranaire rigidifiée par interactions électrostatiques entre le biopolymère R1 et le deuxième réactif R2. L'enveloppe intermédiaire rigidifiée ainsi formée autour du coeur liquide l'encapsule totalement et l'isole de l'extérieur, et notamment de l'enveloppe externe gélifiée.

De préférence, le biopolymère R1 est un polymère chargé (ou polyélectrolyte) de type anionique ou cationique.

De préférence, le deuxième réactif R2 est un polymère chargé (ou polyélectrolyte) de charge opposée au biopolymère R1, de type cationique ou anionique, de préférence hydrophile.

Selon d'autres variantes, le biopolymère R1 est un mélange de polymères chargés de même polarité.

Selon d'autres variantes, le deuxième réactif R2 est un mélange de polymères chargés de même polarité, mais de polarité opposée à celle du biopolymère R1.

Selon un autre mode de réalisation, le biopolymère R1 est un monomère ou un mélange de monomères, propre à polymériser avec le deuxième réactif R2, sous forme de polymère ou de monomère, éventuellement en présence d'un agent de polymérisation.

Ledit deuxième réactif R2 peut être présent dans le bain de rigidification, et passer à travers l'enveloppe externe gélifiée perméable, pour polymériser avec le biopolymère R1.

De manière alternative, le deuxième réactif R2 peut être présent dans la composition intermédiaire, et la polymérisation est provoquée par la perméation d'un agent de polymérisation, contenu dans le bain de rigidification.

Dans ces deux cas, la coacervation de l'enveloppe intermédiaire est due à la polymérisation du biopolymère

R1 et du deuxième réactif R2.

A titre de biopolymère R1 et de deuxième réactif R2, on peut mentionner par exemple, des monomères propres à former des cocacervats de polyuréthanes, tels polyisocyanates et polyols, ou encore des monomères propres à former des coacervats de polyacrylamide.

Selon une première variante, le biopolymère R1 est un polymère anionique hydrophile et le deuxième réactif R2 est un polymère cationique hydrophile.

Selon cette variante, la composition intermédiaire comprenant le biopolymère R1, anionique hydrophile, est une composition aqueuse.

Selon cette variante, le deuxième réactif R2, est un polymère cationique hydrophile qui peut être contenu le cas échéant, dans un bain de rigidification aqueux ou bien dans la composition intermédiaire aqueuse.

A titre de biopolymère R1 adapté à cette variante, on peut mentionner par exemple, l'acide polyacrylique, les polysaccharides.

A titre de deuxième réactif R2 adapté à cette variante, on peut évoquer par exemple, la gélatine, le chitosan.

Selon une autre variante, le biopolymère R1 est un polymère cationique hydrophile et le deuxième réactif R2 est un polymère anionique hydrophile.

Selon cette variante, la composition intermédiaire comprenant le biopolymère R1, cationique hydrophile, est une composition aqueuse.

Selon cette variante, le deuxième réactif R2, anionique hydrophile, peut être contenu dans un bain de rigidification aqueux ou bien dans la composition intermédiaire aqueuse.

A titre de biopolymère R1 adapté à cette variante, on peut citer par exemple, gélatine, le chitosan.

A titre de deuxième réactif R2 adapté à cette variante, on peut évoquer par exemple, l'acide polyacrylique, les polysaccharides.

Selon une autre variante, le biopolymère R1 un polymère cationique lipophile et le deuxième réactif R2 est un polymère anionique hydrophile.

Selon cette variante, la composition intermédiaire comprenant le biopolymère R1, cationique lipophile, est une composition huileuse.

Selon cette variante, le deuxième réactif R2, anionique lipophile, est contenu dans un bain de rigidification huileux.

A titre de biopolymère R1 adapté à cette variante, on peut citer par exemple, l'aminosilicone.

A titre de deuxième réactif R2 adapté à cette variante, on peut mentionner par exemple, l'acide polyacrylique.

Dans le cadre de la présente description, on entend par « **polymère de type anionique** » ou « **polymère anionique** » un polymère comportant des fonctions chimiques de type anionique. On peut aussi parler de polyélectrolyte anionique.

Par « **fonction chimique de type anionique** », on entend une fonction chimique AH capable de céder un proton pour donner une fonction $A^-$. Selon les conditions du milieu dans lequel il se trouve, le polymère de type anionique comporte donc des fonctions chimiques sous forme AH, ou bien sous forme de sa base conjuguée $A^-$.

Comme exemple de fonctions chimiques de type anionique, on peut citer les fonctions acide carboxylique -COOH, éventuellement présentes sous forme d'anion carboxylate $-COO^-$.

Comme exemple de polymère de type anionique, on peut citer tout polymère issu de la polymérisation d'unités monomères comportant au moins une fonction chimique de type acide carboxylique. De tels monomères sont par exemple, l'acide acrylique, l'acide maléique, ou tout monomère éthyléniquement insaturé comportant au moins une fonction acide carboxylique.

Parmi les exemples de polymère de type anionique appropriés à la mise en oeuvre de l'invention, on peut mentionner les copolymères d'acide acrylique ou d'acide maléique et d'autres monomères, tels que l'acrylamide, les acrylates d'alkyle, les acrylates d'alkyle en $C_5$-$C_8$, les acrylates d'alkyle en $C_{10}$-$C_{30}$, les méthacrylates d'alkyle en $C_{12}$-$C_{22}$, les méthacrylates méthoxypolyéthylèneglycol, les acrylates d'hydroxyester.

Dans le cadre de la présente description, on entend par « **polymère de type cationique** » ou « **polymère cationique** » un polymère comportant des fonctions chimiques de type cationique. On peut aussi parler de polyélectrolyte cationique.

Par « fonction chimique de type cationique », on entend une fonction chimique B capable de capter un proton pour donner une fonction $BH^+$. Selon les conditions du milieu dans lequel il se trouve, le polymère de type cationique comporte donc des fonctions chimiques sous forme B, ou bien sous forme $BH^+$, son acide conjugué.

Comme exemple de fonctions chimiques de type cationique, on peut citer les fonctions amine primaire, secondaire et tertiaire, éventuellement présentes sous forme de cations ammoniums.

Ces fonctions peuvent être comprises au sein de la chaîne principale des polymères cationiques ou bien portées par ladite chaîne ou bien portées par des chaînes latérales.

Comme exemple de polymère de type cationique, on peut citer tout polymère issu de la polymérisation d'unités monomères comportant au moins une fonction chimique de type amine primaire, secondaire ou tertiaire. De tels monomères sont par exemple, des monomères comportant des fonctions aziridine, ou tout monomère éthyléniquement insaturé comportant au moins une fonction amine primaire, secondaire ou tertiaire.

Parmi les exemples de polymère de type cationique appropriés à la mise en oeuvre de l'invention, on peut mentionner les polymères silicone modifiés par des fonctions amine primaire, secondaire ou tertiaire, tel l'amodiméthicone, dérivé d'un polymère silicone (polydiméthylsiloxane, aussi appelé diméthicone) :

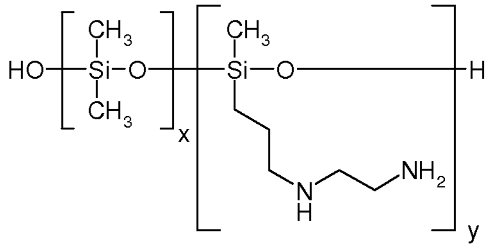

Amodiméthicone

On peut également citer des dérivés de l'amodiméthicone, comme par exemple, des copolymères de l'amodiméthicone, l'aminopropyl diméthicone, et plus généralement des polymères silicones comportant des fonctions amines.

On peut citer le copolymère de bis-isobutyl PEG-14/amodiméthicone et le bis-hydroxy/méthoxy amodiméthicone.

On peut également évoquer les polymères de type polysaccharide comprenant des fonctions amine, tel que le chitosan.

On peut également citer les polymères de type polypeptide comprenant des fonctions amine, tel que la polylysine.

On peut également citer les polymères de type polyéthylèneimine comprenant des fonctions amine, tel que la polyéthylèneimine linéaire ou branchée.

De préférence, le biopolymère R1, et éventuellement le deuxième réactif R2 lorsqu'il est présent, est sous forme d'un latex de polymères.

Dans le cadre de la présente invention, on entend par « **latex** » une dispersion aqueuse stable de particules de polymères, généralement de taille comprise entre 100 nm et 10 $\mu$m, de préférence entre 100 nm et 1 $\mu$m, ou encore entre 1 $\mu$m et 10 $\mu$m.

Les dispersions de latex naturel sont disponibles commercialement, et peuvent être diluées avant utilisation afin de diminuer leur fraction massique de particules de polymères. En général, dans le cadre de la présente invention, on utilise des dispersions de latex naturel de fraction massique comprise de 10% à 60%, de préférence de 20% à 40%.

Une composition de latex de polymère peut être rigidifiée par migration, à travers l'enveloppe gélifiée, d'ions calcium contenus dans le bain de rigidification.

Dans le cas d'un latex naturel, celui-ci se transforme en caoutchouc lors de l'étape de rigidification.

La composition intermédiaire, comprenant le biopolymère R1, peut en outre comprendre un agent de charge, notamment lorsque le biopolymère R1 est sous forme d'un latex de polymère.

Cet agent de charge permet de renforcer la rigidité et la résistance de l'enveloppe intermédiaire rigidifiée.

A titre d'agents de charge adaptés, on peut mentionner la silice, le noir de carbone, et en général tout composé inorganique sous forme de particules colloïdales.

*Étape de dissolution de l'enveloppe externe gélifiée*

Au cours du procédé selon l'invention, lorsque la capsule comprend une enveloppe intermédiaire, le procédé comprend en outre, une étape de dissolution de l'enveloppe externe gélifiée.

L'étape de dépolymérisation a pour but de supprimer l'enveloppe externe gélifiée, sans altérer la structure de l'enveloppe intermédiaire rigidifiée.

Cette étape peut être effectuée par toute méthode de dépolymérisation de l'hydrogel formé lors de l'étape de gélification. Dans le cas d'une enveloppe externe gélifiée d'alginate, la dépolymérisation peut être effectuée par immersion dans une solution de dépolymérisation, comme par exemple, une solution de citrate de sodium concentrée à une teneur massique de 5% minimum, typiquement de 10%, ou bien une solution tampon phosphate salin (encore appelé tampon PBS).

On peut encore citer des solutions d'ions tartrates, d'acide phytique ou d'EDTA, toute solution d'espèces dites chélatantes pour les cations divalents, ou encore des solutions de polymères d'acide acrylique type carbomer, carbopol, polyacrylamide ou polyacrylate.

De manière générale, l'enveloppe intermédiaire rigidifiée n'est pas altérée par l'étape de dépolymérisation de l'enveloppe externe gélifiée.

*La capsule selon l'invention*

La présente invention a également pour objet une capsule comprenant un coeur liquide, et au moins une enveloppe externe encapsulant totalement le coeur liquide à sa périphérie, ladite enveloppe externe étant propre à retenir le coeur liquide lorsque la capsule est plongée dans un gaz et comprenant au moins un polyélectrolyte gélifié et/ou un biopolymère rigidifié, ladite capsule comprenant au moins une cellule eucaryote, préférentiellement de mammifère.

Par **« au moins une cellule eucaryote** » on entend une cellule isolée ou un groupe de cellules notamment un tissu. Il peut s'agir de cellules différenciées ou indifférenciées, de cellules immortalisées ou de cellules tumorales. De même, ces cellules peuvent être adhérentes ou non adhérentes. Ces cellules peuvent être des cellules issues de tout organe ou de tout tissu par exemple, le tissu hépatique, le tissu mammaire, le tissu musculaire ou la peau, plus généralement des co-cultures de tissus tels que par exemple, des tissus épithéliaux avec leur tissu conjonctif de soutien sous jacent, par exemple, le derme et l'épiderme, la muqueuse intestinale ou bronchique. Les cellules eucaryotes sont des cellules de « **mammifères** » typiquement, des cellules issues de tout animal notamment des cellules humaines ou issues d'animaux largement utilisés comme modèle d'étude tel que par exemple, le lapin, le porc, le cobaye, la souris ou le cochon d'inde.

Les capsules selon l'invention ont un diamètre moyen de 100 à 1000 $\mu$m, préférentiellement de 200 à 900 $\mu$m, 300 à 850 $\mu$m, 400 à 800 $\mu$m, 450 à 700 $\mu$m.

Les capsules selon l'invention peuvent comprendre l'ensemble des caractéristiques précédemment évoquées lors de l'exposé de leur procédé d'obtention selon l'invention notamment en combinaison avec les caractéristiques suivantes.

Le coeur liquide est généralement constitué d'une composition interne, généralement liquide ou légèrement visqueuse, qui peut être aqueuse. La composition interne peut également être une dispersion de gouttes d'eau dans une phase huileuse, ou bien une dispersion de gouttes d'huile dans une phase aqueuse, ou tout type d'émulsion multiple de type eau/huile/eau ou huile/eau/huile.

Le coeur liquide peut éventuellement comprendre des particules solides en suspension, telles que des nanoparticules métalliques, des particules minérales ou des particules composites par exemple. Avantageusement, lorsqu'elles sont présentes, la taille desdites particules est comprise de 10 nm à 10 $\mu$m.

Le coeur liquide comprend généralement un ou plusieurs agents actifs, choisis parmi les agents cosmétiques, pharmaceutiques, comestibles, ou lubrifiants, qui peuvent être hydrophiles ou hydrophobes.

Dans une variante, le coeur liquide comprend également, un principe actif cosmétique tel que le hyaluronate de sodium ou d'autres molécules hydratantes/réparatrices, de vitamines, des enzymes, des actifs anti-rides, anti-âge, protecteurs/anti radicalaires, antioxydants, apaisants, adoucissants, anti irritants, tenseurs/lissants, émollients, amincissants, anti capitons, raffermissant, gainants, drainants, anti inflammatoires, dépigmentants, blanchissants, autobronzants, exfoliants, stimulant le renouvellement cellulaire ou stimulant la microcirculation cutanée, absorbants ou filtrants les UV, antipelliculaires.

Dans une autre variante, le coeur liquide comprend un principe actif choisi avantageusement parmi les anticoagulants, les anti-thrombogéniques, les agents anti-mitotiques, les agents anti-prolifération, antiadhésion, anti-migration, les promoteurs d'adhésion cellulaire, les facteurs de croissance, les molécules antiparasitaires, les anti-inflammatoires, les angiogéniques, les inhibiteurs de l'angiogenèse, les vitamines, les hormones, les protéines, les antifongiques, les molécules antimicrobiennes, les antiseptiques ou les antibiotiques.

Le coeur liquide peut comprendre également des excipients, tels que des épaississants, ou des modificateurs de rhéologie. Ces épaississants sont par exemple, des polymères, des cross-polymères, des microgels, des gommes ou des protéines, dont des polysaccharides, des celluloses, des polyosides, des polymères et co-polymères à base de silicone, des particules colloïdales (silice, argiles, latex...).

En variante, le coeur liquide contient des **agents réactifs** tels que des protéines, des polysaccharides, des acides gras ou leur mélange synthétisés par les cellules eucaryotes ou destinés à former un bioréacteur ou à former des tissus en croissance ou matures notamment pour des implants.

Préférentiellement le pH du coeur liquide est entre 7,2 et 7,4.

L'enveloppe externe est formée d'une composition aqueuse ou huileuse externe et comprend au moins un polyélectrolyte qui est différent du biopolymère de l'enveloppe intermédiaire, et au moins un tensio-actif.

Selon l'invention, le polyélectrolyte de l'enveloppe externe est choisi parmi les polysaccharides, les polyélectrolytes de synthèse à base d'acrylates (polyacrylate de sodium, de lithium, de potassium ou d'ammonium, ou polyacrylamide), ou les polyélectrolytes de synthèse à base de sulfonates (poly(styrène sulfonate) de sodium, les alginates d'alcalin (tel qu'un alginate de sodium ou un alginate de potassium), les géllanes les pectines et leur mélange.

L'enveloppe externe comprend de préférence une quantité réduite de tensio-actif.

Le pourcentage massique de tensio-actifs compris dans l'enveloppe externe est généralement inférieur ou égal à 0,5%, de préférence inférieur ou égal à 0,2%, 0,1% ou 0,05% et préférentiellement inférieur ou égal à 0,025%,

0,03% ou 0,01%, par rapport à la masse de l'enveloppe externe.

Dans le cadre de la présente description, on entend par « **tensio-actif** » une molécule amphiphile présentant deux parties de polarité différente, l'une lipophile et apolaire, l'autre hydrophile et polaire. Un tensio-actif peut être de type ionique (cationique ou anionique), zwitterionique ou non-ionique. On peut citer par exemple, le dodécylsulfate de sodium (SDS), le lauryl éther sulfate de sodium (SLES), le triméthyldécylammonium, l'acide glycocholique, l'acide taurocholique, les lécithines, les alkylpolyglucosides (APG) ou les esters du diglycérol.

L'enveloppe intermédiaire comprend au moins un biopolymère choisi parmi les protéines notamment de la matrice extracellulaire, les protéoglycannes, les glycosaminoglycanes, les polysaccharides et leur forme non hydrolysée ou partiellement hydrolysée. Préférentiellement, le biopolymère est choisi parmi le collagène, la gélatine, la laminine, l'entractine, au moins un GAG tel que l'héparane sulfate ou leur mélange.Typiquement, l'enveloppe intermédiaire comprend un mélange de biopolymères tel que le Geltrex™ ou Matrigel™.

Avantageusement, le collagène est de type 1, 2, 3 ou 4. Il peut être non hydrolysé ou partiellement hydrolysé (hydrolyse chimique ou enzymatique).

Préférentiellement, l'enveloppe intermédiaire est formée d'un mélange d'un biopolymère et d'un polyélectrolyte.

Le ratio solution de biopolymère / solution de polyélectrolyte en volumes est supérieur à 75/25, de préférence de 75/25 à 99,9/0,1; 80/20 à 90/10 ; 70/30 à 80/20 ; 65/35 à 75/25. Par exemple, l'enveloppe intermédiaire est un mélange de collagène, préférentiellement, de collagène et d'un polyélectrolyte tel que l'alginate.

Le ratio collagène / alginate en masse est supérieur à 0,6, de préférence de 0,6 à 3; 0,8 à 1,8 ; 0,7 à 1,7 ; 0,8 à 1.

Les inventeurs ont montré que la quantité de biopolymère permet de changer les caractéristiques de la capsule. Ainsi, trop peu de polymère ne permet pas l'adhésion cellulaire des cellules eucaryotes adhérentes présentes dans le coeur liquide, ou permet l'adhésion mais on observe une prolifération cellulaire réduite. Dans le cas des cellules tumorales ou de cellules non adhérentes présentes dans le coeur liquide, l'absence d'adhésion sur la face interne de l'enveloppe intermédiaire crée la formation de sphéroides ou une suspension de cellules dans le coeur liquide.

En présence d'un biopolymère avec une répartition homogène dans l'enveloppe intermédiaire, on observe une adhésion cellulaire, une bonne prolifération cellulaire et une organisation cellulaire en tissu. Par exemple, dans le cas de cellules épithéliales de peau par exemple, on observe l'adhésion des cellules à la surface de l'enveloppe intermédiaire et la formation d'un épithélium pluristratifié. Dans le cas de cellules de derme comme les fibroblastes, présents au sein de l'enveloppe intermédiaire, on observe une structure en couche homogène et disséminée que l'on retrouve naturellement dans le derme. Ceci est observé par exemple, lorsque l'enveloppe intermédiaire a un ratio en masse collagène/alginate supérieur à 0,6.

L'enveloppe intermédiaire comprend au moins un polyol. Typiquement, le polyol est choisi parmi un monosaccharide, un disaccharide, un polymère de polyol et leur mélange. Préférentiellement, le polyol est un monosaccharide choisi parmi le glycérol, l'érythritol, le xylitol, l'arabitol, le ribitol, le sorbitol, le dulcitol, le mannitol, le volemitol et leur mélange. Avantageusement, le polyol est un disaccharide choisi parmi le maltitol, l'isomaltitol, le lactitol et leur mélange. Préférentiellement le polyol est le sorbitol.

L'enveloppe intermédiaire est particulièrement avantageuse en ce qu'elle permet de compartimenter les capsules et d'offrir une surface propice à l'adhésion cellulaire ainsi qu'un compartiment supplémentaire propice à la survie cellulaire, indépendant du coeur de la capsule.

Dans le cadre de la présente invention, le coeur liquide et/ou l'enveloppe intermédiaire et/ou l'enveloppe externe comprend au moins un **agent nutritif.** L'agent nutritif peut être un acide aminé essentiel (arginine, histidine, isoleucine, leucine, lysine, méthionine, phénylalanine, thréonine, tryptophane, valine, glutamine, cystéine, tyrosine) ou un mélange d'acides aminés essentiels, des sels minéraux (notamment NaCl, KCl, CaCl2, MgCl2, NaH2PO4 ou leur mélange), des sucres tel que le pyruvate de sodium et/ou des vitamines. L'agent nutritif peut être du sérum (tel que le sérum de veau foetal), des facteurs de croissance cellulaire (EGF, FGF, PDGF), des facteurs de différentiation (exemple fibronectine), des inhibiteurs enzymatiques (inhibiteurs de la trypsine par exemple, tel que l'alpha-1 antitrypsine) et/ou des antibiotiques (Pénicilline G, Streptomycine, Amphotéricine B).

Avantageusement, l'agent nutritif est introduit dans un tampon tel qu'un tampon phosphate (PBS phosphate-buffered saline) ou un milieu de culture cellulaire par exemple, du RPMI (Roswell Park Memorial Institute médium), du MEM (Minimum Essential Médium Eagle), ou du DMEM (Dulbecco/Vogt modified Eagle's minimal essential médium).

Préférentiellement le pH du coeur liquide et/ou de l'enveloppe intermédiaire et/ou de l'enveloppe externe est entre 7,2 et 7,4.

Une capsule peut contenir 1 à $10^{12}$ cellules, préférentiellement, 10 à $10^{10}$, 20 à $10^{9}$, 30 à $10^{8}$, 40 à $10^{7}$, 50 à $10^{6}$, 100 à $10^{5}$, 150 à $10^{4}$, 200 à $10^{3}$ cellules eucaryotes de mammifère.

*Capsules gélifiées et rigidifiées*

Selon un premier mode de réalisation (voir figure 1), une capsule 10 selon l'invention comprend en outre, une enveloppe externe gélifiée 40 encapsulant totalement à sa périphérie l'enveloppe rigidifiée 30.

Selon une première variante, les capsules gélifiées et rigidifiées comprennent des cellules eucaryotes 70 uniquement dans le coeur liquide 20 (figure 5A). Selon d'autres variantes, les capsules gélifiées et rigidifiées comprennent des cellules eucaryotes 70 dans le coeur liquide 20 et dans l'enveloppe intermédiaire 30 lorsque ladite capsule ne comprend qu'une enveloppe intermédiaire ou dans ses deux enveloppes intermédiaires 30 et 90 lorsque ladite capsule 80 en comprend deux (figures 5B et 5D). Selon une troisième variante, les capsules gélifiées et rigidifiées comprennent des cellules eucaryotes 70 dans le coeur liquide 20, dans l'enveloppe intermédiaire et dans l'enveloppe externe 40 lorsque ladite enveloppe externe contient au moins un biopolymère (figure 5C).

De telles capsules correspondent aux capsules gélifiées et rigidifiées définies ci-dessus, et sont typiquement obtenues par la production de gouttes multi-composantes par co-extrusion par convoyage séparé dans une enveloppe à flux multiples. Dans le cas d'une triple enveloppe, les trois flux sont : un premier flux constitué de la composition interne, un deuxième flux constitué de la composition intermédiaire et un troisième flux constitué de la composition externe. Les gouttes multi-composantes subissent ensuite une étape de gélification puis de rigidification selon le procédé de l'invention.

De préférence, l'enveloppe externe gélifiée des capsules selon l'invention possède une épaisseur comprise de 10 $\mu$m à 500 $\mu$m, de préférence de 20 $\mu$m à 200 $\mu$m, et avantageusement de 45 $\mu$m à 150 $\mu$m, plus préférentiellement, 50 $\mu$m à 100 $\mu$m.

La finesse de l'épaisseur de l'enveloppe externe gélifiée permet généralement de rendre cette enveloppe externe transparente.

Les capsules selon l'invention présentent généralement un rapport volumique entre le coeur et l'ensemble des enveloppes intermédiaire et externe supérieur à 2, et de préférence inférieur à 50.

Selon un mode de réalisation particulier, les capsules selon l'invention présentent généralement un rapport volumique entre le coeur et l'ensemble des enveloppes intermédiaire et externe compris entre 5 et 10.

La présence d'un tensio-actif dans la composition externe permet d'améliorer la formation et la gélification des gouttes multi-composantes selon le procédé tel que décrit précédemment. Néanmoins, afin d'améliorer la survie cellulaire, le pourcentage massique de tensio-actif compris dans une capsule selon l'invention est préférentiellement inférieur ou égal à 0,050%, de préférence inférieur ou égal à 0,025% et préférentiellement inférieur ou égal à 0,010%, voire inférieur ou égal à 0,005%, préférentiellement inférieur ou égal à 0,001%, voire inférieur ou égal à 0,0005% par rapport à la masse totale de la capsule. Préférentiellement, le pourcentage massique de tensio-actif compris dans l'enveloppe externe, est préférentiellement inférieur ou égal à 0,050%, de préférence inférieur ou égal à 0,025% et préférentiellement inférieur ou égal à 0,010%, voire inférieur ou égal à 0,005%, par rapport à la masse totale de l'enveloppe externe.

*Capsules rigidifiées*

Selon un autre mode de réalisation (voir figure 2A), une capsule 50 selon l'invention comprend un coeur liquide 20 et une enveloppe externe rigidifiée 30 encapsulant totalement à sa périphérie le coeur liquide 20.

Selon une première variante, les capsules rigidifiées 50 comprennent des cellules eucaryotes 70 uniquement dans le coeur liquide 20 (figure 3A). Selon une seconde variante, les capsules rigidifiées comprennent des cellules eucaryotes 70 dans le coeur liquide 20 et dans l'enveloppe externe rigidifiée 30 (figures 3B).

De telles capsules correspondent à des capsules rigidifiées, dépourvues d'enveloppe gélifiée. Ces capsules sont typiquement obtenues par la production de gouttes multi-composantes par co-extrusion par convoyage séparé dans une triple enveloppe de trois flux : un premier flux constitué de la composition interne, un deuxième flux constitué de la composition intermédiaire et un troisième flux constitué de la composition externe. Les gouttes multi-composantes subissent ensuite une étape de gélification, de rigidification et enfin une étape de dépolymérisation selon le procédé de l'invention.

Après élimination de l'enveloppe externe gélifiée, l'enveloppe intermédiaire rigidifiée devient l'enveloppe externe rigidifiée des capsules rigidifiées. Ces capsules bénéficient alors des propriétés de surface de l'enveloppe rigidifiée, qui peut être de type hydrophile ou de type lipophile.

L'enveloppe rigidifiée est destinée à apporter des nouvelles propriétés de surface aux capsules de type coeur-enveloppe préalablement décrites, en s'affranchissant de la limite fixée par la nature de l'enveloppe externe, qui était jusqu'à présent essentiellement de type hydrogel (cf. notamment WO 2010/063937).

Etant donné la diversité dans le choix des matériaux constituant l'enveloppe intermédiaire rigidifiée, les capsules ainsi obtenues peuvent présenter tout type de fonctionnalité à leur surface.

On peut donc envisager la préparation de capsules présentant des propriétés de surface adaptées à divers domaines, comme par exemple, dans les applications en biotechnologie.

On peut donc également envisager l'obtention de performances d'encapsulation très supérieures, sans contraintes sur la nature de la solution à encapsuler. Ceci est particulièrement recherché dans certaines applications pour lesquelles il n'y a pas de solutions existantes satisfaisantes.

Comme exemple de propriétés de surface intéressantes qui n'étaient pas disponibles avec les capsules comportant une enveloppe gélifiée connues jusqu'à présent, et qui sont désormais accessibles avec les capsules rigidifiées selon l'invention, on peut citer des propriétés d'étanchéité, de rigidité ou au contraire d'élasticité, ou encore présentant des propriétés de biomimétisme.

En particulier, lorsque l'enveloppe rigidifiée est à base de latex, on obtient des capsules rigidifiées comportant une enveloppe externe de grande étanchéité, même vis-à-vis de l'eau.

En particulier, lorsque l'enveloppe rigidifiée comprend au moins un biopolymère est choisi parmi les protéines de la matrice extracellulaire, les protéoglycannes, les glycosaminoglycanes, les polysaccharides et leur forme non hydrolysée ou partiellement hydrolysée, l'enveloppe rigidifiée permet l'adhésion cellulaire. Préférentiellement, le biopolymère est choisi parmi le collagène, la gélatine, la laminine, l'entractine , au moins un GAG tel que l'héparane sulfate ou leur mélange. Typiquement, le biopolymère est un mélange tel que le Geltrex™, Matrigel™ ou le collagène. Ainsi, l'invention permet l'obtention de capsules rigidifiées comportant une enveloppe dont la surface interne est biomimétique et propice à la croissance de cellules végétales, animales ou humaines.

De manière générale, une enveloppe rigidifiée obtenue par exemple, par coacervation de polymères est plus rigide, plus étanche et moins perméable qu'une enveloppe gélifiée obtenue par gélification.

On comprend donc que les capsules rigidifiées de l'invention présentent des propriétés accrues d'étanchéité par rapport aux capsules simplement gélifiées de l'art antérieur.

Il est également possible de fonctionnaliser la surface de l'enveloppe rigidifiée afin de doter les capsules des propriétés souhaitées, telles que des propriétés d'hydrophilicité, de lipophilicité, de charges électriques.

On peut notamment envisager encapsuler, à titre d'agent actif, des composés cosmétiques, pharmaceutiques, comestibles, des lubrifiants, des protéines, des réactifs destinés à former un bioréacteur ou de cellules destinées à se diviser.

On peut également envisager encapsuler des cellules pour de la culture cellulaire ou pour des implants ou des cellules destinées à former des tissus. Dans ce cas, l'enveloppe externe rigidifiée des capsules est avantageusement perméable aux nutriments du milieu extérieur afin que les cellules se développent de manière efficace.

On peut également envisager d'encapsuler des protéines, des polysaccharides ou des acides nucléiques ou d'encapsuler des cellules en vue qu'elles produisent ces protéines, polysaccharides ou acides nucléiques. Dans ce cas, l'enveloppe externe rigidifiée des capsules, typiquement à base de latex, est avantageusement perméable à l'eau uniquement. Le relargage du coeur est alors typiquement provoqué par éclatement de la capsule par choc osmotique lorsque la capsule entre en contact de l'eau.

De préférence, l'enveloppe rigidifiée des capsules selon l'invention possède une épaisseur comprise de 10 $\mu$m à 1000 $\mu$m, de préférence de 1 $\mu$m à 1000 $\mu$m, et avantageusement de 20 $\mu$m à 500 $\mu$m.

Les capsules selon l'invention présentent généralement un rapport volumique entre le coeur et l'enveloppe rigidifiée supérieur à 2, et de préférence inférieur à 50.

Selon un mode de réalisation particulier, les capsules selon l'invention présentent généralement un rapport volumique entre le coeur et l'enveloppe rigidifiée compris entre 5 et 10.

Les capsules selon l'invention, pourvues ou dépourvues d'enveloppe externe gélifiée, possèdent généralement une taille moyenne comprise de 100 $\mu$m à 6 mm, de préférence de 100 $\mu$m à 500 $\mu$m.

Pour une utilisation des capsules en culture cellulaire ou en biologie de manière générale, une taille avantageuse des capsules se situe typiquement de 100 $\mu$m à 1000 $\mu$m, préférentiellement de 250 à 700 $\mu$m.

*Capsules gélifiées*

Selon un autre mode de réalisation (voir figure 2B), une capsule 60 selon l'invention comprend un coeur liquide 20 et une enveloppe externe gélifiée 40 encapsulant totalement à sa périphérie le coeur liquide 20.

Selon une première variante, les capsules gélifiées 60 comprennent des cellules eucaryotes 70 uniquement dans le coeur liquide 20 (figure 4A). Selon une seconde variante, les capsules gélifiées 60 comprennent des cellules eucaryotes 70 dans le coeur liquide 20 et dans enveloppe externe gélifiée 40 lorsque ladite enveloppe externe gélifiée comprend au moins un biopolymère (figures 4B).

Ces capsules sont typiquement obtenues par la production de gouttes multi-composantes par co-extrusion par convoyage séparé dans une double enveloppe par co-extrusion de deux compositions.

Dans ce cas, la double enveloppe convoi deux flux : un premier flux constitué de la composition interne, un deuxième flux constitué de la composition externe puis selon le procédé de l'invention, les gouttes obtenues subissent une étape de gélification.

*Capsules modèle de culture tridimensionnelle*

Afin de mettre en place un modèle de culture tridimensionnelle, les capsules utilisées peuvent être des capsules gélifiées, des capsules gélifiées rigidifiées ou des capsules rigidifiées. Préférentiellement, ces capsules peuvent contenir

des cellules eucaryotes de mammifère dans le coeur liquide et/ou dans l'enveloppe intermédiaire et/ou dans l'enveloppe externe, lorsqu'elle contient un biopolymère.

Préférablement, l'enveloppe externe comprend un alginate, l'enveloppe rigide comprend du collagène ou un mélange de collagène, de gélatine, de laminine, d'entractine et d'héparane sulfate. Un polyol tel que le sorbitol peut être ajouté dans la composition de l'enveloppe intermédiaire.

Les cellules eucaryotes sont préférentiellement des cellules murines ou humaines notamment, il peut s'agir de cellules tumorales ou de cellules d'un tissu spécifique tel que le sang, le sein, le foie, le derme ou l'épiderme notamment.

A titre illustratif, afin d'obtenir des modèles de peau, plusieurs variantes peuvent être mises en oeuvre notamment, à partir d'une capsule comprenant une enveloppe intermédiaire, une enveloppe externe et un coeur liquide.

Selon une première variante, les capsules contiennent dans le coeur liquide des kératinocytes. Ces capsules sont particulièrement avantageuses en ce qu'elles constituent un bon modèle de l'épiderme et permettent notamment le criblage de principes actifs cosmétiques destinés à l'épiderme.

Selon une seconde variante, l'enveloppe intermédiaire comprend des fibroblastes et le coeur liquide ne comprend pas de cellules. Il s'agit dans ce cas d'un modèle de derme avantageux qui permet notamment le criblage de principes actifs cosmétiques destinés au derme.

Selon un mode particulier de mise en oeuvre de l'invention, le coeur liquide comprend des kératinocytes et l'enveloppe intermédiaire comprend des fibroblastes et/ou des mélanocytes. Une telle capsule constitue un modèle de peau d'intérêt en ce qu'il constitue un modèle tridimensionnel de la jonction dermo-épidermique permettant un criblage d'actif tout en s'affranchissant de l'influences de la matrice extracellulaire.

Il faut noter que la reconstruction d'un tissu cutané au sein de capsules d'alginate nécessite de permettre la compartimentation de ces capsules en deux zones correspondant aux deux feuillets composant la peau, à savoir l'épiderme et le derme. Du point de vu biologique, la composition de l'enveloppe intermédiaire permet la survie et la croissance des fibroblastes qui seront disséminés en son sein, recréant ainsi une matrice similaire à la matrice dermique. Enfin, la composition de l'enveloppe intermédiaire permet l'adhésion des kératinocytes à sa surface.

La capsule selon l'invention répond à l'ensemble de ces critères et permet l'établissement d'un modèle fidèle de peau.

Les capsules rigides dans lesquelles l'enveloppe gélifiée a été dépolymérisée sont d'intérêt pour l'établissement de modèles de lame basale afin d'étudier par exemple les interactions entre un type cellulaire (cellule épithéliale ou tumorale) avec la lame basale. Dans ce cas, l'enveloppe rigide peut comprendre du collagène ou un mélange de collagène, de gélatine, de laminine, d'entractine et d'héparane sulfate. Préférablement, un polyol tel que le sorbitol est ajouté dans la composition de l'enveloppe. Selon cette variante, les cellules étudiées sont comprises dans le coeur liquide. Ce modèle permet notamment l'étude de la migration cellulaire notamment le cas de métastases.

<u>Procédé de stockage de cellules eucaryotes</u>

Dans l'industrie pharmaceutique ou dans l'industrie cosmétique, les capsules précitées sont notamment remplies de produits biologiquement ou cosmétiquement actifs. Elles sont utilisées notamment pour protéger leur contenu et contrôler le relargage du produit qu'elles contiennent. Les capsules selon l'invention sont particulièrement adaptées pour le stockage ou la conservation d'échantillons biologiques notamment de cellules eucaryotes, préférentiellement de mammifère sous la forme de cellules isolées ou de tissus, pour l'obtention de modèles de culture tridimensionnelle, pour la production ou le criblage à haut débit d'actif notamment cosmétique.

La présente invention concerne donc un procédé de stockage ou de conservation de cellules eucaryotes, préférentiellement de mammifère notamment, sous la forme de cellules isolées ou de tissus comprenant une étape de préparation d'une capsule par le procédé selon l'invention et une étape de stockage desdites capsules obtenues.

Le procédé de préparation de capsules permet d'obtenir au moins une enveloppe extérieure d'un seul tenant garantissant l'herméticité de cette dernière et ainsi la bonne conservation du coeur liquide

Le coeur liquide peut éventuellement comprendre un **agent de stockage,** tel qu'un tampon (tampon Hepes) ou un agent de cryoprotection à savoir, l'acétamide de méthyle, le méthanol, l'éthylène glycol, la polyvinylpyrrolidone, le diméthylsulfoxyde (DMSO) ou le glycérol. Les conditions de stockages sont connues de l'homme du métier. Ainsi, des capsules contenant des cellules eucaryotes ainsi qu'un agent de stockage pourront être conservées dans de l'azote liquide pendant des durées assez longues.

Dans le cadre de la présente invention, le coeur liquide, l'enveloppe intermédiaire et/ou l'enveloppe externe comprend au moins un **agent nutritif.**

Préférentiellement le pH du coeur liquide, l'enveloppe intermédiaire et/ou de l'enveloppe externe est entre 7,2 et 7,4.

*Utilisation d'une capsule pour la culture ex vivo de cellules eucaryotes de mammifère et Procédé in vitro de culture de cellules eucaryotes*

L'invention concerne de plus, l'utilisation d'une capsule selon l'invention pour la culture *in vitro* de cellules eucaryotes de mammifère.

L'invention concerne en outre, un procédé *in vitro* de culture de cellules eucaryotes comprenant les étapes suivantes de :

a) mise en culture d'une capsule selon l'invention dans des conditions suffisantes pour la croissance cellulaire, et
b) récolte de ladite capsule.

Les capsules selon l'invention, permettent une mise en culture *in vitro* ou *in capsula* de cellules adhérentes telles que des cellules issues d'un tissu organisé comme la peau, le foie, le sein, ou de cellules non-adhérentes telles que des cellules d'un tissu non organisé comme le sang ou des cellules tumorales.

La capsule selon l'invention permet une mise en culture des cellules eucaryotes de mammifère en suspension et/ou en sphéroïde et/ou en tissu.

Les capsules selon l'invention, peuvent être mises en culture dans des « conditions suffisantes pour la croissance cellulaire » qui sont connues de l'homme du métier tel que notamment en présence d'un tampon ou d'un milieu de base de culture additionné d'au moins un agent nutritif tel que décrit précédemment. Les « conditions suffisantes pour la croissance cellulaire » notamment les conditions et temps d'incubation pourront être adaptés selon le type cellulaire par l'homme du métier, les capsules pourront être cultivées notamment à 37°C dans 5% de CO2.

Les capsules peuvent être mises en culture dans un « milieu de base de culture » approprié pour leur croissance, suivant le type cellulaire utilisé, ce milieu peut être un milieu synthétique avec ou sans sérum, couramment disponible dans le commerce, tel qu'un milieu sans sérum du type RPMI, ou un milieu avec sérum IMDM, MEM ou DMEM. Ce milieu est additionné d'au moins un agent nutritif tel que défini ci-dessus. La présence de sérum dans le milieu de culture n'est pas obligatoire mais améliore les résultats de culture. Le milieu de base de culture peut classiquement contenir ou être additionné d'antibiotiques pour éviter les contaminations au cours de la culture cellulaire, et de glutamine.

Un milieu de base de culture approprié pour des fibroblastes humain peut être par exemple milieu DME complet, soit du Dulbecco-Vogt modification of Eagle's médium (DMEM; Gibco, Burlington, On, Canada) contenant 10% de sérum de veau foetal (SVF; Hyclone, Logan, UT, USA), 100 U/ml de pénicilline G (Sigma) et 25 μg/ml de gentamycine (Schering, Pointe-Claire, Qc, Canada). Les capsules sont incubées à 37°C dans 8% de CO2 et le milieu de culture peut être changé tous les 2 jours.

Un milieu approprié pour des kératinocytes est connu de l'homme du métier par exemple, un milieu de base de culture peut être un milieu DME complet en présence de facteurs de croissance, notamment d'acides aminés, sérum, toxine cholérique, insuline, tri-iodo-thyronine et solution tampon de pH. En particulier, un tel milieu de culture pourra notamment contenir au moins un facteur de croissance mitogénique pour les kératinocytes (par exemple epidermal growth factor (EGF) et/ou keratinocyte growth factor (KGF), en particulier du KGF), de l'insuline, de l'hydrocortisone et facultativement un antibiotique (ex : gentamycine, amphotericine B). Les capsules sont incubées à 37°C dans 5% de CO2 et le milieu de culture peut être changé tous les 2 jours.

Pour les mélanocytes, le milieu de culture adapté contiendra ou non l'ester de phorbol et pourra être composé d'un milieu de base tels que le DMEM/F12 ou le MCDB153 additionné de facteurs de croissance spécifiques aux mélanocytes (tels que par exemple bFGF, SCF, ET-1, ET3, αMSH) et en particulier dans le milieu M2 (Promocell) ou dans d'autres milieux tel que le M254 (Cascades Biologics™). Les capsules sont incubées à 37°C dans 5% de CO2 et le milieu de culture peut être changé tous les 2 jours.

De telles capsules peuvent être mises en culture par exemple juqu'à 1 mois à 37°C

La récolte des capsules peut se faire par simple élimination du milieu de culture par filtration ou par toute autre technique de récupération des capsules.

*Méthode de criblage de principes actifs*

L'invention porte également sur une méthode de criblage de principes actifs comprenant :

a) la mise en culture d'une capsule selon l'invention en présence et en absence d'une substance candidate,
b) la détection d'un phénotype d'intérêt dans les cellules de la capsule cultivée en présence de la substance candidate par rapport aux cellules de la capsule cultivée en absence de la substance candidate, et
c) l'identification de la substance en tant que principe actif cosmétique si un phénotype d'intérêt a été détecté.

Dans la présente demande, on entend par « **principe actif** » une molécule qui possède un effet thérapeutique

ou cosmétique, il s'agit donc d'un actif thérapeutique ou cosmétique. Principe actif peut être libre ou véhiculé (dans un solvant ou en mélange avec un excipant), encapsulé (par exemple, dans des liposomes), ou vectorisé (par exemple, dans des nanoparticules avec des ligands de ciblage en surface). Par exemple, il peut s'agir de toute molécule ayant des propriétés thérapeutiques entrant dans la composition d'un médicament. On pourra citer par exemple, les anticoagulants, les anti-thrombogéniques, les agents anti-mitotiques, les agents anti-prolifération, antiadhésion ou les antibiotiques. Cette liste n'est pas exhaustive et s'étend à tout principe actif thérapeutique connu de l'homme du métier. Il peut s'agir également de toute molécule entrant dans la composition d'une préparation cosmétique qui assure l'efficacité du produit (par opposition aux autres ingrédients de la composition tels que les excipients ou autres additifs (adjuvants, conservateurs) qui assurent une fonction différente). Ainsi, un principe actif peut être toute molécule assurant l'effet cosmétique des produits cosmétiques tels que les produits d'hygiène, les produits de soin, les produits capillaires pour en citer quelque uns. Les cosmétiques sont des produits d'hygiène et d'embellissement. Un cosmétique est une substance ou une préparation destinée à être mise en contact avec diverses parties superficielles du corps humain, en vue, exclusivement ou principalement, de les nettoyer, protéger, parfumer, maintenir en bon état le corps humain, de modifier son aspect ou d'en corriger l'odeur. Ainsi, par « effet cosmétique », on entend dans la présente description, l'effet d'hygiène ou d'embellissement précité que le produit cosmétique est destiné à, et conçu pour, accomplir. A titre de principes actifs cosmétiques, on pourra citer par exemple les acides de fruits (exfoliants), le rétinol ou vitamine A (antioxydant), certaines huiles essentielles, l'aloe vera, les extraits d'algues, et les acides aminés. Cette liste n'est pas exhaustive et s'étend à tout principe actif cosmétique connu de l'homme du métier.

La « **substance candidate** » selon l'invention, est une molécule d'origine naturelle ou synthétique dont on veut tester les effets thérapeutiques ou cométiques. Cette molécule peut être une molécule chimique de petite taille ou de grande taille tel qu'un polymère, une molécule biologique tel qu'un peptide, une protéine, un saccharide ou un polysaccharide, un acide nucléique ou un acide gras.

Ladite substance candidate peut être ajoutée au tampon ou au milieu de culture dans lequel les capsules sont incubées.

On entend par « **phénotype d'intérêt** » toute modification d'un caractère biologique de la cellule, une telle modification pouvant se traduire par exemple, par l'arrêt de prolifération cellulaire, la mort cellulaire, l'expression de marqueurs cellulaires ou une perte d'adhésion intercellulaire ou un décollement des cellules des parois de la capsule.

Le phénotype d'intérêt peut être détecté par observation directe notamment par un marquage fluorescent spécifique tel que fluorescence directe (GFP) ou par immunofluorescence, ou par toute autre technique génomique ou protéomique connue de l'homme du métier.

La méthode de criblage selon l'invention est avantageuse en ce qu'elle permet un criblage à haut débit dans des environnements 3D physiologiquement pertinents, elle permet également une détection, une sélection et une récupération des cellules analysées pouvant se faire par des moyens automatisés. Inversement, par l'encapsulation d'une cellule individuelle puis une mise en culture, la méthode selon l'invention permet une exposition simultanée d'un très grand nombre de colonies cellulaires à des conditions expérimentales strictement identiques.

Bien qu'ayant des significations distinctes, les termes « comprenant », « contenant », « comportant » et « consistant en » ont été utilisés de manière interchangeable dans la description de l'invention, et peuvent être remplacés l'un par l'autre.

L'invention sera mieux comprise à la lecture qui va suivre, donnée uniquement à titre d'exemple, et faite en se référant aux dessins annexés, sur lesquels :

- la Figure 1 est une vue à grande échelle, en coupe suivant un plan vertical médian d'une capsule gélifiée et rigidifiée selon l'invention ; et
- la Figure 2 est une vue à grande échelle, en coupe suivant un plan vertical médian d'une capsule rigidifiée (A) ou gélatinisée (B) selon la divulgation.
- la Figure 3 est une vue à grande échelle, en coupe suivant un plan vertical médian d'une capsule rigidifiée dont le coeur liquide contient des cellules eucaryotes (A) ou dont le coeur liquide et l'enveloppe rigidifiée (B) contiennent des cellules eucaryotes.
- la Figure 4 est une vue à grande échelle, en coupe suivant un plan vertical médian d'une capsule gélifiée dont le coeur liquide contient des cellules eucaryotes (A) ou dont le coeur liquide et l'enveloppe gélifiée (B) contiennent des cellules eucaryotes.
- la Figure 5 est une vue à grande échelle, en coupe suivant un plan vertical médian d'une capsule gélifiée et rigidifiée selon l'invention comprenant un coeur liquide, une enveloppe externe et au moins une enveloppe intermédiaire. Dans la figure 5A, la capsule gélifiée et rigidifiée comprend des cellules eucaryotes dans le coeur liquide. Dans la figure 5B, la capsule gélifiée et rigidifiée comprend des cellules eucaryotes dans le coeur liquide et dans l'enveloppe intermédiaire. Dans la figure 5C, la capsule gélifiée et rigidifiée comprend des cellules eucaryotes dans le coeur liquide, dans l'enveloppe intermédiaire et dans l'enveloppe externe. Dans la figure 5D, la capsule gélifiée et rigidifiée comprend deux enveloppes intermédiaires, le coeur liquide ainsi que les deux enveloppes intermédiaires comprenant

des cellules eucaryotes.

- la Figure 6 porte sur la conception et le principe de fonctionnement du dispositif microfluidique pour la formation d'une microcapsule. La plate-forme microfluidique est composée d'un système d'injection fluidique externe, d'un micro-dispositif de co-extrusion et d'un bain de gélification hors puce (non schématisé). La vue agrandie de la puce montre la configuration à 3 voies avec la suspension cellulaire (CS), la solution intermédiaire (IS), et la solution d'alginate (AL) circulant respectivement dans le capillaire le plus interne, intermédiaire et le plus externe. Les orifices d'entrée de la puce sont connectés à 3 seringues contrôlées par 2 pompes à seringue. Les microgouttelettes liquides de composé chutent dans un bain de calcium iso-osmotique à 100 mM. La gélification de la coque d'alginate médiée par le calcium fige la structure de la capsule alors que les solutions internes se diffusent et maintiennent les cellules encapsulées. L'analyse du jet à la sortie de l'embout par une caméra haute vitesse, montre qu'à un bas débit q (débit total), on observe une formation de gouttelettes de l'ordre du millimètre. À un débit q élevé, la longueur intacte du jet est trop longue par comparaison à la distance entre l'embout et le bain de gélification. Aucune formation de gouttelettes n'est observée lorsque l'écoulement entre en contact avec le bain. À un débit *q* intermédiaire (typiquement entre 50 et 150 $ml.h^{-1}$), une dispersion du jet et une formation de gouttes se produit avant l'impact.
- la Figure 7 porte sur la caractérisation morphométrique et mécanique des microcapsules d'alginate. (A) est un tracé à points en 2D typique du rayon moyen en fonction de la circularité de capsules (sans cellules) directement collectées à partir du bain de gélification montrant l'existence de deux populations de capsules : la fraction des petites capsules (R~150 μm) sphériques (45%) et la fraction des capsules plus grosses ellipsoïdales ou déformées (55%) suite à une coalescence des gouttelettes. (B) est un tracé du rapport d'aspect de la capsule $h/R_{out}$ en fonction du rapport entre les débits interne et externe $q_{in}/q_{out.}$ Les points noirs sont les données expérimentales. La ligne en pointillés est la courbe théorique dérivée de la conservation du volume (voir exemple 5, section Méthodes).
- la Figure 8 (A) est le tracé de la déformation de la capsule $\Delta R/R0$ en fonction de la différence de pression osmotique $\pi 0$. (B) est le Module de Young *E* d'un gel d'alginate à partir d'un essai de gonflement osmotique. Le module de Young *E* du gel d'alginate est dérivé de la pente de la ligne en pointillés qui est ajustée aux données du tracé A. Histogramme représentatif de la distribution des valeurs du module de Young (n=26).
- la Figure 9 (A) Micro-indentation de capsules de gel d'alginate en utilisant la microscopie à force atomique. Des courbes force-déplacement d'approche (trait fin, courbe du haut) et de rétraction (trait épais, courbe du dessous) typiques ont été obtenues sur une seule capsule. (B) Élongation de fils d'alginate macroscopiques. Tracé de stress-élongation pour 5 cylindres de gel d'alginate différents (longueur au repos d'environ 0,2 m, diamètre d'environ 1 mm). Le stress est dérivé du poids des masses calibrées en supposant un rapport de Poisson du gel v= 1/2. La ligne est un ajustement polynomial aux données de second ordre générant une dépendance aux contraintes phénoménologiques du module de Young à une déformation importante.
- la Figure 10 (A) Distribution du nombre moyen de cellules par capsule ajustée avec une courbe Gaussienne. Le comptage des cellules est obtenu à partir de micrographies en contraste de phase des capsules individuelles fusionnées à des micrographies en épifluorescence de cellules encapsulées colorées avec des colorants pour cellules vivantes/mortes. (B) Tracé de la rigidité du MCS en fonction du temps à partir de mesures obtenues par clichés acquis par microscopie à contraste de phase, d'un sphéroïde de cellules CT26 en expansion à l'intérieur d'une capsule jusqu'à confluence. Le temps t=0 correspond à l'encapsulation.
- les Figures 11 et 12 portent sur l'analyse quantitative de la croissance de sphéroïdes et de la déformation des capsules. Des tracés temporels représentatifs montrent l'influence de la rigidité de la capsule (via l'épaisseur de la coque) sur la croissance et les caractéristiques mécaniques du MCS. La **figure 11A** représente l'étude du rayon du sphéroïde $R_{MCS}$ normalisé par rapport au rayon interne de la capsule non déformée $R_0$ en fonction du temps. La **figure 11B** représente l'étude du taux de croissance apparent $3\dot{R}_{MCS}/R_{MCS}$ en fonction du temps. La **figure 12A** représente l'étude du rapport d'aspect $h/R_{out}$ de la capsule en fonction du temps. Les points sont les données expérimentales. Les lignes sont les prédictions théoriques en supposant que le gel d'alginate est un matériau incompressible. La **figure 12B** représente l'étude de la pression exercée par le sphéroïde sur la paroi de la capsule en fonction du temps. Les points à centre vide et les lignes fines correspondent à une capsule fine (*h*=8 μm). Les points pleins et les lignes noires épaisses correspondent à une capsule épaisse (h=28 μm). Les différentes phases discutées dans le texte sont identifiées par des rectangles grisés. La confluence est considérée comme le temps de référence t=0.
- La figure 13 porte sur l'analyse statistique (n=23 pour les capsules fines et n=17 pour les capsules épaisses). La **figure 13A** représente l'étude du taux de croissance apparent du sphéroïde selon une échelle logarithmique pour des monocouches cellulaires (2D), des sphéroïdes en expansion libre (3D, libres), des sphéroïdes encapsulés à confluence (3D, t=0), et au cours des derniers stades (phase 3) pour les deux épaisseurs de coque (3D, fine et épaisse). Le taux de pression augmente (**13B**) juste après la confluence (phase 2) et au cours des derniers stades (phase 3). La **figure 13C** représente l'étude de l'intensité en contraste de phase en fonction de la distance radiale par rapport au centre du MCS et du temps. La ligne claire est la paroi externe de la capsule. Barres d'échelle, 20 heures et 50 μm.

- la Figure 14 Croissance de sphéroïdes à l'intérieur de capsules d'alginate et après éclatement des capsules. Tracés représentatifs montrant l'évolution temporelle du rayon du sphéroïde $R_{MCS}$ normalisé par rapport au rayon interne initial de la capsule $R_0$ pour différents sphéroïdes CT26 en expansion dans des capsules fines. Le temps t=0 est le moment de la confluence. La brusque augmentation du $R_{MCS}$ au cours des derniers stades correspond à l'éclatement de la capsule. Le sphéroïde croît librement à une vitesse similaire à celle observée lors des tout premiers stades suivant l'encapsulation.
- la Figure 15 (A) Représentation schématique d'une coupe transversale médiane d'un injecteur deux voies (B) Représentation schématique d'une coupe transversale médiane d'un injecteur trois voies.

## EXEMPLES

### Exemple 1 (pas selon l'invention)

***Conditions expérimentales d'obtention de capsules gélifiées et rigidifiées***

Dispositif expérimental

Le procédé de préparation de capsules est basé sur la co-extrusion concentrique de compositions via un dispositif à triple enveloppe pour former des gouttes multi-composante (figure 15B).
Une première composition (C1) circulant dans un premier compartiment 21 d'une triple enveloppe constitue le premier flux.
Une deuxième composition (C2) circulant dans un second compartiment 31 de la triple enveloppe constitue le deuxième flux.
Une troisième composition (C3) circulant dans un troisième compartiment 41 de la triple enveloppe constitue le troisième flux.

Formation de capsules gélifiées et rigidifiées

A la sortie de la triple enveloppe, se forme alors une goutte multi-composante, le premier flux constituant le coeur liquide, le deuxième flux constituant l'enveloppe intermédiaire liquide et le troisième flux constituant l'enveloppe externe liquide de la goutte multi-composante.
La taille du coeur liquide, l'épaisseur de l'enveloppe intermédiaire et de l'enveloppe externe des capsules formées sont contrôlées par l'utilisation de plusieurs pousse-seringues indépendants, réglant les débits d'injection des différentes compositions C1, C2 et C3.
Le débit Q1 de la composition C1 est réglé à 10 mL/h.
Le débit Q2 de la composition C2 est réglé à 1 mL/h.
Le débit Q3 de la composition C3 est réglé à 1 mL/h, et peut être diminué jusqu'à 0,1 mL/h.
Chaque goutte multi-composante se détache de la triple enveloppe et chute dans un volume d'air, avant d'être immergée dans une solution gélifiante de lactate de calcium concentrée à 1M.
Une fois l'enveloppe externe gélifiée, les capsules gélifiées formées sont rincées dans une solution de rinçage à base d'eau, puis sont immergées dans un bain de rigidification.

Formation de capsules rigidifiées

Les capsules gélifiées et rigidifiées ainsi formées sont ensuite immergées dans une solution de dépolymérisation de citrate concentrée à 10%.
Une fois l'enveloppe externe dépolymérisée et éliminée, les capsules rigidifiées obtenues sont rincées dans une solution de rinçage à base d'eau et stockées dans une solution de stockage à base d'eau.

### Exemple 2 : Capsules à double enveloppe à base de latex naturel /alginate

La composition C1 est une solution aqueuse de colorant amaranthe à 1 mM.
La composition C2 est une dispersion aqueuse de latex naturel (nom chimique Cis 1,4-polyisoprène, famille des diènes, exemple de latex naturel commercial: natural Rubber grade TSR, SRM, SIR, STR, SVR, ADS, RSS, Crepes, DPNR, de chez Astlett Rubber Inc.) diluée jusqu'à une fraction massique de particules de polymères de 20% à 40% par rapport à la masse totale de la dispersion de latex naturel, comprenant également 1% en masse de tensioactif de type ionique ou non ionique selon le grade.
Dans cet exemple, on fixe la fraction massique particules de polymères à 30% (la dispersion de latex est titrée

par gravimétrie après un lavage par centrifugation) et on utilise le tensioactif SDS (dodécylsulfate de sodium).

La composition C3 est une solution aqueuse possédant un pourcentage massique d'alginate de sodium de 2,0% et un pourcentage massique de SDS de 0,1%.

Les capsules obtenues, d'un diamètre classique de quelques millimètres, sont maintenues dans la solution gélifiante d'ions calcium une minute, puis sont rincées avec de l'eau distillée. Elles sont stockées ensuite dans une solution isotonique à la solution interne. On obtient ainsi la double coacervation par perméation des ions calcium à travers l'enveloppe d'alginate gélifiée. Les capsules peuvent ensuite être incubées 10 minutes dans une solution de citrate à 10% afin de dissoudre la membrane extérieure d'hydrogel d'alginate. On obtient ainsi des capsules présentant une enveloppe extérieure de latex naturel rigidifiée.

**Exemple 3 Capsules à double enveloppe latex naturel/CB alginate**

L'Exemple 3 est réalisé dans les mêmes conditions que l'Exemple 2, sauf que la composition C2 comprend en outre du noir de carbone « CB : Carbon Black ». Pour ce faire on prépare une solution de CB (ex Noir de Carbone N234 de chez CABOT Corporation) en présence de 2% de tensioactif SDS, la fraction massique de particules de polymères étant toujours comprise entre 20% et 40 % par rapport à la masse totale de la dispersion de latex naturel, la fraction de CB étant comprise de 1% à 15%.

Pour cet exemple, on fixe la fraction massique de particules de polymères à 30% et la fraction massique de CB à 5% par rapport à la masse totale de la composition C2.

Apres gélification de l'enveloppe d'alginate les capsules sont incubées dans de l'eau distillée pendant environ 20 minutes. Le tensioactif diffuse vers l'extérieur des capsules au travers de l'enveloppe d'alginate, et provoque la coacervation du mélange mixte latex naturel/CB, donnant naissance à une enveloppe rigidifiée de caoutchouc renforcé.

**Exemple 4 Capsules à double enveloppe latex naturel/silice colloïdale et alginate**

L'Exemple 4 est réalisé dans les mêmes conditions que l'Exemple 2, sauf que la composition C2 comprend en outre de la silice colloïdale de diamètre moyen 100 nm (aerosil de chez Degussa, Ludox chez Sigma), selon une fraction massique de 1% à 15% par rapport à la masse totale de la composition C2.

Pour cet exemple, on fixe la fraction massique de particules de polymères à 30% et la fraction massique de silice colloïdale à 5% par rapport à la masse totale de la composition C2.

On obtient ainsi des capsules comportant une enveloppe rigidifiée de caoutchouc renforcé.

Les capsules préparées selon l'invention sont faciles à former, elles présentent une enveloppe résistante, de faible épaisseur, qui permet d'assurer la désagrégation efficace de la capsule lorsque le liquide contenu dans la capsule doit être libéré.

**Exemple 5 : Capsules à simple enveloppe à base d'alginate (pas selon l'invention)**

*I. Méthodes*

I.A. Fabrication du dispositif de co-extrusion

L'unité centrale des dispositifs microfluidiques consiste en trois tubes capillaires en verre co-alignés dans le plan axial. Le capillaire effilé le plus externe est obtenu en étirant un capillaire arrondi en coupe transversale (Vitrocom, diamètre interne (d.i.) de 600 $\mu$m, diamètre externe (d.e.) de 840 $\mu$m) avec une étireuse de micropipettes (P2000, Sutter Instrument). Le capillaire le plus interne (d.i. de 100 $\mu$m, d.e. de 170 $\mu$m) et intermédiaire (d.i. de 300 $\mu$m , d.e. de 400 $\mu$m) ont été maintenus selon une forme cylindrique et ont été coupés à la longueur souhaitée. Les extrémités des capillaires ont été polies avec des films micro-abrasifs (grain de 1 $\mu$m, 3M) afin d'éviter une quelconque forme de biseau générant des perturbations de l'écoulement et pour obtenir le diamètre de pointe souhaité (typiquement entre 130 et 180 $\mu$m). Un revêtement hydrophobe (1H, 1H,2H,2H-perfluorooctyltriméthoxysilane, ABCR) a été appliqué sur les parois des capillaires conformément à des protocoles standard (Perret, E., et al. Langmuir 18, 846.-854 (2002)) afin d'empêcher toute humidification des parois externes de la pointe de l'injecteur par la solution d'alginate. L'assemblage du dispositif de co-extrusion a été réalisé sous un microscope binoculaire. Le capillaire le plus externe a d'abord été collé à une lame de verre, qui sert de support au dispositif. Ensuite, les deux autres capillaires cylindriques ont été insérés et scellés de manière séquentielle en utilisant une résine époxy (Loctite 3430, Radisopares-RS Components). Les alignements coaxiaux et longitudinaux ont été manuellement contrôlés pendant le séchage de la résine à température ambiante. Les orifices d'entrée de la puce ont été fabriqués en collant les raccords d'aiguilles de seringue à embout mousse (NN-1950R, Terumo) au sommet des extrémités libres des capillaires.

I.B. Fonctionnement du dispositif de co-extrusion.

Les trois phases liquides (suspension cellulaire CS, solution intermédiaire IS et solution d'alginate AL - voir la Figure 6) ont été chargées dans des seringues (10MDR-LL-GT SGE, Analytical Science) dotées d'aiguilles raccordées à des tubulures en Téflon (Bohlender, diamètre interne de 0,5 mm). Les autres extrémités des tubulures ont été insérées dans les orifices d'entrée appropriés du dispositif de co-extrusion, qui est clampé verticalement à un montant à l'intérieur d'une hotte à flux laminaire. Les seringues ont été montées sur des pompes à seringue (PHD 4400, Harvard Apparatus) qui contrôlent l'injection des liquides aux débits souhaités. Dans ces travaux, les inventeurs ont principalement utilisé deux ensembles de débits : 1) pour les capsules fines : $q_{CS}$=50 ml $h^{-1}$, $q_{IS}$=50 ml $h^{-1}$, $q_{AL}$=40 ml $h^{-1}$, et 2) pour les capsules épaisses : $q_{CS}$=20 ml $h^{-1}$, $q_{IS}$=20 ml $h^{-1}$, $q_{AL}$=30 ml $h^{-1}$. Après l'initiation des débits, les microgouttelettes de composé sont dirigées vers un bain de gélification contenant du chlorure de calcium 100 mM (VWR) et des traces du tensioactif Tween 20 (Merck), et sont placées à approximativement 0,5 m en dessous de l'orifice de sortie du dispositif. Un fonctionnement pendant quelques secondes ont été suffisant pour produire quelques $10^4$ capsules, qui ont été immédiatement lavées dans une solution de sorbitol iso-osmotique et ont été transférées dans le milieu de culture approprié. Après utilisation, le dispositif microfluidique a été nettoyé avec un désinfectant (Biocidal ZF, Biovalley), de l'éthanol et de l'eau désionisée. Avant l'utilisation suivante, la puce a été rincée avec une solution de sorbitol.

I.C. Préparation de solutions aqueuses et de suspensions cellulaires.

La phase la plus externe (solution AL) a été préparée par la dissolution d'alginate de sodium à 2,5 % p/v (FMC, Protanal LF200S) dans de l'eau, et en ajoutant 0,5 mM de tensioactif dodécylsulfate de sodium (SDS) (VWR). La solution a été filtrée à 1 $\mu$m (Pall Life Science) et a été stockée à 4 °C. La phase intermédiaire (IS) est généralement une solution de sorbitol 300 mM (Merck). La phase la plus interne (CS) a été obtenue en détachant les cellules des parois du flacon de culture avec de la trypsine-EDTA à 0,5 % (Invitrogen). Après un lavage dans le milieu de culture approprié et une centrifugation délicate (300 x g, 5 minutes, 20 °C), elles ont été remises en suspension dans une solution de sorbitol 300 mM à une concentration approximative de 3 x $10^6$ cellules par ml.

I.D. Lignées cellulaires, monocouche et cultures de cellules encapsulées.

Les inventeurs ont utilisé des cellules de carcinome du côlon murin de type sauvage CT26 (achetées auprès de l'American Tissue Culture Collection, ATCC CRL-2638) et des cellules CT26 stablement transfectées avec du LifeAct-mCherry. Des tests ont également été réalisés avec des cellules HeLa et des cellules de sarcome murin (S180, aimable don de Chu Yeh-Shiu, IMCB, Singapour).

Toutes les cellules ont été maintenues dans un milieu de Eagle modifié selon Dulbecco (DMEM, Invitrogen) supplémenté avec du sérum de veau foetal à 10 % (FBS, Invitrogen) et des antibiotiques (100 $\mu$g $ml^{-1}$ de streptomycine et 100 unités $ml^{-1}$ de pénicilline (Gibco BRL) dans une atmosphère humidifiée contenant du $CO_2$ à 5 % à 37 °C en remplaçant le milieu tous les 2 jours. Les cellules ont été cultivées sous forme de monocouches sous-confluentes afin de préparer les suspensions cellulaires utilisées pour l'encapsulation dans des sphères creuses d'alginate.

Une fois que les capsules cellulaires ont été formées en suivant le protocole décrit ci-dessus, elles ont été placées à l'intérieur d'un incubateur (37 °C, $CO_2$ à 5 %, environ 100 % d'humidité relative). Parmi les milliers de capsules cellulaires collectées, la majorité a été maintenue dans une boîte de Pétri contenant un milieu de culture et ont été cultivées dans les mêmes conditions que les monocouches cellulaires. Dans chaque cycle d'encapsulation, plusieurs dizaines de capsules ont été sélectionnées pour une imagerie haute résolution. En fonction des exigences de configuration du microscope et de la durée de la session d'imagerie souhaitée (de quelques heures à quelques semaines), ces capsules cellulaires sélectionnées ont été transférées dans des chambres de cultures ou des dispositifs dédié(e)s (voir la description ci-dessous).

À titre de comparaison avec notre méthode, des sphéroïdes CT26 ont également été cultivés selon la technique sur lit d'agarose classique (Hirschhaeuser, F. et al. J. Biotechnol. 148, 3.-15 (2010)).

I.E. Coloration des sphéroïdes fixés.

Les sphéroïdes ont été fixés dans du PFA à 4 % dans du PBS pendant 1 heure à température ambiante. Pour la coloration de l'actine corticale, ils ont été incubés avec 0,5 $\mu$g $ml^{-1}$ de phalloïdine conjuguée à de l'Alexa488 (Molecular Probes) dans une solution de PBS avec du Triton-X100 à 1 % v/v (Sigma) à 4 °C pendant 2 heures à toute une nuit. Un marquage immunologique de la fibronectine a été réalisé selon une procédure standard. En bref, les sphéroïdes ont été perméabilisés en utilisant du Triton-X100 à 2 % v/v dans du PBS. Les anticorps primaires (anticorps anti-fibronectine produit chez un lapin, Sigma) et secondaires (IgG de chèvre anti-lapin (H+L) conjuguée à de l'Alexa Fluor 568, Invitrogen) ont été dilués à 1/250 puis ont été incubés pendant 12 heures.

I.F. Évaluation de la viabilité cellulaire et numération cellulaire.

Afin d'évaluer l'efficacité de l'encapsulation et la concentration de l'ensemencement cellulaire, des images en contraste de phase des capsules ont été prises immédiatement après l'encapsulation et le nombre de cellules par capsule a été estimé en utilisant des plugins standard ImageJ (Schneider, C. A., et al. Nat. Methods 9, 671.-675 (2012)).

La viabilité cellulaire a été caractérisée à différents stades de la culture cellulaire encapsulée avec des colorants pour cellules vivantes/mortes calcéine AM/éthidium homodimère-1 (EthD-1) (Invitrogen). Afin d'évaluer la nocivité potentielle du procédé d'encapsulation sur les cellules en suspension, les inventeurs ont incubé les capsules cellulaires immédiatement après la formation avec de l'EthD-1 à 4 $\mu$M et de la calcéine AM à 2,5 $\mu$M pendant 30 minutes à 1 heure. Le nombre de cellules viables a été déterminé en comptant les cellules rouges (mortes) et vertes (vivantes) en utilisant un microscope à épifluorescence inversé (Axiovert-200M, Carl Zeiss) équipé d'une lampe Hg et d'une caméra EM CCD (C 9100-02, Hamamatsu Photonics). À titre de comparaison, les mêmes mesures ont été réalisées sur la suspension cellulaire avant l'encapsulation. Lorsque les sphéroïdes se sont formés et ont subi une expansion au sein des capsules, le même protocole a été adapté en augmentant les concentrations des colorants de dix fois et les durées d'incubation de 2 à 4 heures. Les plans équatoriaux des sphéroïdes ont ensuite été visualisés sous forme d'images par microscopie confocale. Alors que les cellules rouges (mortes) pouvaient être détectées dans le noyau du sphéroïde, les cellules vivantes étaient pratiquement non colorées au-delà d'une bordure périphérique de quelques couches. Ce faible marquage interne est dû au fait que l'activité estérase intracellulaire ubiquitaire des cellules périphériques est suffisante pour estérifier toutes les molécules de calcéine AM perméables aux cellules avant qu'elles ne puissent pénétrer plus profondément dans le sphéroïde.

I.G. Imagerie de la croissance des sphéroïdes à long terme et de la déformation des capsules.

La croissance des MCS à l'intérieur des capsules et la déformation de la coque ont été surveillées par microscopie à contraste de phase. Pour toutes les sessions d'encapsulation, 24 capsules ont été sélectionnées à partir du lot entier de capsules cellulaires et ont été individuellement transférées dans chaque puits d'une plaque de culture multipuits (Falcon). Chaque capsule a été visualisée sous forme d'images toutes les 3 heures avec un microscope inversé Nikon EZ (objectifs à sec 10x/0,25NA ou 20x/0,4NA) équipé d'une caméra couleur CCD (Nikon Digital sight DS Fi1) pilotée par le logiciel NIS Element. La capture des images a été réalisée à température ambiante et a duré approximativement 5 minutes. Entre chaque acquisition, la plaque à 24 puits contenant les capsules a été maintenue dans l'incubateur. La moitié du milieu de culture a été renouvelée tous les 2 jours. Pour l'acquisition des séquences en temps réel à une résolution temporelle plus élevée (1 séquence toutes les 5 minutes) sur des périodes de temps prolongées (environ 15 jours), les inventeurs ont également utilisé un microscope inversé (Nikon Eclipse Ti, objectif à sec 10x/NA0,3) équipé d'une platine motorisée (Märzhäuser) et d'un système de contrôle du climat (The Brick, Life Imaging Systems). Le microscope et la caméra (CoolSNAP HQ$^2$, Photometrics) ont été pilotés par le logiciel Metamorph (Molecular Devices). Afin d'empêcher tout déplacement ou dérive des capsules dans le puits en dehors du champ de visualisation, les inventeurs ont conçu une chambre d'observation fabriquée sur mesure. Des puits de Phytagel (Sigma) de type entonnoir ont été préparés en utilisant des moules coniques en PDMS (élastomère polydiméthylsiloxane, Sylgard-184, Dow Corning) adaptés aux puits d'une plaque à 24 puits à fond en verre (Radnor, PA). Cette configuration facilite le chargement des capsules individuelles, qui sont dirigés vers le centre du puits. Les orifices (500 $\mu$m de diamètre) dans les répliques de phytagel, qui servent principalement de micro-conduit, se sont également avérés efficaces pour limiter les mouvements des sphéroïdes encapsulés, et ce sans induire de contraintes qui pourraient altérer la croissance du MCS.

I.H. Imagerie de l'organisation cellulaire en 3D des sphéroïdes encapsulés.

Afin de visualiser les couches cellulaires périphériques et le noyau des sphéroïdes en expansion à une résolution subcellulaire, les inventeurs ont utilisé la microscopie confocale par fluorescence monophotonique ou multiphotonique.

Une imagerie confocale des cellules vivantes a été réalisée en utilisant un microscope inversé (LSM710, Carlz Zeiss) équipé d'une chambre de régulation du climat (Pecon) contrôlant le pourcentage de $CO_2$, la température et l'humidité. Les échantillons ont été préparés en immergeant les capsules dans une solution d'agarose à bas point de fusion à 0,3 % (Invitrogen) (milieu de culture exempt de sérum, 37 °C) dans une boîte de Pétri à fond en verre fabriquée sur mesure (diamètre des puits d'environ 2 mm). Après une gélification de granules d'agarose (10 minutes, température ambiante), la boîte de Pétri a été remplie avec le milieu de culture. Ce montage a permis d'immobiliser les capsules, une étape nécessaire pour l'acquisition d'images par le mode « z-stack acquisition » (acquisition automatisée de plusieurs images XY le long de l'axe Z). Le pourcentage d'agarose a été choisi pour générer un stress minimal sur les MCS en expansion. Une comparaison de la cinétique de croissance entre les MCS se déplaçant librement et les MCS incorporés dans l'agarose n'a révélé aucune différence significative. Afin de surveiller la dynamique cellulaire au sein du sphéroïde, nous avons utilisé la lignée cellulaire CT26 stablement transfectée avec du LifeAct-mCherry ou des cellules CT26 de

type sauvage incubées dans du FM4-64 (Invitrogen, 2 $\mu$g $ml^{-1}$). La fluorescence a été acquise en utilisant un laser pompé par diode à l'état solide à 561 nm (15 mW) et un objectif à immersion dans l'huile 25x/0,80NA. Les images de la surface des sphéroïdes fixés colorés avec la phalloïdine-Alexa488 ont été visualisées avec un laser argon à 488 nm (25 mW) et un objectif à immersion dans l'huile 63x/1,4. Les images individuelles et les piles d'images ont été traitées en utilisant le logiciel Zen 2011 (Carl Zeiss) et ImageJ ou Fiji (Schindelin, J. et al. Nature Methods 9, 676.-682 (2012)). Des vidéos en ligne ont été éditées en utilisant After Effects puis ont été compressées en utilisant Media Encoder (Adobe).

Une microscopie multiphotonique a été réalisée afin d'accéder au noyau des sphéroïdes encapsulés. Deux types de microscopes ont été utilisés : 1) un microscope à balayage laser biphotonique vertical (Lavision) équipé d'un objectif à immersion dans l'eau 20x/0,95NA (Olympus) ; 2) un microscope inversé LSM710 NLO (Carl Zeiss) équipé d'objectifs à immersion dans l'huile 25x/0,80NA ou à immersion dans l'eau 40x/1NA (Carl Zeiss). Les configurations ont été couplées à des lasers femtoseconde (690-1020 nm, de Coherent ou de Spectra Physics). Les images de l'intérieur des sphéroïdes fixés colorés par la phalloïdine-Alexa488 ont été acquises à une longueur d'onde laser de 920 nm. De la sulforhodamine B (SRB, Sigma) a été ajoutée au milieu à la concentration de 40 $\mu$g $ml^{-1}$. Les meilleures conditions pour une imagerie vivante des sphéroïdes dans un milieu de culture supplémenté avec de la SRB ont été obtenues pour une excitation à 800 nm (Marmottant, P. et al. Proc. Natl. Acad. Sci. U.S.A. 106, 17271.-17275 (2009)). Les capsules ont été montées tel que décrit pour l'imagerie vivante confocale monophotonique.

I.I. Mesures morphométriques des capsules.

La caractérisation des tailles et des formes des capsules a été déterminée sur des capsules contenant des cellules et sur des capsules vides. Les mesures sur les capsules vides, qui ont été obtenues en remplaçant la phase CS par une solution de sorbitol iso-osmotique, ont été réalisées immédiatement après l'encapsulation et au bout d'une semaine de séjour dans le milieu de culture à 37 °C (afin de tenir compte des potentielles modifications morphologiques induites par le vieillissement). Aucune différence significative n'a été observée entre ces diverses conditions. Les images de grands champs de visualisation de capsules densément regroupées ont été acquises par microscopie à contraste de phase et ont été analysées en utilisant ImageJ. Le rayon moyen de la capsule a été défini comme : $R = \sqrt{S/\pi}$ , où S est la surface transversale équatoriale de la capsule. La circularité de la capsule a été mesurée en tant que rapport de l'axe mineur sur l'axe majeur de l'ellipse ajustée à la bordure externe de la coupe équatoriale projetée.

Lorsque les sphéroïdes sont à confluence, les parois externe et interne de la capsule peuvent être facilement détectées en raison du contraste optique élevé. En revanche, pour les capsules vides ou partiellement remplies, la paroi interne de la capsule sont faiblement visible par microscopie à contraste de phase. Les mesures de l'épaisseur de la capsule ont donc été réalisées en dopant la solution d'alginate avec 250 $\mu$g/ml de FITC-dextran de haut poids moléculaire (2 MDa, Sigma). Les images des capsules ont été acquises par microscopie confocale et ont été analysées avec ImageJ. L'influence de bas débits sur le rapport d'aspect $h/R_{out}$ a été évaluée en comparant les données expérimentales avec la valeur théorique calculée à partir de la conservation du volume :

$$\frac{h}{R_{out}} = 1 - \left( \frac{q_{in}/q_{out}}{1 + q_{in}/q_{out}} \right)^{1/3}$$

I.J. Mesures de l'élasticité des gels d'alginate.

Trois méthodes différentes ont été utilisées pour mesurer le module de Young des gels d'alginate.

Les inventeurs ont d'abord réalisé des mesures de micro-indentation par AFM sur des capsules vides.

Des capsules d'alginate disposées au fond d'une boîte de Pétri remplie d'un milieu de culture ont été placées sur la platine à échantillons d'un système AFM Catalyst (Bruker) monté sur un microscope optique inversé (IX71, Olympus) en mode force (Figure 9A). Les inventeurs ont utilisé des porte-à-faux TR400 attachés à des billes sphériques de $SiO_2$ (diamètre de 5 $\mu$m) et ayant une constante de raideur nominale $k_{cantilever}$ = 0,06 N/m (Novascan). La sensibilité des photodiodes a été étalonnée avant et après des mesures sur une surface de mica fraîchement clivé dans du PBS. La constante de raideur a été déterminée en utilisant la méthode des fluctuations thermiques mise en oeuvre dans le logiciel Bruket Nanoscope 7.2. Les courbes force-distance (F-z) ont été enregistrées pour des déplacements d'une amplitude pic à pic d'environ 2 $\mu$m à 0,25-1 Hz. Le seuil de déflexion relative a été contrôlé pour atteindre une déformation de capsule comprise entre 200 nm et 500 nm. Les données ont été analysées dans le cadre d'une indentation de la charge ponctuelle dans des sphères creuses. La relation force fonctionnelle (F) - déformation ($\delta$) (Fery, A. & Weinkamer,

R. Polymer 48, 7221.-7235 (2007)) est la suivante : $$F = \frac{4}{3\sqrt{1-\nu^2}} E \frac{h^2}{R} \delta .$$ La déformation a été calculée en termes de point de contact ($Z_c$) et de décalage de la déflexion ($d_0$) en tant que $d = z\text{-}z_c\text{-}(d\text{-}d_0)$. De manière expérimentale, le module de Young du gel d'alginate a été dérivé de l'ajustement des traces force-déformation (Figure 9A) en prenant les valeurs mesurées pour les propriétés géométriques ($R$ et $h$) de la capsule et $\nu$=0,5 pour le rapport de Poisson. Les inventeurs ont observé que E= 55±44 kPa (±SD, N=7).

La deuxième méthode consiste à réaliser des mesures de la traction sur des cylindres de gel d'alginate macroscopiques de type spaghetti. Ces fils (longueur $L_0$ d'environ 0,2 m, diamètre $D_0$ d'environ 1 mm) ont été formés avec un simple dispositif d'extrusion à 1 voie doté d'un embout d'une taille d'environ 1 mm, en immergeant la pointe dans le bain de calcium afin de supprimer l'instabilité du capillaire. Un stress contrôlé $\sigma$ a été appliqué avec un ensemble de poids calibrés m suspendus aux cylindres d'alginate. L'élongation $\Delta L/L_0$ de l'échantillon d'alginate a été mesurée avec une règle. En supposant que $\nu$=0,5, le module de Young a été dérivé à partir de $$\sigma = \frac{4mg}{\pi D_0^2 (1-\Delta L/L_0)} = E.\frac{\Delta L}{L_0} .$$ Les inventeurs ont observé $E$=71 ± 12 kPa (±SD, N=9).

Une troisième détermination de $E$ est basée sur un essai de gonflement osmotique. À cette fin, les inventeurs ont remplacé la suspension cellulaire par une solution de sorbitol avec du dextran à 5 % p/v, $P_m$=2 MDa et 500 kDa (Sigma Biochemika). La solution du bain de calcium et le milieu de culture de stockage ont également été supplémentés avec du dextran à 5 % p/v. L'équilibre iso-osmotique de toutes les solutions a été contrôlé. Afin d'obtenir un gonflement détectable, des capsules à parois très fines ont été préparées ($q_{in}/q_{out}$=10, ce qui correspond à une épaisseur de coque h d'environ 5-7 µm). Une dilution par étapes du dextran a entraîné le gonflement osmotique des capsules. Les différences de concentration dans le dextran ont été converties en pressions osmotiques $\pi_0$ et la dilatation des capsules $\Delta R/R_0$ a été directement mesurée. Au premier ordre, dans la limite d'une légère déformation, le module de Young de l'alginate a été dérivé en équilibrant l'énergie élastique de la coque sphérique et l'effet obtenu par la différence de pression osmotique :

$$E \approx \frac{1}{4ho/Ro} \cdot \left( \frac{\Delta R/R o}{\pi o} \right)^{-1}$$

I.K. Détermination du module de Young d'un gel d'alginate d'après le gonflement osmotique d'une capsule

Considérant une capsule sphérique constituée d'une coque d'alginate renfermant une solution de dextran de haut poids moléculaire ($P_m$ = 500 kDa ou 2 MDa) immergée dans une solution de dextran moins concentrée, étant donné que la coque est perméable à l'eau (porosité estimée d'environ 6 nm) mais pas au dextran (rayons de Stockes d'environ 15 nm et 27 nm), les molécules d'eau se diffusent dans la capsule, qui gonfle jusqu'à ce que la force élastique de la capsule étirée équilibre la pression osmotique.

Au début de l'essai, les concentrations de dextran à l'intérieur et à l'extérieur de la capsule sont équivalentes. Le rayon initial de la capsule est $R_0$. Ensuite, la concentration de dextran dans le bain externe est réduite par dilution, de sorte que la différence de concentration soit $c_0$. Lors du gonflement, le rayon de la capsule augmente de $\Delta R = R\text{-}R_0$ et la différence de concentration est réduite de $c_0$ à $c$ :

$$c = c_0 \cdot \left( \frac{R_0}{R} \right)^3 \qquad (1).$$

L'énergie élastique d'étirement est donnée par (Landau, L. D., et al. Theory of Elasticity, Third Edition: Volume 7. (Butterworth-Heinemann: 1986)) :

$$G_{el} = 4\pi \frac{E}{1-\nu} h (R - R_0)^2 \qquad (2),$$

où $h$ est l'épaisseur de la coque et $\nu$ est le rapport de Poisson. Pour un matériau incompressible, $\nu = 1/2$ et la coque devient plus fine lorsque la capsule gonfle, selon :

$$h = h_o.\left(\frac{R_o}{R}\right)^2 \quad (3),$$

où $h_0$ est l'épaisseur de la capsule non étirée.

Étant donné que les solutés sont très volumineux, la pression osmotique $\Pi$ dévie de manière significative par rapport à la valeur nominale ($\Pi = nk_BT$, où n est le nombre d'espèce actives d'un point de vue osmotique et $k_BT$ l'énergie thermique) et s'avère être indépendante de leur osmolalité nominale au-delà d'un seuil donné ($P_m$ = 200 kDa pour le dextran) (Reid, C. & Rand, R. P Biophys J 73, 1692-1694 (1997)). Différentes expressions empiriques sont rapportées pour ajuster les données de la pression osmotique (Veretout, F Journal of molecular biology 205, 713-728; Bonnet-Gonnet, C. et al. Langmuir 10, 4012-4021 (1994)). À des fins de simplicité, nous considérons l'expression polynomiale bien établie pour $\Pi$ en fonction de c (en pourcentage de poids/volume) :

$$\Pi = \alpha c + \beta c^2 + \gamma c^3 \quad (4),$$

où $\alpha = 286$, $\beta = 57$ et $\gamma = 5$. L'effet généré par la pression osmotique pour gonfler la capsule de $R_0$ à R est donné par :

$$W = \int_{R_o}^{R} \Pi.4\pi R^2 dR \quad (5).$$

En tenant compte de l'effet de dilution (Eq. 1), nous obtenons :

$$W = 4\pi R_0^3\left(\alpha c_0 \ln\left(\frac{R}{R_0}\right) + \frac{1}{3}\beta c_0^2\left(1-\left(\frac{R_0}{R}\right)^3\right) + \frac{1}{6}\gamma c_0^3\left(1-\left(\frac{R_0}{R}\right)^6\right)\right) \quad (6).$$

Le rayon de la capsule à l'équilibre est indiqué par le minimum de l'énergie totale $G_{el} + W$. En supposant en outre de petites déformations, $\Delta R/R_0 \ll 1$, nous arrivons à :

$$\frac{\Delta R}{R_0} = \frac{\Pi_0}{\Pi_c + 4E(h_0/R_0)} \quad (7),$$

où $\Pi_0$ est la pression osmotique à $c_0$, et $\Pi_c = \Pi_0 + 3\beta c_0^2 + 6\gamma c_0^3$.

Ceci révèle que la pression osmotique $\Pi_0$ varie de 0 à 4 kPa au sein de la plage de différences de concentrations explorée. L'approximation indiquée ci-dessus repose sur la supposition que la correction introduite par $\Pi_c$ reste négligeable par rapport au module de Young effectif $E \times 4h_0/R_0$. En prenant $E$ = 68 kPa et $h_0/R_0$ environ 0,05, ceci est uniquement valable pour $c_0 < 2$ %. Dans nos conditions expérimentales ($c_0$ variant de 0 à pratiquement 5 %), une détermination plus précise de E exige d'utiliser l'Eq. 7.

I.L. Analyse de la croissance des sphéroïdes et de la déformation des capsules.

Les images à contraste de phase en temps réel ont été analysées en utilisant un algorithme de détection des bordures basés sur le gradient et fabriqué sur mesure mis en oeuvre dans Matlab (MathWorks). En commençant à partir du centre de la capsule, les profils d'intensité ont été acquis en position radiale et ont été inspectés pour identifier les pics dans la première dérivée afin d'extraire le contour du MCS et de la capsule le renfermant dans chaque structure enregistrée. $R_{out}$ a été dérivé de la surface transversale projetée. Une approche similaire a été suivie pour surveiller le $R_{MCS}$ à l'intérieur de la capsule. Le bruit de fond détecté avant la confluence était principalement dû aux mouvements

de rotation de l'agrégat cellulaire non parfaitement sphérique. Le temps de confluence (t=0) a été déterminé comme le temps pour lequel la croissance des MCS exhibe un point d'inflexion. Les inventeurs ont vérifié que ce temps coïncidait, en moins de 5 minutes, avec la détermination visuelle de la confluence (sur des vidéos à haute résolution temporelle). Les stades pré- et post-confluence ont également été quantifiés par un paramètre de rugosité, $\rho = P/2\sqrt{\pi A}$, P et A étant respectivement le périmètre et la surface d'une coupe transversale équatoriale. Alors que l'évolution temporelle de ρ présente un bruit de fond au cours des premiers stades de la croissance des MCS, elle diminue lorsque le sphéroïde se rapproche de la paroi de la capsule, avant qu'elle ne soit saturée à une valeur minimale proche de la valeur théorique de 1 pour des objets parfaitement sphériques.

I.M. Approche phénoménologique pour l'élasticité non linéaire des capsules d'alginate à d'importantes déformations

Afin de confirmer la mesure du module de Young dérivée de l'essai de gonflement osmotique, les inventeurs ont mis au point un deuxième essai mécanique, consistant à évaluer directement la relation stress (σ)-contrainte (ε) des fils de gel d'alginate. Ces fils (1 mm de diamètre) ont été étirés avec des poids calibrés auxquels étaient soudées de minuscules gouttelettes d'alginate à une extrémité. Dans le régime à faible déformation (typiquement pour l'élongation relative $\varepsilon = \Delta L/L_0 < 10$ %), la réponse de stress-contrainte est linéaire et le module de Young dérivé est tout à fait conforme à celui précédemment mesuré ($E = 71 \pm 12$ kPa). En cas de déformation très importante (> 80 %), l'eau perlait de l'échantillon et une plasticité significative était manifeste. Pour la déformation intermédiaire, le matériau présente une réponse de stress-contrainte non linéaire (Figure 9B). Un tel comportement de durcissement à une contrainte est très courant pour les gels de biopolymère et a déjà été rapporté pour des gels d'alginate (Zhang, J., et al. 2007 Journal of Food Engineering 80, 157-165). Étant donné que les capsules fines ($h/R$ environ 0,1) qui ont été considérablement utilisées dans ces travaux exhibent une déformation radiale maximale $\Delta R/R_0$ d'environ 30 % avant l'éclatement, une détermination précise de la pression exercée par le sphéroïde confiné en expansion exigeait de tenir compte de cet effet. Une approche phénoménologique classique pour l'élasticité non linéaire consiste à considérer un terme correctif dans $\varepsilon^2$ pour le stress ($\sigma = E\varepsilon + A\varepsilon^2$). À l'inverse, en ajustant les données σ-ε avec une expression polynomiale de second ordre, les inventeurs défini un module élastique effectif dépendant de la contrainte $E_{eff}(\varepsilon) = E(1+a\varepsilon)$ et avons observé $a = 1{,}5$. Nous avons utilisé cette expression pour $E$ afin de dériver la pression à partir des données de déformation sur les capsules fines.

I.N. Dilatation d'un récipient sphérique à parois épaisses soumis à une pression interne

Les inventeurs ont supposé que le gel d'alginate est isotrope et que les contraintes sont faibles (c'est-à-dire < 10%). En revanche, si la condition $h/R \ll 1$ n'est pas satisfaite, la supposition d'un stress tangentiel constant à travers l'épaisseur du récipient n'est pas valable. Dans le cas général d'un rapport de Poisson $\nu \neq 1/2$, les inventeurs ont dû faire appel aux expressions pour le stress radial et circonférentiel (Fung, Y. C. Foundations of Solid Mechanics ; Prentice Hall: 1965) :

$$\sigma_r = \frac{PR_{in}^3}{R_{out}^3 - R_{in}^3}\left(1 - \frac{R_{out}^3}{R^3}\right) \quad (1)$$

$$\sigma_\varphi = \frac{PR_{in}^3}{R_{out}^3 - R_{in}^3}\left(2 + \frac{R_{out}^3}{R^3}\right) \quad (2),$$

où $R_{in} \leq R \leq R_{out}$.

Le déplacement radial $u(R)$ est obtenu à partir de la loi de Hooke :

$$u(R) = \frac{(1-\nu)\sigma_r - \nu\sigma_\varphi}{E} R \quad (3).$$

En collectant ces résultats, les inventeurs sont arrivés à :

$$u(R)=\frac{P}{E}\frac{PR_{in}^{3}}{R_{out}^{3}-R_{in}^{3}}\left[(1-2\nu)R+\frac{(1+\nu)}{2}\frac{R_{out}^{3}}{R^{2}}\right] \quad (4).$$

Si le matériau est incompressible, cette équation peut être simplifiée et l'appliquée pour deux cas d'intérêt particuliers, notamment $R=R_{in}$ et $R=R_{out}$ :

$$u(R_{in})=\frac{3}{4}\frac{P}{E}\frac{R_{in}}{1-(R_{in}/R_{out})^{3}} \quad (5),$$

$$u(R_{out})=\frac{3}{4}\frac{P}{E}\frac{R_{in}}{(R_{out}/R_{in})^{3}-1} \quad (6).$$

Finalement, à partir de la conservation du volume de la coque, on a :

$$R_{out}^{3}(t)-R_{in}^{3}(t)=R_{out}^{3}(0)-R_{in}^{3}(0)=\Delta(R_{0}^{3}) \quad (7).$$

En utilisant cette équation, les deux variables temporelles $R_{in}(t)$ et $R_{out}(t)$ sont séparées et on écrit la pression $P(t)$ en fonction soit de $R_{in}(t)$ soit de $R_{out}(t)$. De manière expérimentale, on doit donc mesurer uniquement les rayons initiaux externe et interne et suivre l'évolution du rayon interne ou externe de la capsule.

$$P(t)=\frac{4}{3}E\left[1-\frac{1}{1+\Delta(R_{0}^{3})/R_{in}^{3}(t)}\right]\frac{u(R_{in}(t))}{R_{in}(t)} \quad (8),$$

$$P(t)=\frac{4}{3}E\left[\frac{1}{1-\Delta(R_{0}^{3})/R_{out}^{3}(t)}-1\right]\frac{u(R_{out}(t))}{R_{out}(t)} \quad (9).$$

Notez que, en retournant au cas général décrit par l'Eq. (4) et en construisant le rapport des déplacements au niveau des surfaces interne et externe, on trouve (Dym, C. L. & Williams, H. E. (2007) International Journal of Mechanical Engineering Education 35, 108-113) :

$$\frac{u(R_{out})}{u(R_{in})}=\frac{3(1-\nu)\rho}{2(1-2\nu)+(1+\nu)\rho^{3}} \quad (10),$$

où $\rho = R_{out}/R_{in} > 1$.

Tout d'abord, étant donné que ce rapport est toujours inférieur à l'unité, le déplacement au niveau du rayon externe est plus petit que celui au niveau du rayon interne, qui est intuitif et observé de manière expérimentale. Ensuite, $u(R_{out})/u(R_{in})$ offre une estimation directe du rapport de Poisson, qui s'est avéré être $\nu = 1/2$ .

II Résultats

II.A Formation de microcapsules d'alginate assistée par un dispositif microfluidique

Le protocole pour préparer les microcapsules cellulaires s'inspire du procédé mis au point pour la fabrication de perles liquides de l'ordre du millimètre et est en outre adapté pour réduire le diamètre des capsules et répondre aux exigences d'une culture cellulaire. Le principe d'opération fondamental consiste à générer des coques d'hydrogel renfermant une suspension de cellules par co-extrusion (Figure 6A). Plus précisément, le dispositif microfluidique est assemblé par le co-centrage de trois capillaires en verre (Figure 6B). La suspension cellulaire circule dans le capillaire

le plus interne, alors qu'une solution d'alginate est injectée dans le capillaire effilé le plus externe. La gélification de la coque d'alginate est réalisée hors puce dans un bain de calcium. Un capillaire intermédiaire rempli d'une solution dépourvue de calcium sert de barrière à la diffusion du calcium libéré à partir des stocks intracellulaires et évite ainsi le blocage de la puce. Les inventeurs ont également modifié le mode de formation des gouttelettes. À de faibles débits q, le liquide s'écoule au goutte-à-goutte du capillaire et produit des capsules d'une taille de 2-3 mm (Figure 6C), en conséquence de l'interaction entre la gravité et la tension superficielle. Par opposition, à un débit *q* plus élevé, le liquide émerge sous la forme d'un jet, qui se disperse en gouttelettes en aval en raison de l'instabilité du capillaire. On s'attend alors à ce que la taille des gouttelettes soit étroitement associée au diamètre D de l'orifice. Une limite inférieure pour le débit du liquide est définie par la condition de survenue de la transition goutte à goutte-jet, c'est-à-dire pour un nombre de Weber critique $pV^2D/\sigma \approx 4$ avec un liquide dans la limite non visqueuse et à faible gravité. En négligeant la structure de l'écoulement en trois phases et en supposant un simple liquide avec $\rho = 10^3$ kg m$^{-3}$, $\sigma$ = 50 mN m$^{-2}$, on obtient $V_{min} \sim 1$ m s$^{-1}$, et $q_{min} = \pi(D/2)^2V_{min} \sim 40$ ml h$^{-1}$ pour D = 130 $\mu$m. Une limite supérieure pour q est contrôlée par la hauteur de la chute : la distance d entre l'embout et la surface du bain de gélification doit être plus importante que la longueur intacte du jet, qui peut atteindre 10 à 100 x D (Figure 6c), en fonction de q et des perturbations externes. En revanche, les inventeurs ont observé que le fait d'augmenter d favorisait la coalescence de deux gouttes consécutives avant la gélification, ce qui générait au bout du compte des capsules plus grosses de forme ellipsoïdale. Jusqu'à présent, les inventeurs avaient négligé le fait que le noyau de la gouttelette, la coque et le bain de gélification, étaient des phases aqueuses, donc à priori miscibles. Pour éviter tout mélange, les inventeurs ont ajouté des traces de tensioactif à la solution d'alginate et à la surface du bain de gélification, ce qui réduit la tension superficielle et confère une rigidité transitoire à la goutte de composé lors de l'impact.

II. B Caractérisation des microcapsules

Dans une expérience typique, approximativement la moitié des capsules sont sphériques (tel que déterminé par le paramètre de circularité > 0,8) et monodispersées (Figure 7A). La vitesse de production des capsules (> $10^4$s$^{-1}$) est suffisamment élevée pour permettre une rapide sélection manuelle de 10-100 capsules de forme sphérique. Bien qu'il soit possible d'augmenter la fraction des sphères en forçant l'instabilité du capillaire par des perturbations de l'écoulement contrôlées, une légère anisotropie sera toujours présente en raison de la présence d'une petite queue qui est inhérente à l'impact dans le bain de gélification. Pourtant, cette anisotropie a un effet négligeable sur les mesures mécaniques rapportées ci-dessous. On s'attend à ce que la taille moyenne des gouttelettes de composé soit déterminée par le mode de croissance le plus rapide $2\pi/\lambda$ de l'instabilité de Rayleigh. Étant donné que A est proportionnel au diamètre du jet de liquide $d_{jet}$ pour un contraste de viscosité donné, la conservation du volume entre un cylindre de longueur A et de section $\pi d_{jet}^2/4$ et une goutte d'un rayon *R* entraîne $R = d_{jet}$. Pour la plupart des conditions d'opération, le diamètre de l'embout était D = 130 $\mu$m $\approx d_{jet}$, en produisant une taille de goutte moyenne $R$ = 148 $\pm$ 21 $\mu$m, ce qui est conforme à la prédiction théorique de premier ordre. L'épaisseur de la coque peut être mesurée en imagerie confocale par coloration au dextran fluorescent de haut poids moléculaire d'une capsule d'alginate. Une telle observation permet de constater une séparation claire de la coque de la suspension cellulaire et de la solution intermédiaire de la capsule, un mélange réduit des constituants de la capsule. Ainsi, l'épaisseur de la coque *h* peut être mesurée avec précision. Cependant, de manière plus intéressante, *h* peut être ajustée en faisant varier le rapport entre le débit interne $q_{in}$ (somme des débits de la suspension cellulaire et de la solution intermédiaire) et le débit externe $q_{out}$ de la solution d'alginate. Des modifications du rapport $q_{in}/q_{out}$ ont principalement un effet sur le rapport d'aspect $h/R_{out}$ (Figure 2d), $R_{out}$ étant le rayon externe de la capsule. La production de capsules à parois très fines est limitée par la fragilité de la coque. Cependant, une augmentation du débit d'alginate visant à produire des coques très épaisses a tendance à générer des capsules hétérogènes et déformées. En pratique, pour des capsules d'un rayon d'environ 150 $\mu$m, *h* peut varier, en toute fiabilité, entre 5 et 35 $\mu$m (Figure 7B).

Les inventeurs ont également étudié les propriétés mécaniques des capsules d'alginate. De manière assez surprenante, la rhéologie des gels d'alginate fait toujours l'objet de débats. Hormis les discordances observées entre des travaux qui utilisent des techniques distinctes, le module de Young *E* des gels d'alginate, qui caractérise la rigidité du matériau brut, dépend de nombreux paramètres (concentration en alginate, composition chimique, nature et concentration des cations de réticulation). Pour éviter toute variabilité dépendante du protocole, les inventeurs ont directement évalué l'élasticité du gel brut des capsules en utilisant un essai de gonflement osmotique. D'après la déformation de capsules pré-chargées avec du dextran de haut poids moléculaire et immergées dans une solution graduellement appauvrie en dextran, les inventeurs ont dérivé $E$ = 68 $\pm$ 21 kPa (Figures 8A et 8B, exemple 5 Méthodes). Cette valeur a en outre été confirmée par un test de micro-indentation par AFM (Figure 9A) et une élongation macroscopique de cylindres d'alginate bruts (Figure 9B). Même si les gels d'alginate présentent une structure particulière illustrée par le modèle de la boîte à oeufs, une relation approximative valide pour des gels polymères réticulés[27], $E/3 = kT/\xi^3$, permet d'estimer la taille moyenne des mailles du gel $\xi \approx 6\,nm$, qui est suffisamment importante pour que des protéines globulaires d'un $P_m$ d'environ 150 kDa puisse se diffuser à travers cette dernière. Aucune hystérèse n'a été observée lors de cycles

de gonflement-rétrécissement osmotique et aucune évolution temporelle de la déformation n'a été détectée lorsque la différence de pression osmotique était maintenue pendant des périodes de temps plus longues (données non montrées), ce qui suggère que l'hydrogel se comporte comme un matériau purement élastique.

II.C Analyse quantitative de la croissance d'un sphéroïde confiné dans un environnement élastique

Afin d'obtenir une description quantitative de l'impact du confinement élastique sur la croissance des MCS, les inventeurs ont ajusté la rigidité, $k_{caps}$, des capsules en faisant varier l'épaisseur de la coque ($k_{caps} \propto E \times h$) et ont surveillé l'évolution du rayon moyen des sphéroïdes, $R_{MCS}(t)$, en utilisant la microscopie-vidéo en temps réel. Trois phases distinctes ont été observées. Lors de la phase 1, avant confluence (t<0), le $R_{MCS}$ augmente rapidement à des taux similaires dans les capsules épaisses et fines (Figure 11A). Le sphéroïde croît librement à l'intérieur de la capsule à un taux de croissance constant, $\dot{v}/v = 3\dot{R}_{MCS}/R_{MCS} \approx 1{,}25(jour)^{-1}$, ce qui est similaire au taux de doublement de monocouches cellulaires en 2D (Figures 11B et 13A). La phase 2 débute typiquement lorsque le $R_{MCS}$ approche le rayon interne de la coque $R_{in}$ au sein d'une seule taille cellulaire (environ 10 μm). À t=0 (confluence), le taux de croissance apparent $\dot{v}/v$ diminue d'environ trois fois. La phase 2 correspond à la transition de lissage, et dure approximativement de t=-1 jour à t=+1 jour (Figure 11B). Lors de la phase 3 (t>0), le $R_{MCS}$ se stabilise pratiquement pour les capsules épaisses et continue à lentement augmenter pour les capsules fines (Figure 11A). Une inspection approfondie révèle que $\dot{v}/v$ chute de plus d'un ordre de magnitude par comparaison à la croissance libre du MCS, mais ne devient jamais strictement égale à zéro (Figure 11B). La moyenne d'approximativement 20 capsules indique que $\dot{v}/v$ en phase 3 est d'environ 0,07 $(jour)^{-1}$ pour les capsules fines et de 0,04 $(jour)^{-1}$ pour les capsules épaisses (Figure 13A).

D'un point de vue qualitatif, même si l'on s'attend à une croissance plus lente en cas de confinement dans des capsules plus rigides, une explication quantitative exige de dériver la pression exercée par le MCS en expansion. À titre de première approximation, les capsules doivent être considérées comme des récipients pressurisés à fines parois dans le cadre d'une élasticité linéaire isotrope. La pression qui gonfle la coque est ensuite donnée par:

$$P = \frac{2E}{1-\nu} \cdot \frac{h}{R} \cdot \frac{u(R)}{R},$$

où $u(R)$ est le déplacement radial à une distance $R_{in} \leq R \leq R_{out}$ depuis le centre de la capsule, et $v$ le rapport de Poisson (Landau, L. D., et al 1986, Theory of Elasticity, Third Edition: Volume 7. Butterworth-Heinemann). La lente réduction de $h(t)$ observée (Figure 12A, symboles) est conforme à une dépendance à $1/R^2$ (Figure 12A, lignes), tel qu'attendu pour un gel incompressible ($v$ =1/2). En pratique, les conditions expérimentales exigent des corrections supplémentaires. Tout d'abord, pour les capsules fines, la supposition d'une élasticité linéaire ne peut s'appliquer étant donné que les contraintes excèdent 20 %. Une dépendance phénoménologique du module de Young à la contrainte a été prise en compte pour l'élasticité non linéaire (voir exemple 5 point M.). Ensuite, pour les capsules épaisses ($h/R$ d'environ 0,25), le formalisme complet de la théorie d'un récipient à parois épaisses doit être utilisé (voir exemple 5 point N.). En tenant compte de ces corrections, les inventeurs ont observé que les courbes de pression des capsules fines et épaisses chutent principalement au sein de l'erreur expérimentale (Figure 12B) et exhibent deux caractéristiques principales. Tout d'abord, la pression s'accumule rapidement au cours des premières 24 heures après confluence (Figure 13B, $\dot{P}$ = 2,4 ± 0,5 kPa $(jour)^{-1}$). Ensuite, à une pression seuil $P_{th}$ = 2,2 ± 0,5 kPa, la transition en phase 3 est indiquée par une chute spectaculaire de l'augmentation de la pression, qui atteint une valeur constante aussi faible que $\dot{P}$= 0,2 ± 0,08 kPa $(jour)^{-1}$) (Figure 13B). Le seul fait que $\dot{P}$ reste positif indique que la croissance des sphéroïdes n'est pas interrompue, tel que confirmé par la reprise d'une rapide croissance après la dissolution ou l'éclatement de la capsule (Figure 11A et Figure 14). Dans leur ensemble, ces résultats démontrent que les caractéristiques mécaniques des sphéroïdes confinés peuvent être caractérisées d'un point de vue quantitatif en mesurant la déformation des capsules élastiques. En revanche, afin d'obtenir une compréhension mécanistique d'une croissance de MCS altérée dans des conditions de confinement, il est nécessaire d'étudier le devenir de sphéroïdes post-confluence au niveau cellulaire et moléculaire.

II.D Impacte d'un confinement élastique sur l'organisation cellulaire interne des sphéroïdes

Tel que susmentionné, les stades post-confluence des MCS sont caractérisés par la nette apparition d'un noyau sombre. La réorganisation de la structure du MCS semble concomitante avec la survenue de la phase 3 (Figure 13C). Afin d'élucider la cause de cette perte de transparence significative du noyau du MCS, les inventeurs ont utilisé des colorants fluorescents non perméables aux membranes. Tout d'abord, ils ont utilisé un colorant des protéines hydrosolubles, la sulforhodamine B (SRB), qui s'accumule dans l'espace extracellulaire (cellules perméabilisées ou protéines sécrétées). Par microscopie biphotonique, les inventeurs ont observé que i) un noyau clair est nucléé quelques heures après la confluence (à P environ 0,5 kPa), ii) il se propage vers l'extérieur d'une manière de type fractale (jusqu'à $P \sim P_{th}$), et iii) à des moments ultérieurs dans le temps, le noyau marqué occupe la plus grande fraction du sphéroïde

alors que les 3-4 premières couches cellulaires périphériques demeurent non colorées (données non montrées). À titre de contrôle, un sphéroïde de même taille (R~150 μm), cultivé dans une capsule plus grosse et libéré avant la confluence, révèle difficilement une quelconque coloration. L'organisation du noyau sensible au colorant SRB est par conséquent uniquement induite par le confinement et n'est pas comparable à la formation du noyau nécrotique observée dans les gros sphéroïdes (*R*>400 μm), résultant d'une diffusion limitée de l'oxygène et des nutriments. Ensuite, les inventeurs ont acquis des images de MCS encapsulés marqués avec un colorant sensible aux membranes, le FM4-64. Étant donné que la fluorescence du FM4-64 est plus intense dans un environnement lipophile, les noyaux des cellules vivantes sont négativement colorés. Les événements nécrotiques sont révélés par l'apparition d'une forte fluorescence dans l'intégralité de la cellule. La similarité entre les profils du SRB et du FM4-64 confirme que le noyau induit par le confinement se compose de cellules perméabilisées ou de débris de cellules. Néanmoins, une immunocoloration de MCS post-confluence fixés révèle également la présence de fibronectine (données non montrées), ce qui suggère que le noyau consiste en un mélange de cellules mortes et de protéines de la matrice sécrétées. Cette nature du noyau de type mélange est cohérente avec sa forte cohésivité apparente étant donné qu'il résiste à la dissociation suite à un traitement par la trypsine (données non montrées).

L'imagerie du noyau d'un MCS est une tâche difficile en raison de la diffusion restreinte des colorants extrinsèques et de la profondeur de pénétration limitée de la lumière. En revanche, la bordure des cellules comprimées entre la coque et le noyau est davantage sensible à une microscopie haute résolution des cellules vivantes. Les inventeurs ont obtenu les images de cellules CT26 stablement transfectées avec du LifeAct-mCherry pendant 3 jours avant et après confluence (données non montrées). Au départ, les cellules sont relativement arrondies et modérément mobiles au sein sphéroïde en expansion. Une fois la confluence atteinte, la plupart des cellules périphériques présentent une migration importante et forment de longues et fines protrusions avec des lamellipodes et des filopodes aux extrémités. Des lamellipodes et des filopodes ont également été observés dans des cellules fixées non transfectées colorées par la phalloïdine fluorescente (données non montrées).

Dans leur ensemble, ces données d'imagerie suggèrent que le confinement induit par la capsule provoque une réorganisation au sein du sphéroïde après confluence, qui prend une structure en couches à l'état d'équilibre composée d'un noyau compact constitué de débris cellulaires cimentés par des protéines extracellulaires comme la fibronectine, l'élastine, et une bordure périphérique de cellules allongées hautement motiles.

**<u>Exemple 6 :</u> Encapsulation de cellules CT26 dans une capsule d'alginate simple (pas selon l'invention)**

I Conditions expérimentales

*<u>I. A. Encapsulation</u>*

L'encapsulation des cellules se fait par formation d'un jet composé de deux phases coaxiales. La phase interne contenant les cellules en suspension dans leur milieu de culture, ou un tampon biologique iso-osmotique compatible avec le procédé d'encapsulation ; cette phase composera le coeur des capsules. La phase externe est composée d'une dispersion d'alginate de sodium à 2% m/v ayant un rapport L-guluronic / D-mannuronic (G/M) compris entre 65-75 % / 25-35 % et une viscosité pour une dispersion à 1% m/v à 20°C comprise entre 200 et 400 mPas (i.e. FMC BioPolymer, Protanal LF 200S) et 0.5 mM de sodium dodecyl sulfate (SDS). La phase externe réalisera *in fine* la coque d'alginate de la capsule. Chacune des phases est placée dans une seringue stérile dont le débit est contrôlé par un pousse-seringue. Les seringues sont raccordées à un injecteur à deux voies schématisé, en figure 15A, permettant de réaliser un jet. Selon cette représentation schématique, la phase interne destinée à être encapsulée circule à travers le compartiment 21 pour être injectée au centre du capillaire C. La phase externe destinée à former la coque d'alginate de la capsule circule à travers le compartiment 41 et est injectée à la périphérie interne du capillaire C.

Les débits délivrés par les pousses-seringues sont fonctions de la géométrie de l'injecteur, notamment du diamètre du capillaire de sortie, et de la viscosité des fluides utilisés. Ces débits sont adaptés afin de permettre la formation d'un jet (passage du régime goutte à goutte à un jet) dont la fragmentation en microgouttelettes se fait selon l'instabilité de Plateau-Rayleigh. Cette fragmentation peut être contrôlée en appliquant au fluide de la phase externe une vibration contrôlée par effet piézo-électrique avec une fréquence située entre 0 et 2000 Hz. Afin de prévenir la coalescence des microgouttelettes formées, une électrode cylindrique est placée au niveau du site de fragmentation du jet ; un courant continu de 0 à 2000 V est appliqué et a pour effet de charger électriquement la surface des microgouttelettes assurant ainsi leur répulsion respective et prévenant leur coalescence.

Les microgouttelettes multi-composantes formées lors de la fragmentation du jet, sous l'effet de la gravité, tombent dans une solution aqueuse de chlorure de calcium à 1% (m/v) ce qui a pour effet de réticuler l'alginate à l'extérieur des microgouttelettes et former ainsi la coque d'alginate contenant en son coeur les cellules de la phase interne. Les capsules sont récupérées, rincées dans un tampon physiologique ne dépolymérisant pas l'alginate (i.e. sans phosphates ni chélatants) puis placées dans des flacons de culture cellulaire stérile avec le milieu de culture utilisé

pour les cellules. La coque d'alginate des capsules étant semi-perméable, elle permet la diffusion des nutriments et des gaz nécessaire à la survie et la croissance cellulaire. Les capsules sont incubées à 37°C et 5% de $CO_2$ afin de permettre la croissance des cellules.

*I.B. Survie et croissance cellulaire*

Les cellules utilisées sont les tumorales de la lignée CT26.

Les capsules d'alginate étant optiquement claires, les cellules encapsulées ont été dans un premier temps, observées en microscopie optique afin de déterminer leur morphologie et suivre leur évolution.

La survie cellulaire peut être déterminée par l'utilisation des méthodes classiques colorimétriques (e.g. Tests MTT, XTT, Resazurine) ou fluorométriques (Calcéine, fluorescéine diacétate, propidium iodide) basées sur le métabolisme et la physiologie cellulaire.

En l'occurrence, la survie cellulaire des cellules encapsulées dans des capsules simple a été effectuée par un marquage à la calcéine et au propidium iodide a été réalisé sur les capsules nouvellement formées selon la méthode suivante. Les capsules sont incubées en présence d'une forme estérifiée de la calcéine (Calcein-AM, LifeTechnologies) non fluorescente dans les conditions prescrites par le fabriquant. Ce fluorophore diffuse à travers la capsule et à travers la membrane plasmique et est hydrolysé au sein des cellules dont le métabolisme est actif (i.e. vivantes) ; la calcéine ainsi produite fluoresce dans le vert et reste, du fait de sa charge, dans le cytosol des cellules. Après incubation des capsules avec la calcéine-AM, les capsules sont mises en contact avec le propidium iodide. Ce fluorophore, du fait de sa charge, diffuse uniquement dans les cellules dont la membrane plasmique est endommagée et se fixe sur l'ADN, ce qui a pour effet d'augmenter sa fluorescence de 20 à 30 fois. Ainsi, après exposition des capsules à ces deux fluorophores, l'observation des cellules en microscopie confocale permet de distinguer les cellules vivantes, fluoresçant dans le vert, des cellules mortes, fluoresçant dans le rouge.

II. Résultats

La viabilité cellulaire a été contrôlée grâce au test Live/Dead à J0 et à J15 après l'encapsulation montrant une très bonne survie cellulaire ainsi, les cellules survivent à l'encapsulation et ont une bonne croissance cellulaire au-delà de 15 jours.

L'observation des capsules en microscopie optique ou confocale confirme la formation de sphéroïdes c'est-à-dire d'agrégats de cellules. De telles structures sont observées avec des cellules tumorales qui n'adhèrent pas aux parois de la capsule. Ces capsules constituent donc de bons modèles d'étude des métastases.

Ces capsules sont particulièrement avantageuses pour la culture de cellules non adhérentes en suspension telles que des cellules sanguines.

*Exemple 7*: *Culture Tridimensionnelle de tissu cutané dans des capsules d'alginate structurées*

Afin de dépasser la simple co-culture de cellules dans les capsules d'alginate, les capsules peuvent être incubées plusieurs jours dans des conditions permettant la prolifération cellulaire puis l'organisation des cellules en un ou des tissus semblables aux tissus cutanés. Ainsi les fibroblastes disséminés dans l'enveloppe intermédiaire peuvent, selon les conditions de culture, proliférer puis synthétiser des molécules de la matrice extracellulaire. Cette organisation correspond à l'organisation du derme du tissu cutané. De même, les kératinocytes contenu dans le coeur de la capsule sont destinés à adhérer à la surface interne de l'enveloppe intermédiaire, dans le coeur, proliférer jusqu'à organiser une monocouche cellulaire recouvrant l'intérieur de la capsule. A complétion et dans des conditions de culture définies, les kératinocytes peuvent entrer en différenciation et former un tissu stratifié cohésif semblable à l'épithélium stratifié kératinisé formant l'épiderme du tissu cutané.

Ainsi, il est possible de former des capsules contenant indépendamment, du derme reconstruit, de l'épiderme reconstruit et de la peau reconstruite, association du derme et de l'épiderme reconstruits au sein d'une même capsule.

I. Conditions expérimentales

*IA. Collagène*

Au cours du développement des SkinPearls, plusieurs types de collagène ont été testés. Selon les méthodes d'extraction utilisées, les solutions de collagènes ne sont pas toutes susceptibles de former un gel. A titre indicatif, le Collagène I issue de queues de rats de Gibco à 3mg/ml et la solution de Collagène issue de la peau de bovin à 3mg/ml de Sigma permettent la formation d'un gel.

La cinétique de gélification de la solution de collagène est augmentée par l'effet combiné de la neutralisation

du pH et une élévation de la température à 37°C. En effet, le collagène est soluble dans une solution aqueuse acide, généralement d'acide acétique, et la neutralisation du pH permet de recréer des interactions électrostatiques entre les fibrilles de collagène afin de former des fibres structurées au sein d'un réseau.

En parallèle du collagène a été extrait de queues de rat selon le protocole suivant. Brièvement, deux queues de rat préalablement trempées dans l'éthanol à 70° ont été disséquées et les tendons extraits de leurs fascia. Ces tendons ont alors été trempés dans des solutions d'acide acétique placées à 4°C remuées régulièrement jusqu'à solubilisation. Les solutions devenues plus épaisses sont ensuite centrifugées plusieurs fois afin d'éliminer les débris présents. Les surnageant sont conservés à 4°C jusqu'à utilisation. Ce collagène forme bien un gel lorsque l'on neutralise son pH et il a été substitué au collagène disponible commercialement pour la mise au point des capsules modèles de peau.

*Protocole de neutralisation du pH*

Typiquement un tampon de force ionique élevée est préparé puis mélangé au collagène. Les différents tampons sont ajoutés dans cet ordre. Chacune des solutions, stockée à 4°C, est conservée dans la glace afin de maintenir le tout à 4°C et ainsi ralentir la formation du gel.

| Tampon | Concentration / pH | Volume (μL) (Vf = 2 mL) |
|---|---|---|
| **HEPES** | 200mM / 7.5 | 200 |
| **MEM** | 10X | 200 |
| **DMEM** | 1X / 7.4 | 256 |
| **NaOH** | 1M | 21 |
| **Collagène** | 3 mg/mL / 3.6 | 1333 |

*Marquage du collagène à la rhodamine*

Lors de la réalisation des capsules de culture tridimensionnelle et plus précisément de l'enveloppe intermédiaire formant les capsules, il est intéressant de pouvoir observer la morphologie de cette couche. En effet, de nombreux facteurs peuvent impacter sa formation et il est important de comprendre les paramètres qui contrôlent son épaisseur, son homogénéité ainsi que sa géométrie. Pour cela le collagène a été marqué avec un fluorophore, la Rhodamine, afin de pouvoir réaliser des images 3D des capsules en microscopie confocale. Brièvement, 0,2 mg de rhodamine isothiocyanate (RITC) par mL de collagène sont incubé à 4°C à l'abri de la lumière durant 48 heures. A l'issu de cette incubation le collagène est dialysé contre une solution aqueuse d'acide acétique à 0.05M afin d'éliminer la rhodamine en excès. Le collagène marqué à la rhodamine est dilué dans du collège non marqué quand nécessaire. Ce marquage n'a démontré aucun effet négatif sur la polymérisation du collagène.

*I.B Alginate*

Les capsules sont formées à partir d'une solution d'alginate (Protanal LF 200S, FMC) à 2 % w/v et de SDS à 0.5 mM préalablement filtrée à 0,8 μm. Cette solution est complémentée en mélange streptomycine / pénicilline à 50 U/mL et conservée à 4°C afin de limiter le développement de microorganismes.

La solution d'alginate utilisée pour réaliser partiellement ou totalement l'enveloppe intermédiaire est une solution à 1% w/v, sans SDS, également filtrée à 0.8 μm avant utilisation.

*I.C Tampons*

Les capsules sont formées dans un bain de chlorure de calcium à 1%, filtré à 0,2 μm, en présence d'une goutte de Tween 20 afin de modifier la tension superficielle à la surface du bain et optimiser la formation de capsules rondes. Afin d'éliminer les ions calcium en excès, les capsules, immédiatement après formation, sont rincées dans un tampon HEPES (300 mOsm, pH = 7,5) préparé à partir d'une solution Hepes 5X (119.15 g Hepes, 3.75 g NaOH pastille, eau qsp 500 mL, pH ajusté à 7.5). En effet, le tampon utilisé doit être compatible avec la survie cellulaire et ne doit pas dépolymériser l'alginate comme c'est le cas avec les tampons phosphate ou citrate.

*I.D Culture Cellulaire*

Les fibroblastes de derme humain proviennent de déchets de chirurgie plastique. Ces cellules sont cultivées dans des flasques de culture cellulaire de 75 $cm^2$, en présence de DMEM (LifeTechnologies) supplémenté à 10 % v/v

en sérum de veau foetal (SVF, LifeTechnologies). Les passages sont réalisés à 80 - 90 % de confluence, 2 à 3 fois par semaine.

Les kératinocytes proviennent d'un épiderme humain adulte issu de déchets de chirurgie plastique. Ces cellules sont cultivées sur des flasques de culture cellulaire de 75 $cm^2$ préalablement enduites de collagène de type I issu de queue de rat. Les kératinocytes sont cultivées dans du milieu Epilife (LifeTechnologie) complété en Epilife Defined Growth Supplement (EDGS, LifeTechnologies). Les passages sont réalisés à 70 - 80 % de confluence et le milieu est renouvelé tous les deux jours.

Afin de réaliser les passages, les milieux de culture sont préalablement retirés, le tapis cellulaire est rincé avec 3 mL de solution de Trypsine à 0.05 % (LifeTechnologies) immédiatement jeté puis renouvelé. Les flasques sont alors placées dans l'incubateur quelques minutes afin que les cellules se détachent de la surface. La trypsine est alors neutralisée en ajoutant 5 mL d'inhibiteur de la trypsine (LifeTechnologies). La suspension cellulaire est alors centrifugée (180 g, 8 min) puis le culot est dispersé dans 1 mL du milieu de culture correspondant au type cellulaire. Cette suspension est alors utilisée pour ensemencer de nouvelles flasques, avec un ratio de 3 flasques ensemencées pour 1 flasque à 80% de confluence.

*I. E Préparation des cellules pour l'encapsulation*

Les cellules sont traitées comme lors d'un passage. Une fois la suspension cellulaire obtenue, les cellules sont comptées à l'aide de cellules de numération (i.e. KovaSlide) en réalisant un test d'exclusion au bleu trypan. Seules les cellules vivantes, non marquées par le bleu trypan, sont comptées. Les fibroblastes destinés à être localisés dans l'enveloppe intermédiaire des capsules seront dispersés dans la phase de l'enveloppe intermédiaire, décrite ci-après, à hauteur de 0.3 à 0.75 M de cellules par mL. Les kératinocytes, destinés à être encapsulés dans le coeur des capsules sont dispersés dans le milieu de culture ou le tampon biologique à hauteur de 0.5 - 1.5 M de cellules / mL.

*I.F Préparation de l'enveloppe intermédiaire*

La reconstruction d'un tissu cutané au sein de capsules d'alginate nécessite de permettre la compartimentation de ces capsules en deux zones correspondant aux deux feuillets composant la peau, à savoir l'épiderme et le derme. Du point de vue physico-chimique, l'enveloppe intermédiaire, à l'interface entre la coque d'alginate et le coeur de la capsule, doit avoir une composition dont la viscosité est inférieure à celle de la phase formant l'enveloppe d'alginate et supérieure à celle de la phase destinée à former le coeur de la capsule. De plus, sa composition doit permettre une réticulation ou une polymérisation rapide lors de la formation des capsules afin de prévenir les phénomènes d'écoulement et donc la désorganisation de la structure. Du point de vu biologique, la composition de l'enveloppe intermédiaire doit permettre la survie et la croissance des fibroblastes qui seront disséminées en son sein, recréant ainsi une matrice similaire à la matrice dermique. Enfin, la composition de l'enveloppe intermédiaire doit permettre l'adhésion des kératinocytes à sa surface.

Afin de résoudre l'ensemble de ces contraintes, l'enveloppe intermédiaire est composée de 50 à 80 % de Matrigel (BD Biosciences), ou de 50 à 80 % de collagène de type I (Gibco) dont le pH est neutralisé extemporanément par l'ajout de tampon biologique et d'hydroxyde de soude. La polymérisation des ces composés matriciels étant insuffisamment rapide, 20 à 50 % d'une solution d'alginate de sodium à 4% w/v sont ajoutés. Enfin, les fibroblastes sont dispersés dans cette mixture afin d'obtenir une concentration cellulaire comprise entre 0.3 et 0.75 M de cellules / mL.

*I.G Formation des capsules*

Les capsules sont formées par fragmentation de jet comme décrit précédemment avec des modifications afin de permettre la structuration des capsules. Pour cela un injecteur à trois voies est utilisé (Figure 15B). La phase interne formant le coeur de la capsule circule dans un premier compartiment 21 et est injectée au centre du capillaire C. Cette phase interne est gainée de la phase intermédiaire circulant dans le compartiment 31 et qui formera l'enveloppe intermédiaire de la capsule. La phase externe circulant dans le compartiment 41 est destinée à former la coque d'alginate de la capsule et est injectée à la périphérie interne du capillaire C. Chacune des voies 21, 31 et 41 est injectée dans le capillaire C de sortie et est destinée à former, de l'extérieur à l'intérieur, la coque d'alginate, l'enveloppe intermédiaire et le coeur de la capsule. Le débit de chacune des voies est contrôlé par un pousse-seringue électrique. La seringue de la phase interne est également équipée d'un système magnétique permettant d'homogénéiser la suspension cellulaire, sans cisaillement, afin d'éviter la sédimentation des cellules et assurer une homogénéité au cours de la manipulation. La fragmentation du jet et la coalescence des microgoutelettes peuvent être contrôlées par vibration piézo-électrique et formation d'un champ électrique respectivement. Les capsules formées sont recouvrées comme décrit précédemment puis incubées à 37°C et 5% de $CO_2$.

Le type de capsule produit dépend du type de cellules présentes. Les capsules pour les modèles de peau

contiennent à la fois les fibroblastes dans l'enveloppe intermédiaire et les kératinocytes dans le coeur. Les capsules pour les modèles d'épiderme contiennent uniquement les kératinocytes dans le coeur tandis que les capsules pour les modèles de derme contiennent uniquement les fibroblastes dans l'enveloppe intermédiaire.

*I.H Morphologie des capsules*

Après formation, les capsules sont observées directement en suspension dans le milieu de culture à l'aide d'un microscope inversé. La forme des capsules (i.e. circularité) et la polydispersité des tailles sont déterminées par calibration des microphotographies acquises avec ce microscope. De même, la répartition des cellules au sein de la capsule et leur localisation au sein de l'enveloppe intermédiaire et du coeur des capsules sont également vérifiées.

*I.I Structure des capsules*

Afin de vérifier l'organisation des différentes couches des capsules formées, un marquage de l'enveloppe intermédiaire est réalisé en substituant une partie du collagène de la composition par du collagène marqué à la rhodamine B, marqueur fluorescent. Après formation des capsules selon la méthode décrite précédemment, la répartition tridimensionnelle de l'enveloppe intermédiaire est déterminée par acquisition d'images en microscopie confocale sur l'ensemble de la capsule.

*I.J Caractérisation de la viabilité et de la prolifération cellulaire*

La viabilité cellulaire est déterminée par imagerie des capsules marquées à la calcéine et iodure de propidium selon la méthode décrite précédemment. Le suivi est réalisé sur plusieurs jours afin de déterminer l'évolution de la prolifération et de l'organisation des cellules.

II Résultats

II.A Culture tridimensionnelle Modèle de derme

Les capsules selon l'invention ont été utilisées afin de mettre en place des modèles de derme reconstruit. Ainsi ces capsules contiennent des fibroblastes disséminés dans une matrice que l'on souhaite la plus proche possible du derme. Le derme est un tissu conjonctif riche en collagène, élastine, fibronectine et glycosaminoglycane qui lui confèrent ses propriétés mécaniques. Le derme réalise un tissu de soutien et nourricier pour l'épiderme : en effet l'épiderme étant avasculaire, les nutriments et les échanges gazeux proviennent essentiellement du derme. Les cellules principales du derme sont les fibroblastes ; disséminés dans la matrice ils entretiennent sa composition et jouent un rôle important lors des phénomènes de cicatrisation. Par ailleurs, les fibroblastes sécrètent des cytokines et facteurs de croissance qui stimulent et régulent la prolifération des kératinocytes. De même la matrice dermique a une composition particulière favorisant l'adhésion des kératinocytes de l'épiderme et régulant leur prolifération et différentiation. Au niveau cosmétique le derme est la cible des traitements anti-âge : le maintien et la stimulation de la synthèse des composants de la matrice extra cellulaire est la principale cible du traitement antiride.

*Capsule à une seule enveloppe (pas selon l'invention)*

Dans un premier modèle, les fibroblastes ont été encapsulés au sein de l'enveloppe d'alginate composant l'enveloppe externe de la capsule. L'objectif, *in fine,* est de contenir ces cellules dans ce compartiment, de réaliser une couche intermédiaire de composition spécifique pour promouvoir l'adhésion des kératinocytes dans le coeur de la capsule. Cependant, après encapsulation des fibroblastes et réalisation d'un test de survie (Type Live/Dead Calcéine-AM et iodure de propidium), il apparait que toutes les cellules sont mortes, aucune ne semble survivre. Cette toxicité est due à la présence dans l'alginate de sodium dodecyl sulfate (SDS) à hauteur de 0.5 mM. Le SDS est essentiel à la formation des capsules et la concentration utilisée est la limite inférieure endeçà de laquelle il n'est plus possible de former des capsules homogènes. Hors, l'établissement d'un test de cytotoxicité démontre l'effet cytotoxique du SDS vis-à-vis des fibroblastes et notamment de la lignée 3T3. Ainsi, les inventeurs ont montré qu'entre 0,05 mM et 0,4 mM de SDS dans le milieu de culture, la survie cellulaire est entre 75 et 100%, elle descend à 38% à 0,5mM de SDS. Par conséquent, cette concentration de SDS entraine la mort de plus de 60% des cellules. Il n'est pas possible d'éliminer le SDS, cependant les fibroblastes peuvent être encapsulés directement dans l'enveloppe intermédiaire. Cette option offre la possibilité de permettre de modifier la composition de celle-ci afin de se rapprocher le plus possible de celle de la matrice extracellulaire dermique.

*Capsule à enveloppe intermédiaire d'alginate*

Les capsules selon cette variante comprennent des cellules uniquement dans l'enveloppe intermédiaire. Dans un premier temps les inventeurs ont réalisés des capsules dont l'enveloppe intermédiaire était composée d'une dispersion d'alginate à 1% w/v contenant des fibroblastes *de la lignée 3T3* à hauteur de 0,75.$10^6$ cellules / mL. Différents débits de chacune des phases formant les différentes couches des capsules ont été testées, puis, une fois rincées, les capsules sont suspendues dans du milieu de culture complet et placées dans l'incubateur à 37°C et 5% de $CO_2$. La viabilité cellulaire a été contrôlée grâce au test Live/Dead à J0, J2, J6 et J28 après l'encapsulation au cours de ce test les cellules vivantes sont marquées par la calcéine et les cellules mortes par l'iodure de propidium. L'acquisition du signal des cellules à travers les capsules est effectuée par microscopie confocale. Les inventeurs ont ainsi montré qu'une partie des cellules est morte après l'encapsulation, du fait du stress provoqué par le traitement enzymatique pour leur détachement des flasques de culture mais aussi par le cisaillement lors de leur mélange avec l'alginate et pendant la formation des capsules. Cependant, une partie des cellules reste vivante jusqu'à un mois après l'encapsulation. Les inventeurs ont observé que les cellules prolifèrent mais restent restreintes à quelques zones de la capsule sous forme de sphéroïdes ou de bâtonnets. Cela est dû au fait que les fibroblastes ne sécrètent pas d'enzymes capables de lyser l'alginate prévenant ainsi toute progression des cellules à travers le réseau d'alginate. Les cellules prolifèrent en comblant les défauts présents dans cette matrice.

*Capsule à enveloppe intermédiaire de collagène*

La même expérience a été réalisée en substituant l'enveloppe intermédiaire d'alginate par une couche de collagène. Les capsules selon cette variante comprennent également, des cellules uniquement dans l'enveloppe intermédiaire. Le collagène est l'un des composants majeur de la matrice extracellulaire dermique et est susceptible d'être dégradé par les collagénases sécrétées par les fibroblastes. La viabilité cellulaire a été contrôlée grâce au test Live/Dead à J0, J4, J8, J15 et J19 après l'encapsulation au cours de ce test les cellules vivantes sont marquées par la calcéine et les cellules mortes par l'iodure de propidium. Les résultats montrent dans ce cas une très bonne survie cellulaire, un étalement des cellules et une prolifération rapide couvrant l'intégralité de la capsule.

Les fibroblastes dans ce cas prolifèrent rapidement ce qui peut être problématique lors de la formation des capsules modèle de peau par la suite car les kératinocytes prolifèrent moins vite. En effet, les kératinocytes mettent entre 14 et 21 jours pour former les différentes couches qui composent l'épiderme. Ainsi, il convient de contrôler la prolifération des fibroblastes. Pour cela les inventeurs ont testé différentes compositions de couche intermédiaire en mélangeant différentes proportions d'alginate et de collagène. A titre d'exemple, les inventeurs ont montré que la croissance des fibroblastes au sein d'une couche intermédiaire composée pour moitié d'alginate à 1%w/v et pour autre moitié de collagène est limitée à une croissance sous forme de feuillets.

Ainsi, la viabilité cellulaire a été contrôlée grâce au test Live/Dead à J0 et à J15 après l'encapsulation montrant une très bonne survie cellulaire. De plus, à J0, le collagène marqué à la RITC est visible, à J15 seuls les signaux fluorescents des cellules vivantes et mortes sont enregistrés démontrant que l'ensemble du collagène est lysé par les fibroblastes au cours de leur croissance.

En conclusion, il est possible de contrôler la prolifération et la répartition des fibroblastes en modifiant la composition de l'enveloppe intermédiaire où sont localisées les cellules et plus particulièrement sur le ratio entre alginate et collagène.

Ces paramètres ont été caractérisés en utilisant des fibroblastes de la lignée murine 3T3. Afin de déterminer la viabilité de ce modèle pour des applications ultérieures, des modèles de derme ont été produits à partir de fibroblastes de derme humain adulte (HDFa). La culture de cellules HDFa par les inventeurs dans des capsules à enveloppe intermédiaire de collagène montre que les cellules ont survécu à l'encapsulation, adhèrent et s'étalent rapidement sur l'enveloppe intermédiaire de collagène. De plus, le test de viabilité cellulaire (test Live/Dead à J0 et à J5 après l'encapsulation) montre une très bonne survie cellulaire, validant ce modèle pour une optimisation et un développement futur.

*Capsule à enveloppe intermédiaire d'un mélange alginate/collagène*

La même expérience a été réalisée en substituant l'enveloppe intermédiaire d'alginate par une couche de comprenant 25% d'alginate pour 75% de collagène.

*Morphologie des capsules* : Les capsules formées selon la méthode décrite ci-dessus sont sphériques, leur surface externe lisse et de diamètre avoisinant les 500 - 600 μm selon les débits utilisés et le diamètre de capillaire de sortie de l'injecteur. Les cellules sont absentes de la couche externe d'alginate formant la capsule et sont restreintes à la couche intermédiaire composée d'une solution de collagène dont le pH a été neutralisé à 0.2% m/v (75%) et d'une solution d'alginate à 1% m/v (25%), légèrement plus dense optiquement, ainsi que dans le coeur des capsules.

*Organisation de la couche intermédiaire :* La couche intermédiaire peut être visualisée à l'intérieur des capsules

par imagerie confocale du collagène marqué par un fluorophore. Les images acquises montrent une répartition du collagène de la couche intermédiaire structurée en une couche homogène à travers les capsules, tapissant l'intérieur de celles-ci et délimitant un coeur liquide.

*Caractérisation de la viabilité et de la prolifération cellulaire :* La viabilité cellulaire a été caractérisée directement après l'encapsulation mais également après plusieurs jours de culture dans un incubateur à 37°C et 5% de $CO_2$. La majorité des cellules donnent un signal vert et présentent donc un métabolisme actif. Quelques cellules donnent un signal rouge traduisant une perméabilité membranaire donc une cellule morte. Dans l'ensemble, plus de 80 % des cellules sont viables après l'encapsulation. Après plusieurs jours d'incubation, les cellules prolifèrent et s'organisent devenant de moins en moins distinguables. Proportionnellement les cellules vivantes restent majoritaires.

II.B Culture tridimensionnelle Modèle d'épiderme

L'épiderme est un tissu épithélial stratifié kératinisé. Les kératinocytes constituent les principales cellules de ce tissu. In vivo, la couche la plus basale, au contact de la jonction dermo-épidermique (JDE) séparant le derme de l'épiderme, constitue la couche germinative. Cette couche assure un renouvellement des couches superficielles en stimulant la prolifération des kératinocytes. Une fois que les cellules perdent leurs contacts avec la JDE, elles initient leur différenciation en corneocytes. Cette spécialisation cellulaire assure une forte cohésion formant la barrière épidermique supportée principalement par la couche la plus externe : le stratum corneum. Ainsi, pour initier la formation d'un épiderme dans les capsules selon l'invention, il convient de réaliser une matrice favorable à l'adhésion des kératinocytes, leur prolifération en une couche confluente qui initiera la stratification. Les étapes finales de kératinisation nécessitent en plus un stimulus lié au contact de l'air. Ainsi les modèles d'épiderme selon l'invention, en l'absence de ce stimulus ne pourront présenter qu'un épiderme n'ayant pas réalisé sa différentiation terminale. Les capsules selon cette variante comprennent des cellules uniquement dans le coeur liquide.

Le collagène est un substrat de choix pour l'adhésion des kératinocytes. Dans un premier temps les inventeurs ont réalisé des capsules avec une couche intermédiaire faite uniquement de collagène, les kératinocytes de la lignée HaCaT sont présents dans le coeur liquide. Un marquage des kératinocytes par la calcéine montre que les kératinocytes survivent à l'encapsulation (à J0), adhèrent à la matrice de collagène (à J5) et prolifèrent en formant des «patchs» (à J16), feuillets de cellules cohésives, qui envahissent progressivement la surface interne de la capsule.

Ainsi, lorsque la couche intermédiaire est composée uniquement de collagène, la structure de la matrice de collagène devient hétérogène lors de sa gélification induisant une adhésion cellulaire en plaque de cellules. En effet, au cours de l'encapsulation, la dispersion de collagène dont le pH a été neutralisé commence à gélifier modifiant ainsi sa viscosité. De plus la gélification n'est pas instantanée comme peut l'être la réticulation de l'alginate au contact des ions calcium. Ainsi, le collagène glisse des parois de la capsule et s'amoncèle au bas de celle-ci avant de gélifier totalement, ou, de façon moindre, crée des instabilités à l'origine de mélanges des cellules en suspension dans le coeur avec le collagène.

Afin de remédier aux problèmes liés à la cinétique de gélification du collagène, les inventeurs ont mélangé le collagène à l'alginate. Cela ayant pour but de créer un patron pour la gélification du collagène. Dans un premier temps les inventeurs ont déterminé les ratios d'alginate et de collagène qui favorisaient l'adhésion des kératinocytes de la lignée HaCaT. Pour cela des gels d'alginate avec des concentrations croissantes de collagène (0%, 0.05%, 0.1%, 0.15% et 0.2% m/v) et décroissantes en alginate (1%, 0,75%, 0,5%, 0,25% et 0% m/v) ont été réalisés, puis ensemencé de kératinocytes. Le control est l'adhésion de ces cellules, dans les mêmes conditions, au plastique traité pour la culture cellulaire. Au bout de quelques heures les cellules ont été observées en microscopie optique. Les cellules non adhérentes sont parfaitement rondes et réfringentes tandis que les cellules adhérentes s'étalent et ont une forme polyédrique. La figure 14 présente ces observations.

De ces observations il apparait que l'adhésion des kératinocytes débute dès 25% v/v de solution de collagène dans l'alginate (concentration finale en collagène de 0,05% m/v) tandis que ces cellules sont incapables d'adhérer au gel d'alginate pur. Plus la teneur en collagène augmente, plus les cellules sont adhérentes et s'étalent. Des capsules dont la couche intermédiaire contenait un mélange d'alginate et de collagène afin de promouvoir l'adhésion des kératinocytes ont été réalisées avec alginate/collagène 0.5% m/v / 0.1 % m/v, 0,25% m/v / 0,15 % m/v, 0% m/v / 0,2% m/v.

Un marquage des kératinocytes par la calcéine montre que les kératinocytes survivent à l'encapsulation (à J0), adhèrent à la matrice (à J5) pour l'ensemble des capsules. Néanmoins, les cellules adhèrent et prolifèrent mieux (J16) en présence de collagène seulement.

Il semble qu'à mélange égal de solutions d'alginate (1% m/v) et de collagène (0.2% m/v) les cellules adhèrent peu et beaucoup meurent. La réticulation centripète de l'alginate est susceptible d'organiser le collagène de manière particulière et de façon moins propice aux cellules comparativement aux observations réalisées sur gel en boîte de culture. En présence de volumes supérieurs de solution de collagène (correspondant à des concentrations finales en collagène de 0,15 à 0,20 % m/v), les kératinocytes adhèrent et prolifèrent mais restent confinés à des patchs isolés après 16 jours de culture. La distribution du collagène de la couche intermédiaire n'est pas homogène ce qui ne permet

pas aux kératinocytes de migrer sur toute la surface interne de la capsule. Afin de résoudre cette distribution, à concentration égale de collagène, la concentration finale d'alginate peut être augmentée à 1,0 % m/v permettant une meilleure rigidification de la couche intermédiaire et une meilleure répartition du collagène.

## Revendications

1. Capsule comprenant un coeur liquide, au moins une enveloppe externe encapsulant totalement le coeur liquide à sa périphérie, ladite enveloppe externe étant propre à retenir le coeur liquide lorsque la capsule est plongée dans un gaz et comprenant au moins un polyélectrolyte gélifié et/ou un biopolymère rigidifié, et une enveloppe intermédiaire rigide comprenant au moins un biopolymère, ladite enveloppe intermédiaire étant située entre le coeur liquide et ladite enveloppe externe, ladite capsule étant **caractérisée en ce qu'**elle comprend au moins une cellule eucaryote de mammifère.

2. Capsule selon la revendication 1 **caractérisée en ce que** le coeur liquide et/ou l'enveloppe intermédiaire rigide comprend au moins une cellule eucaryote de mammifère.

3. Capsule selon l'une quelconque des revendications 1 ou 2 **caractérisée en ce que** le biopolymère de ladite enveloppe intermédiaire est choisi parmi les protéines de la matrice extracellulaire, les protéoglycannes, les glycosaminoglycanes, les polysaccharides et leur forme non hydrolysée ou partiellement hydrolysée.

4. Capsule selon l'une quelconque des revendications 1 à 3 **caractérisée en ce que** le coeur liquide et/ou l'enveloppe intermédiaire comprennent indépendamment l'une de l'autre, une cellule eucaryote de mammifère choisie parmi les fibroblastes, les mélanocytes, les kératinocytes et leurs mélanges.

5. Capsule selon l'une quelconque des revendications 1 à 4 **caractérisée en ce qu'**elle est obtenue par la mise en oeuvre d'un procédé comprenant les étapes suivantes de :

   a) formation d'une goutte liquide multi-composante comprenant :

      • un coeur liquide,
      • une enveloppe intermédiaire liquide formée d'une composition aqueuse comprenant au moins un biopolymère, encapsulant totalement à sa périphérie le coeur liquide, et
      • une enveloppe externe liquide formée d'une composition aqueuse, différente de la composition intermédiaire, ladite composition aqueuse comprenant au moins un polyélectrolyte et au moins un tensio-actif, ladite enveloppe externe liquide encapsulant totalement à sa périphérie l'enveloppe intermédiaire,

   le coeur liquide et/ou l'enveloppe intermédiaire liquide comprenant au moins une cellule eucaryote de mammifère
   b) gélification par immersion de ladite goutte liquide multi-composante dans une solution gélifiante contenant un réactif propre à gélifier le polyélectrolyte de l'enveloppe externe liquide, pour obtenir une capsule gélifiée comprenant une enveloppe externe gélifiée,
   c) rigidification de la composition intermédiaire de l'enveloppe intermédiaire liquide, pour obtenir une capsule gélifiée et rigidifiée comprenant une enveloppe intermédiaire rigidifiée, et
   d) récupération desdites capsules gélifiée et rigidifiée.

6. Capsule selon la revendication 5 **caractérisée en ce que** le procédé comprend en outre, une étape de dissolution de l'enveloppe externe gélifiée.

7. Procédé de préparation d'une capsule selon l'une quelconque des revendications 1 à 4 **caractérisé en ce qu'**il comprend les étapes suivantes de :

   a) formation d'une goutte liquide multi-composante comprenant :

      • un coeur liquide,
      • une enveloppe intermédiaire liquide formée d'une composition aqueuse comprenant au moins un biopolymère, encapsulant totalement à sa périphérie le coeur liquide, et
      • une enveloppe externe liquide formée d'une composition aqueuse, différente de la composition intermédiaire, ladite composition aqueuse comprenant au moins un polyélectrolyte et au moins un tensio-actif,

ladite enveloppe externe liquide encapsulant totalement à sa périphérie l'enveloppe intermédiaire,

le coeur liquide et/ou l'enveloppe intermédiaire liquide comprenant au moins une cellule eucaryote de mammifère
b) gélification par immersion de ladite goutte liquide multi-composante dans une solution gélifiante contenant un réactif propre à gélifier le polyélectrolyte de l'enveloppe externe liquide, pour obtenir une capsule gélifiée comprenant une enveloppe externe gélifiée,
c) rigidification de la composition intermédiaire de l'enveloppe intermédiaire liquide, pour obtenir une capsule gélifiée et rigidifiée comprenant une enveloppe intermédiaire rigidifiée, et
d) récupération desdites capsules gélifiée et rigidifiée.

**8.** Procédé selon la revendication 7 **caractérisé en ce que** le procédé comprend en outre, une étape de dissolution de l'enveloppe externe gélifiée.

**9.** Méthode de criblage de principes actifs comprenant :

a) la mise en culture d'une capsule selon l'une quelconque des revendications 1 à 6 en présence et en absence d'une substance candidate,
b) la détection d'un phénotype d'intérêt dans les cellules de la capsule cultivée en présence de la substance candidate par rapport aux cellules de la capsule cultivée en absence de la substance candidate, et
c) l'identification de la substance en tant que principe actif si un phénotype d'intérêt a été détecté.

**10.** Méthode de criblage selon la revendication 9 **caractérisée en ce que** le principe actif est un principe actif cosmétique.

**11.** Utilisation d'une capsule selon l'une quelconque des revendications 1 à 6 pour la culture *in vitro* de cellules eucaryotes de mammifère.

**12.** Utilisation selon la revendication 11 **caractérisée en ce que** lesdites cellules eucaryotes de mammifère sont des cellules humaines.

**13.** Procédé *in vitro* de culture de cellules eucaryotes de mammifère comprenant les étapes suivantes de :

a) mise en culture d'une capsule selon l'une quelconque des revendications 1 à 6 dans des conditions suffisantes pour la croissance cellulaire, et
b) récolte de ladite capsule.

**14.** Procédé selon la revendication 13 **caractérisé en ce qu'**il comprend préalablement à l'étape a), la préparation d'une capsule par le procédé selon l'une ou l'autre des revendications 7 et 8.

**15.** Procédé selon l'une ou l'autre des revendications 13 et 14 **caractérisé en ce que** l'enveloppe intermédiaire et le coeur liquide comprennent au moins une cellule eucaryote de mammifère.

## Patentansprüche

**1.** Kapsel, aufweisend einen flüssigen Kern, mindestens eine äußere Hülle, die den flüssigen Kern an seinem Umfang vollständig einkapselt, wobei die äußere Hülle geeignet ist, den flüssigen Kern zu halten, wenn die Kapsel in ein Gas getaucht ist, und mindestens ein geliertes Polyelektrolyt und /oder ein versteiftes Biopolymer aufweist, und eine steife Zwischenhülle, die mindestens ein Biopolymer aufweist, wobei sich die Zwischenhülle zwischen dem flüssigen Kern und der äußeren Hülle befindet, wobei die Kapsel **dadurch gekennzeichnet ist, dass** sie mindestens eine eukaryotische Säugetierzelle aufweist.

**2.** Kapsel gemäß Anspruch 1, **dadurch gekennzeichnet, dass** der flüssige Kern und/oder die steife Zwischenhülle mindestens eine eukaryotische Säugetierzelle aufweist.

**3.** Kapsel gemäß irgendeinem der Ansprüche 1 oder 2, **dadurch gekennzeichnet, dass** das Biopolymer der Zwischenhülle aus Proteinen der extrazellulären Matrix, Proteoglykanen, Glykosaminoglykanen, Polysacchariden und ihrer nichthydrolisierten oder teilhydrolisierten Form ausgewählt ist.

4. Kapsel gemäß irgendeinem der Ansprüche 1 bis 3, **dadurch gekennzeichnet, dass** der flüssige Kern und/oder die Zwischenhülle unabhängig voneinander eine eukaryotische Säugetierzelle aufweisen, die aus Fibroblasten, Melanozyten, Keratinozyten und ihren Mischungen ausgewählt ist.

5. Kapsel gemäß irgendeinem der Ansprüche 1 bis 4, **dadurch gekennzeichnet, dass** sie durch das Durchführen eines Verfahrens erhalten wird, welches die folgenden Schritte aufweist:

a) Ausbilden eines flüssigen Mehrkomponenten-Tropfens, aufweisend:

• einen flüssigen Kern,
• eine flüssige Zwischenhülle, die aus einer wässrigen Zusammensetzung gebildet ist, die mindestens ein Biopolymer aufweist, die den flüssigen Kern an seinem Umfang vollständig eingekapselt, und
• eine flüssige äußere Hülle, die aus einer wässrigen Zusammensetzung gebildet ist, die sich von der Zwischenzusammensetzung unterscheidet, wobei die wässrige Zusammensetzung mindestens einen Polyelektrolyten und mindestens ein Tensid aufweist, wobei die flüssige äußere Hülle an ihrem Umfang die Zwischenhülle vollständig eingekapselt,

wobei der flüssige Kern und/oder die flüssige Zwischenhülle mindestens eine eukaryotische Säugetierzelle aufweist,
b) Gelieren durch Eintauchen des flüssigen Mehrkomponenten-Tropfens in eine Gelierlösung, die ein Reagens enthält, das in der Lage ist, den Polyelektrolyten der flüssigen äußeren Hülle zu gelieren, um eine gelierte Kapsel zu erhalten, die eine gelierte äußere Hülle aufweist,
c) Versteifen der Zwischenzusammensetzung der flüssigen Zwischenhülle, um eine gelierte und versteifte Kapsel zu erhalten, die eine versteifte Zwischenhülle aufweist, und
d) Wiederherstellen der gelierten und versteiften Kapseln.

6. Kapsel gemäß Anspruch 5, **dadurch gekennzeichnet, dass** das Verfahren ferner einen Schritt des Auflösens der gelierten äußeren Hülle aufweist.

7. Verfahren zum Herstellen einer Kapsel gemäß irgendeinem der Ansprüche 1 bis 4, **dadurch gekennzeichnet, dass** es die folgenden Schritte aufweist:

a) Ausbilden eines flüssigen Mehrkomponenten-Tropfens, aufweisend:

• einen flüssigen Kern,
• eine flüssige Zwischenhülle, die aus einer wässrigen Zusammensetzung gebildet ist, die mindestens ein Biopolymer aufweist, die den flüssigen Kern an seinem Umfang vollständig eingekapselt, und
• eine flüssige äußere Hülle, die aus einer wässrigen Zusammensetzung gebildet ist, die sich von der Zwischenzusammensetzung unterscheidet, wobei die wässrige Zusammensetzung mindestens einen Polyelektrolyten und mindestens ein Tensid aufweist, wobei die flüssige äußere Hülle an ihrem Umfang die Zwischenhülle vollständig eingekapselt,

wobei der flüssige Kern und/oder die flüssige Zwischenhülle mindestens eine eukaryotische Säugetierzelle aufweist,
b) Gelieren durch Eintauchen des flüssigen Mehrkomponenten-Tropfens in eine Gelierlösung, die ein Reagens enthält, das in der Lage ist, den Polyelektrolyten der flüssigen äußeren Hülle zu gelieren, um eine gelierte Kapsel zu erhalten, die eine gelierte äußere Hülle aufweist,
c) Versteifen der Zwischenzusammensetzung der flüssigen Zwischenhülle, um eine gelierte und versteifte Kapsel zu erhalten, die eine versteifte Zwischenhülle aufweist, und
d) Wiederherstellen der gelierten und versteiften Kapseln.

8. Verfahren gemäß Anspruch 7, **dadurch gekennzeichnet, dass** das Verfahren ferner einen Schritt des Auflösens der gelierten äußeren Hülle aufweist.

9. Verfahren zum Aussieben von aktiven Wirkstoffen, aufweisend:

a) Kultivieren einer Kapsel gemäß irgendeinem der Ansprüche 1 bis 6 in Anwesenheit und in Abwesenheit einer Kandidatensubstanz,

b) Detektieren eines Phänotyps von Interesse in den Zellen der in Anwesenheit der Kandidatensubstanz kultivierten Kapsel im Vergleich zu den Zellen der in Abwesenheit der Kandidatensubstanz kultivierten Kapsel, und
c) Identifizieren der Substanz als aktiven Wirkstoff, wenn ein Phänotyp von Interesse detektiert wurde.

**10.** Verfahren zum Aussieben gemäß Anspruch 9, **dadurch gekennzeichnet, dass** der aktive Wirkstoff ein kosmetischer aktiver Wirkstoff ist.

**11.** Verwendung einer Kapsel gemäß irgendeinem der Ansprüche 1 bis 6 für die *in vitro* -Kultur von eukaryotischen Säugetierzellen.

**12.** Verwendung gemäß Anspruch 11, **dadurch gekennzeichnet, dass** die eukaryotischen Säugetierzellen menschliche Zellen sind.

**13.** *In vitro* -Verfahren zum Kultivieren von eukaryotischen Säugetierzellen, aufweisend die folgenden Schritte:

a) Kultivieren einer Kapsel gemäß irgendeinem der Ansprüche 1 bis 6 unter Bedingungen, die für das Zellwachstum ausreichend sind, und
b) Ernten der Kapsel.

**14.** Verfahren gemäß Anspruch 13, **dadurch gekennzeichnet, dass** es vor dem Schritt a) das Herstellen einer Kapsel mittels eines Verfahrens gemäß dem einen oder dem anderen der Ansprüche 7 und 8 aufweist.

**15.** Verfahren gemäß dem einen oder dem anderen der Ansprüche 13 und 14, **dadurch gekennzeichnet, dass** die Zwischenhülle und der flüssige Kern mindestens eine eukaryotische Säugetierzelle aufweisen.

**Claims**

**1.** A capsule comprising a liquid core, at least one external envelope totally encapsulating the liquid core at its periphery, said external envelope being able to retain the liquid core when the capsule is immersed in a gas and comprising at least one gelled polyelectrolyte and/or one stiffened biopolymer, and one stiff intermediate envelope comprising at least one biopolymer, said intermediate envelope being located between the liquid core and said external envelope, said capsule being **characterised in that** it comprises at least one eukaryotic mammalian cell.

**2.** The capsule according to claim 1, **characterised in that** the liquid core and/or the stiff intermediate envelope comprises at least one eukaryotic mammalian cell.

**3.** The capsule according to any of claims 1 or 2, **characterised in that** the biopolymer of said intermediate envelope is selected from proteins of the extra-cellular matrix, proteoglycans, glycosaminoglycans, polysaccharides and their non-hydrolysed or partly hydrolysed form.

**4.** The capsule according to any of claims 1 to 3, **characterised in that** the liquid core and/or the intermediate envelope comprise independently of each other, an eukaryotic mammalian cell selected from fibroblasts, melanocytes, keratinocytes and mixtures thereof.

**5.** The capsule according to any of claims 1 to 4, **characterised in that** it is obtained by applying a method comprising the following steps:

a) forming a multi-component liquid drop comprising:

- a liquid core,
- a liquid intermediate envelope formed with an aqueous composition comprising at least one biopolymer, totally encapsulating at its periphery the liquid core, and
- a liquid external envelope formed with an aqueous composition, different from the intermediate composition, said aqueous composition comprising at least one polyelectrolyte and at least one surfactant, said liquid external envelope totally encapsulating at its periphery the intermediate envelope, the liquid core and/or the liquid intermediate envelope comprising at least one eukaryotic mammalian cell,

b) gelling by immersion of said multi-component liquid drop in a gelling solution containing a reagent capable of gelling the polyelectrolyte of the liquid external envelope, in order to obtain a gelled capsule comprising a gelled external envelope,
c) stiffening the intermediate composition of the liquid intermediate envelope, in order to obtain a gelled and stiffened capsule comprising a stiffened intermediate envelope, and
d) recovering said gelled and stiffened capsules.

6. The capsule according to claim 5, **characterised in that** the method further comprises a step for dissolving the gelled external envelope.

7. A method for preparing a capsule according to any of claims 1 to 4, **characterised in that** it comprises the following steps:

a) forming a multi-component liquid drop comprising:

• a liquid core,
• a liquid intermediate envelope formed with an aqueous composition comprising at least one biopolymer, totally encapsulating at its periphery the liquid core, and
• a liquid external envelope formed with an aqueous composition, different from the intermediate composition, said aqueous composition comprising at least one polyelectrolyte and at least one surfactant, said liquid external envelope totally encapsulating at its periphery the intermediate envelope, the liquid core and/or the liquid intermediate envelope comprising at least one eukaryotic mammalian cell,

b) gelling by immersion of said multi-component liquid drop in a gelling solution containing a reagent capable of gelling the polyelectrolyte of the liquid external envelope, in order to obtain a gelled capsule comprising a gelled external envelope,
c) stiffening the intermediate composition of the liquid intermediate envelope, in order to obtain a gelled and stiffened capsule comprising a stiffened intermediate envelope, and
d) recovering said gelled and stiffened capsules.

8. The method according to claim 7, **characterised in that** the method further comprises a step for dissolving the gelled external envelope.

9. A method for screening active ingredients comprising:

a) cultivating a capsule according to any of claims 1 to 6 in the presence and in the absence of a candidate substance,
b) detecting a phenotype of interest in the cells of the capsule cultivated in the presence of the candidate substance as compared with the cells of the capsule cultivated in the absence of the candidate substance, and
c) identifying the substance as an active ingredient if a phenotype of interest has been detected.

10. The method according to claim 9, **characterised in that** the active ingredient is a cosmetic active ingredient.

11. The use of a capsule according to any of claims 1 to 6 for *in vitro* cultivation of eukaryotic mammalian cells.

12. The use according to claim 11, **characterised in that** said eukaryotic mammalian cells are human cells.

13. An *in vitro* method for cultivating eukaryotic mammalian cells comprising the following steps:

a) cultivating a capsule according to any of claims 1 to 6 under sufficient conditions for cell growth, and
b) harvesting said capsule.

14. The method according to claim 13, **characterised in that** it comprises prior to step a), the preparation of a capsule by the method according to either one of claims 7 and 8.

15. The method according to either one of claims 13 and 14, **characterised in that** the intermediate envelope and the liquid core comprise at least one eukaryotic mammalian cell.

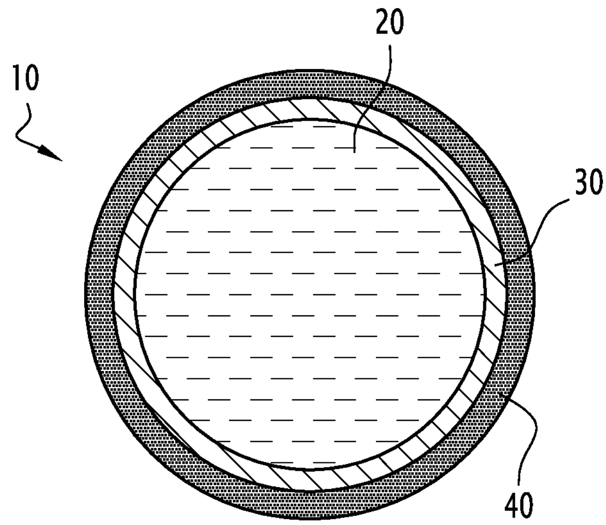


FIG.1

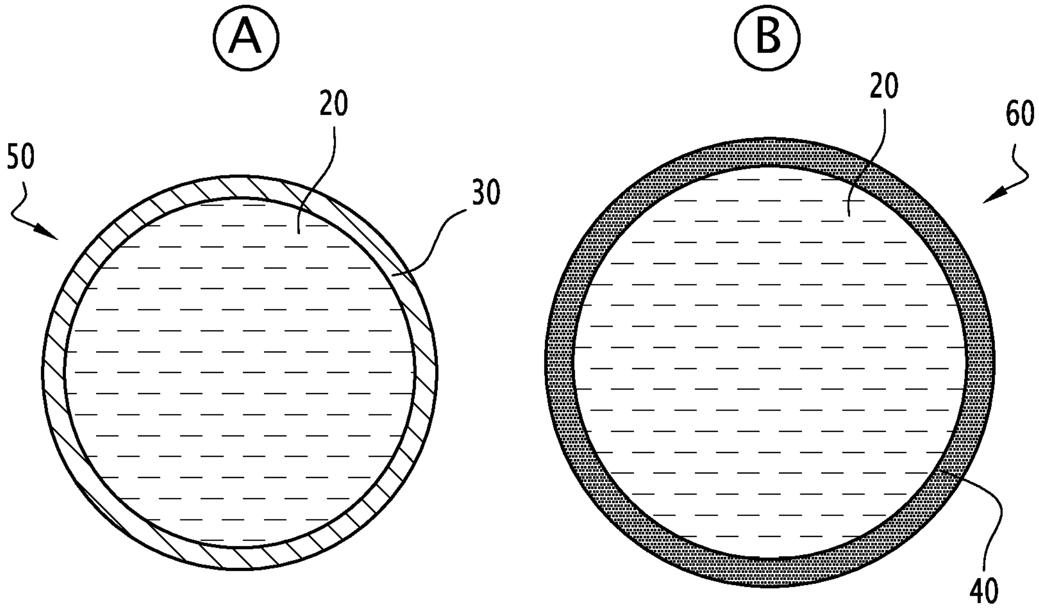


FIG.2

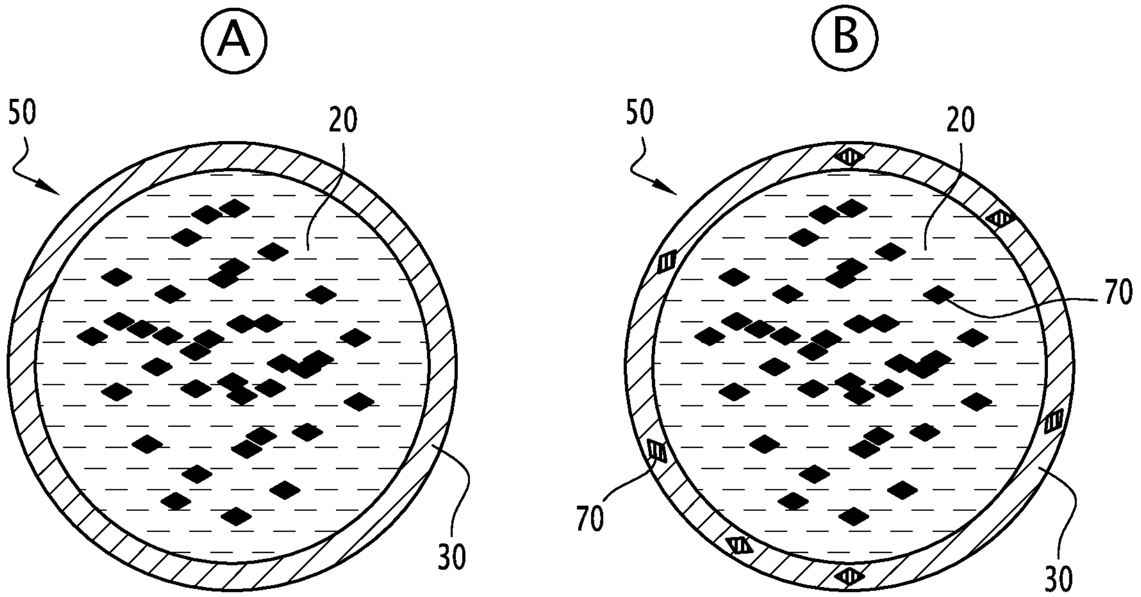


FIG.3

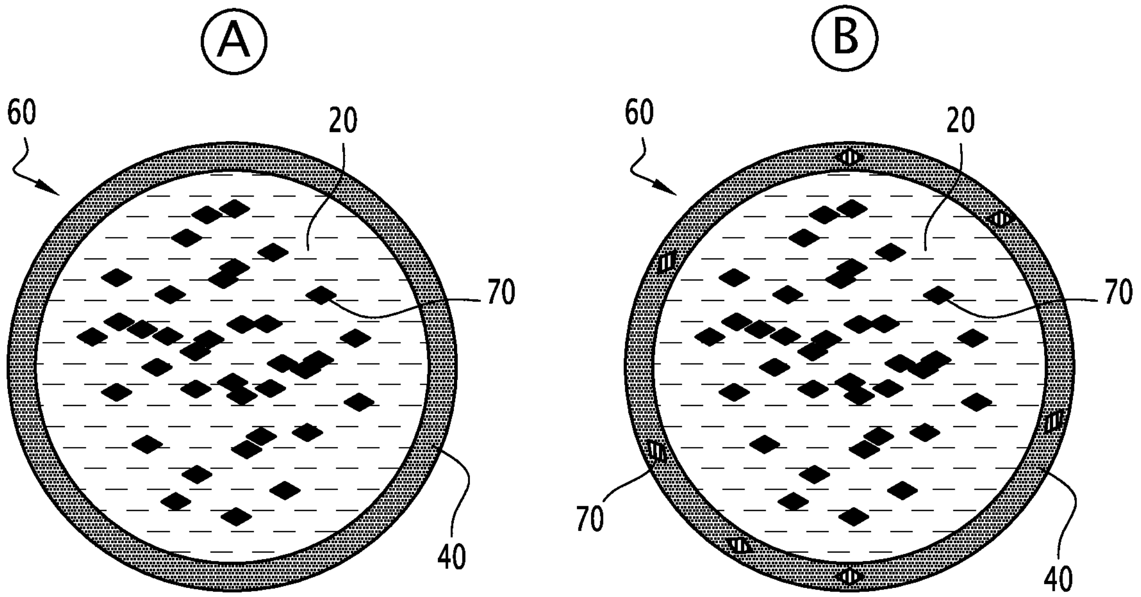


FIG.4

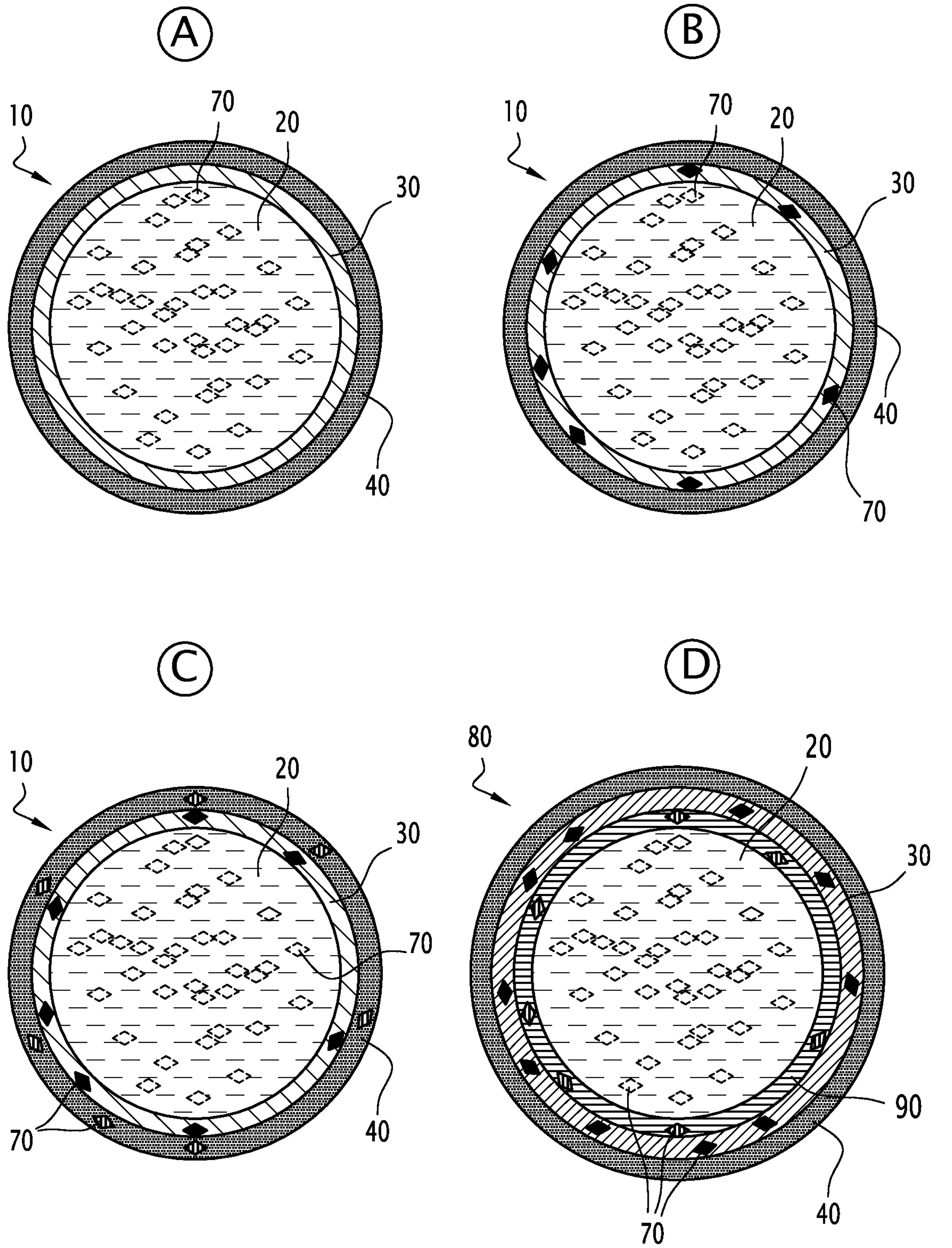


FIG.5

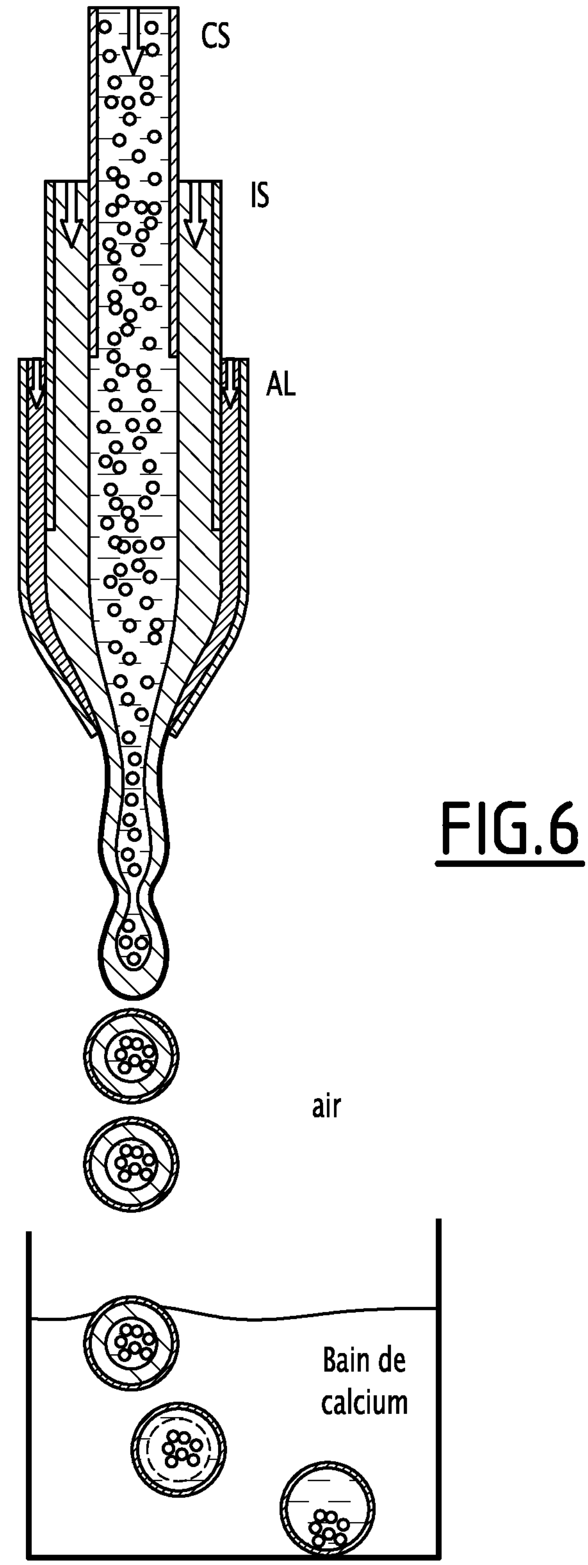


FIG.6

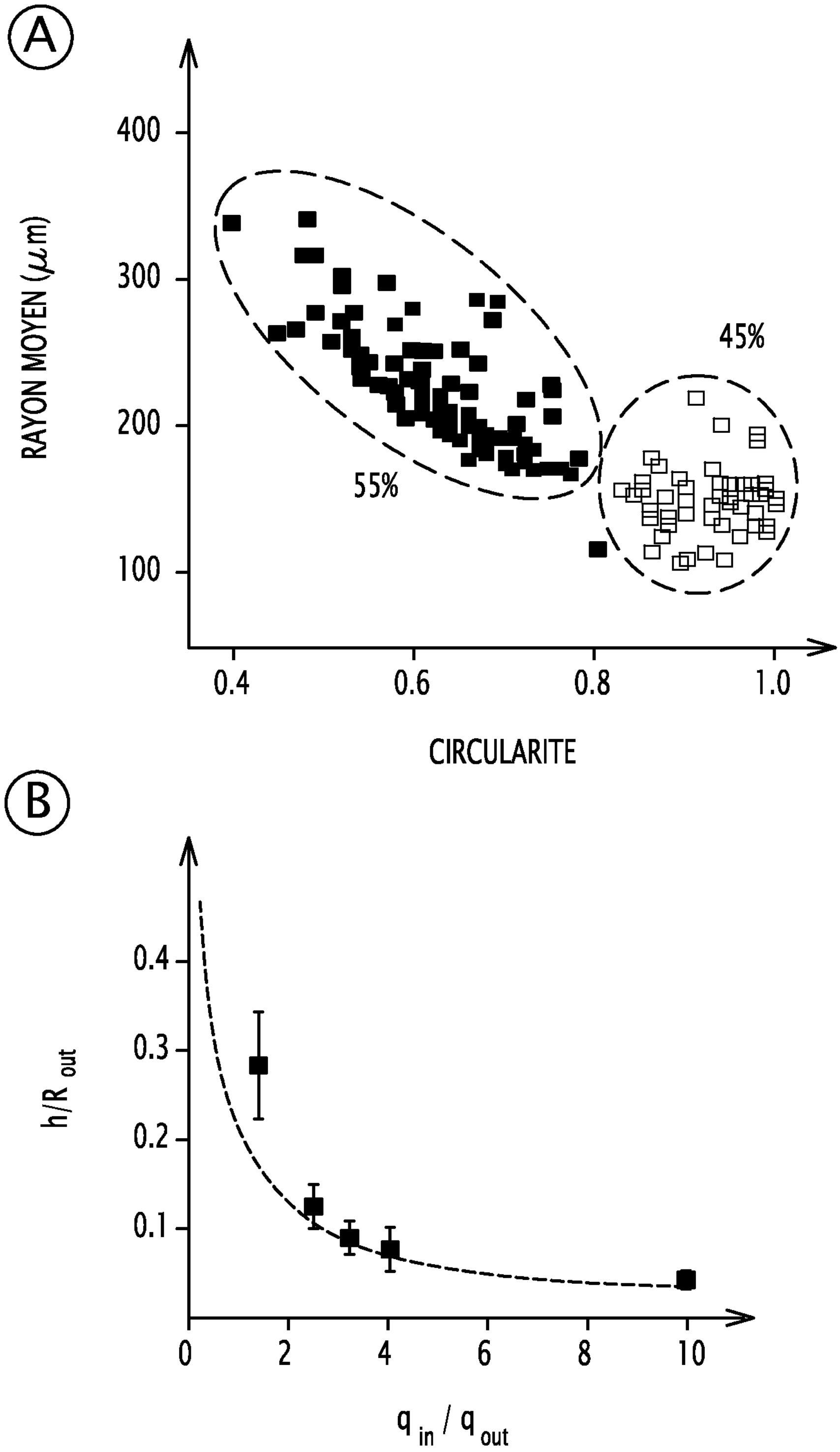
A
400
300
200
100
RAYON MOYEN (μm)
45%
55%
0.4
0.6
0.8
1.0
CIRCULARITE
B
0.4
0.3
0.2
0.1
h/R out
0
2
4
6
8
10
q in / q out

FIG.7

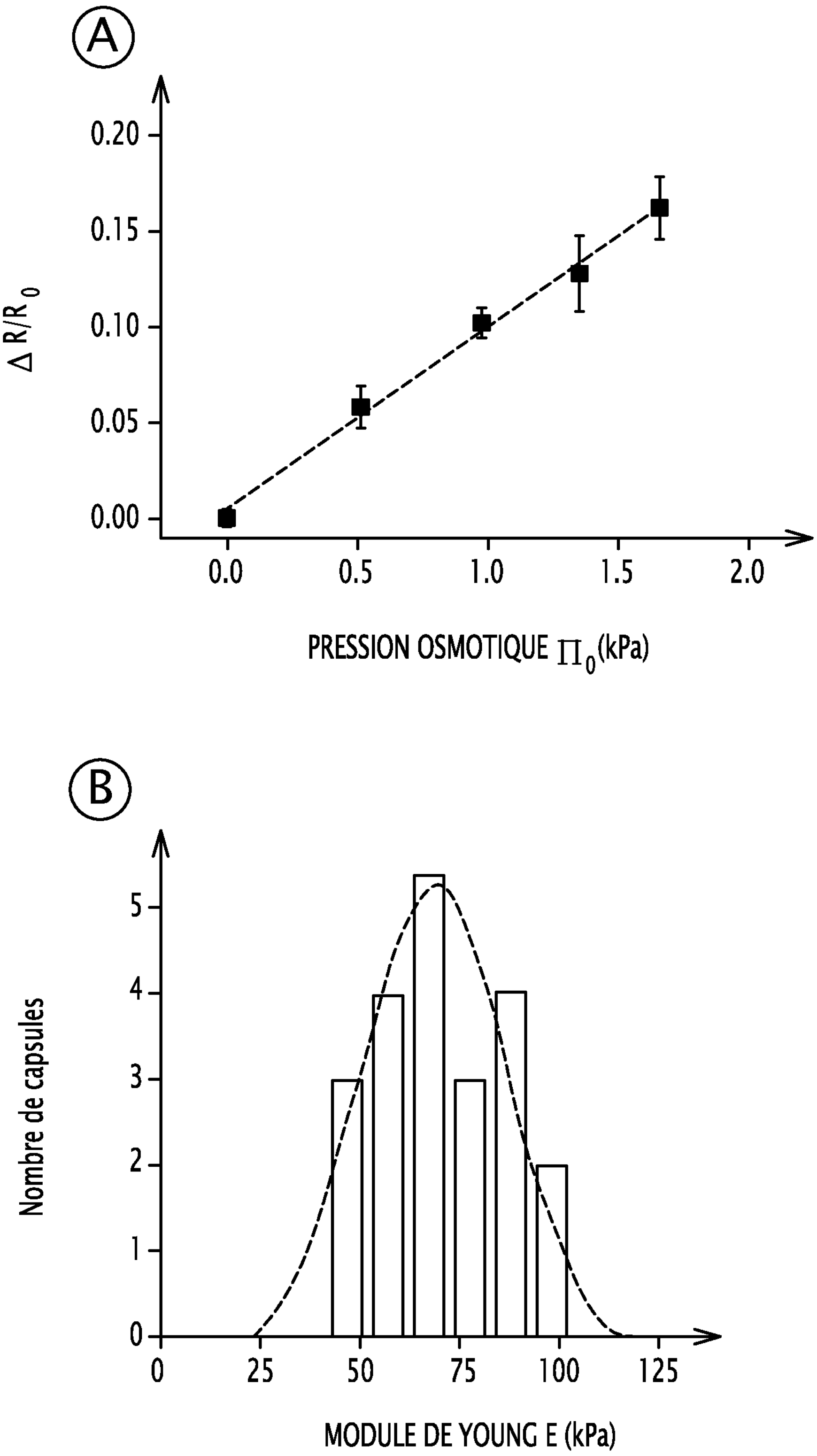
A
0.20
0.15
0.10
0.05
0.00
Δ R/R0
0.0
0.5
1.0
1.5
2.0
PRESSION OSMOTIQUE Π0(kPa)
B
5
4
3
2
1
0
Nombre de capsules
0
25
50
75
100
125
MODULE DE YOUNG E (kPa)

FIG.8

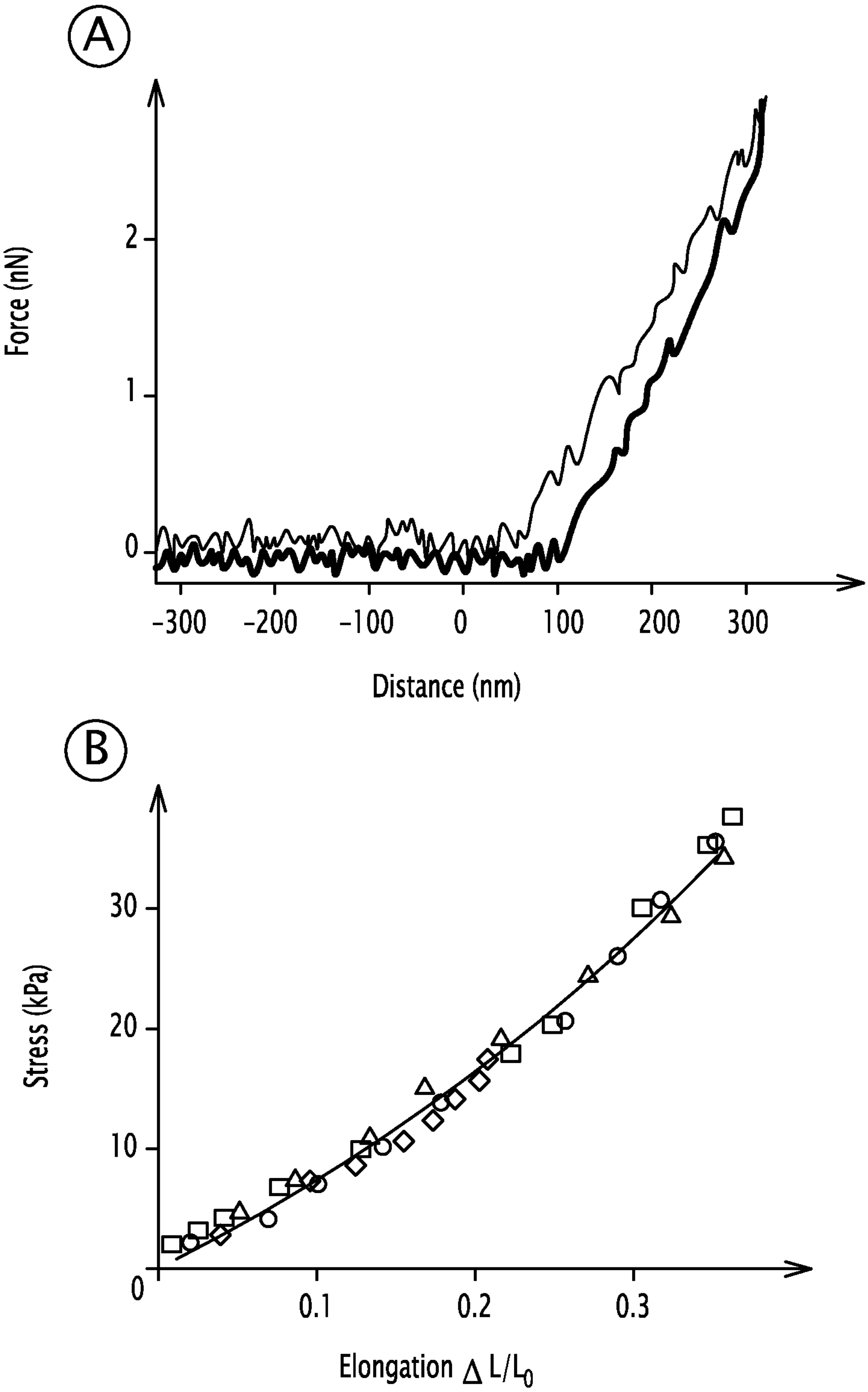
A
Force (nN)
2
1
0
-300
-200
-100
0
100
200
300
Distance (nm)
B
Stress (kPa)
30
20
10
0
0.1
0.2
0.3
Elongation Δ L/L0

FIG.9

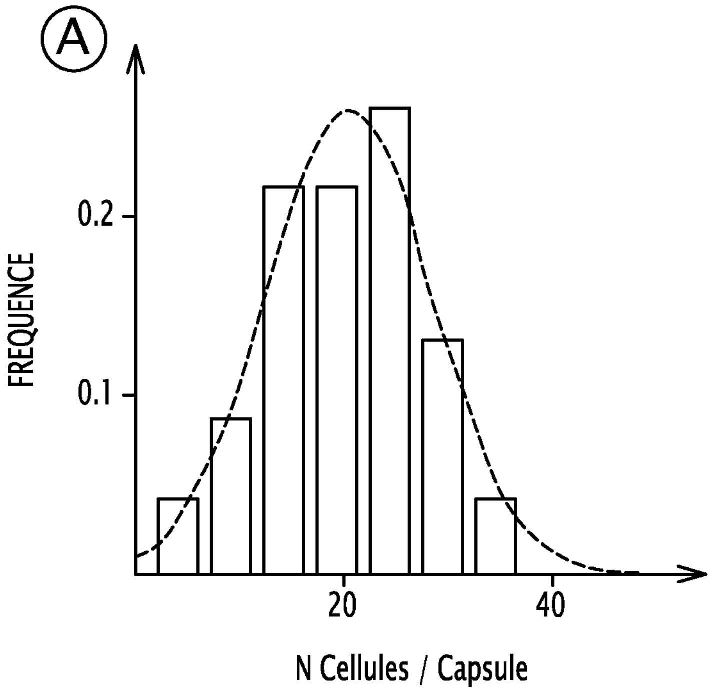
A
FREQUENCE
0.2
0.1
20
40
N Cellules / Capsule

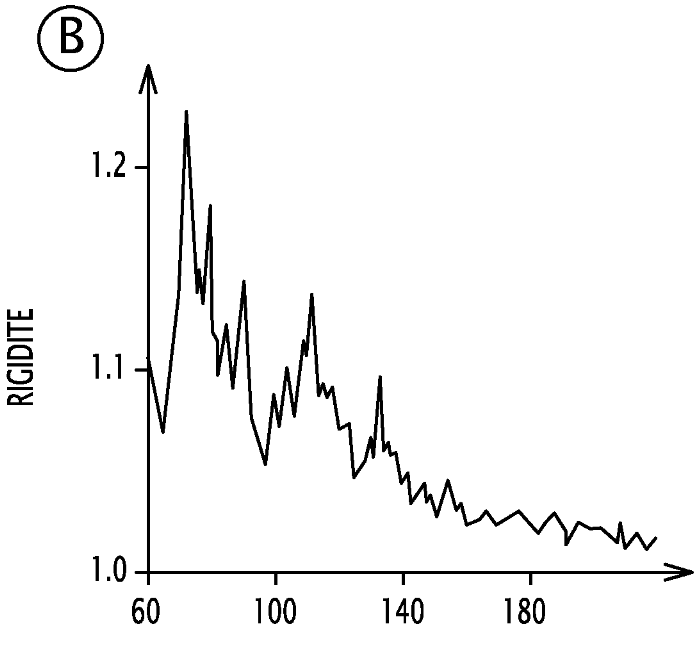
B
RIGIDITE
1.2
1.1
1.0
60
100
140
180
TEMPS (HEURES)

FIG.10

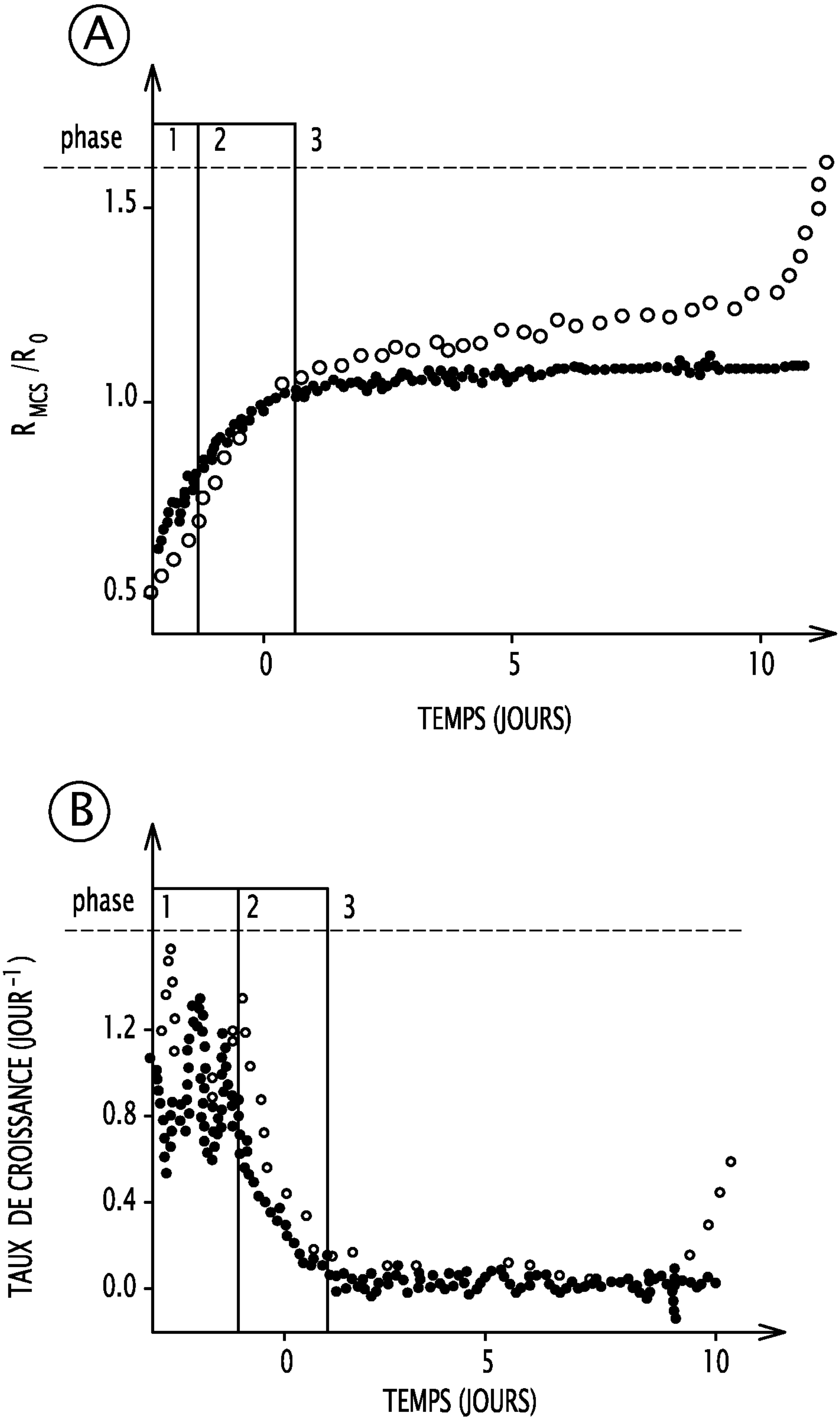
A
phase
1
2
3
1.5
1.0
0.5
$R_{MCS}$ /$R_0$
0
5
10
TEMPS (JOURS)
B
phase
1
2
3
1.2
0.8
0.4
0.0
TAUX DE CROISSANCE (JOUR$^{-1}$)
0
5
10
TEMPS (JOURS)

FIG.11

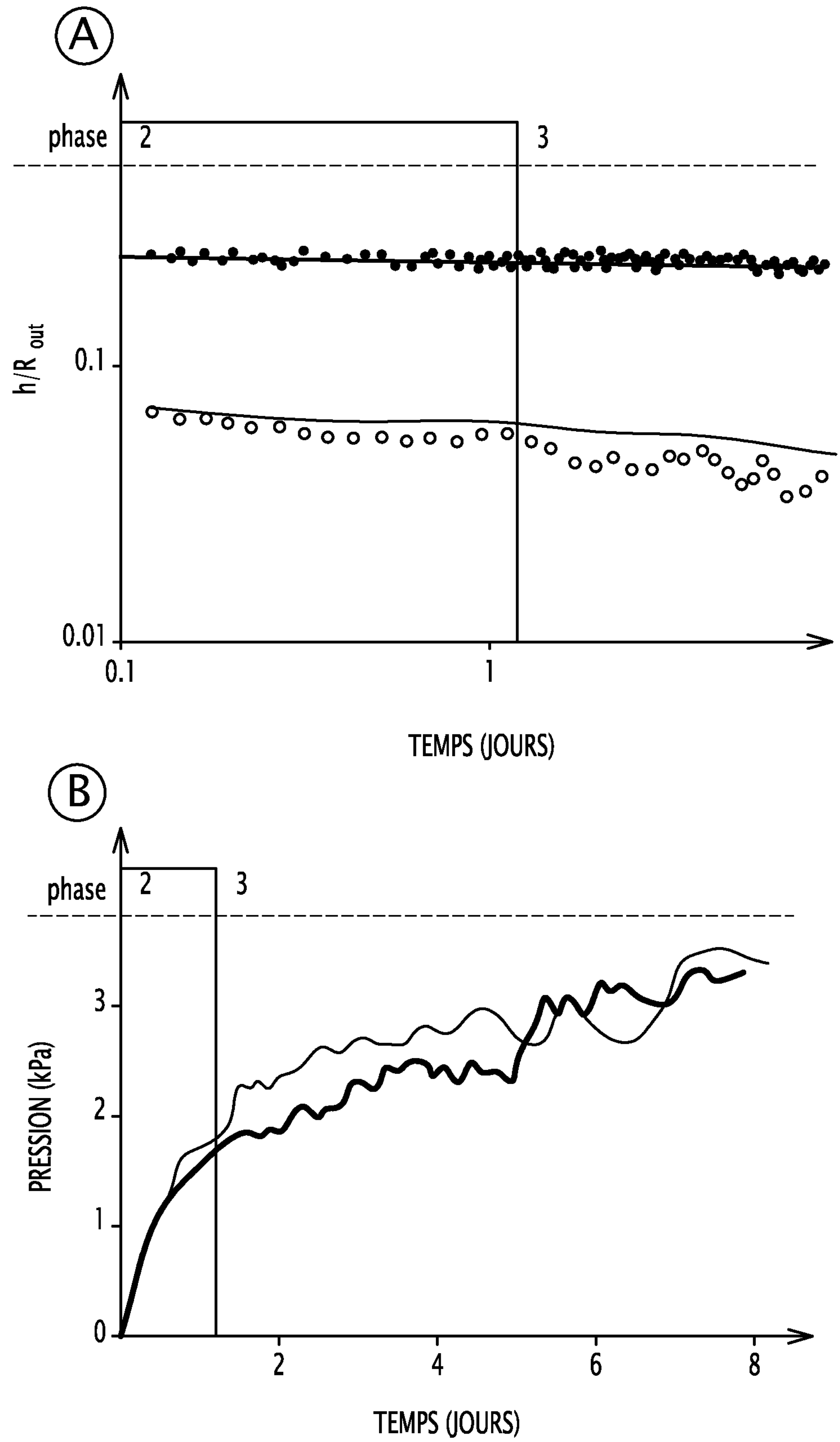
A
phase
2
3
h/R out
0.1
0.01
0.1
1
TEMPS (JOURS)
B
phase
2
3
PRESSION (kPa)
3
2
1
0
2
4
6
8
TEMPS (JOURS)

FIG.12

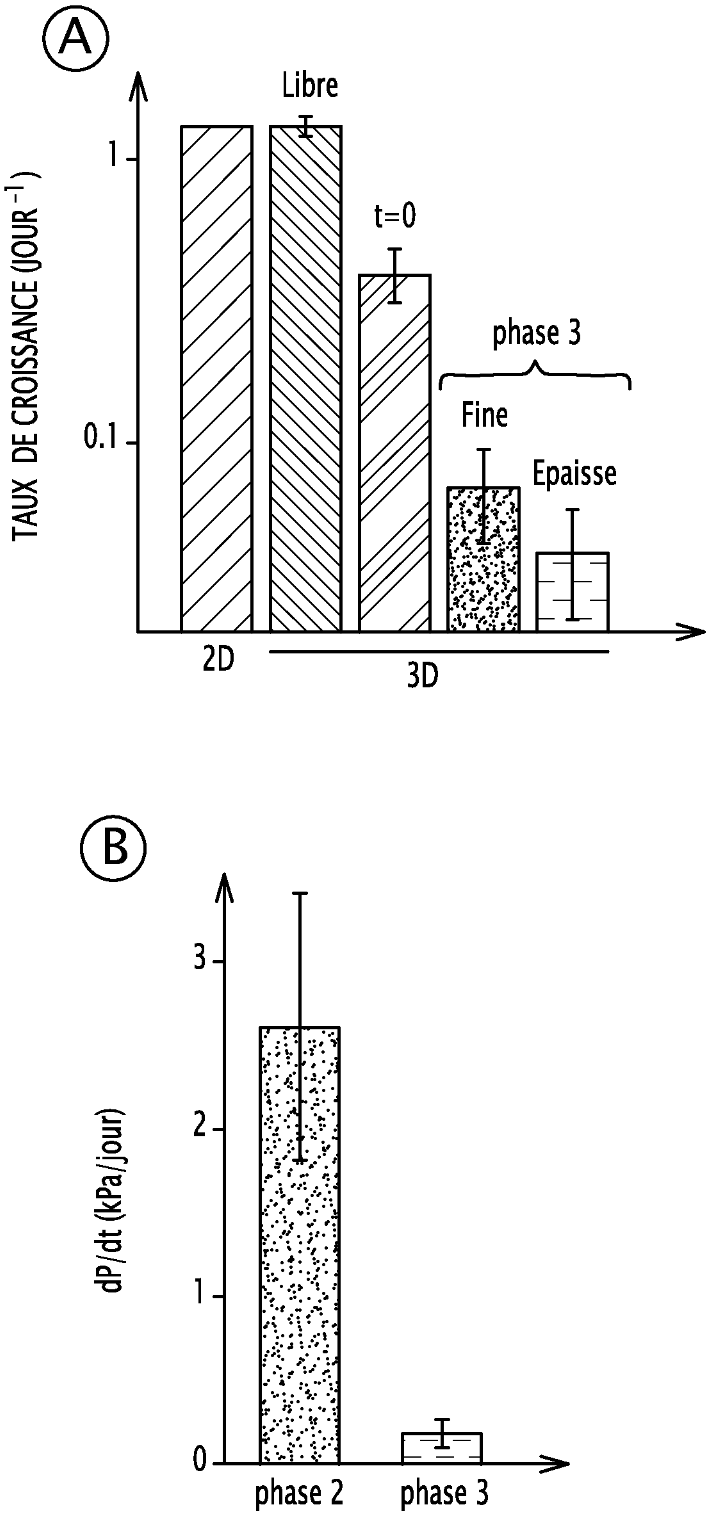
A
TAUX DE CROISSANCE (JOUR⁻¹)
1
0.1
Libre
t=0
phase 3
Fine
Epaisse
2D
3D
B
dP/dt (kPa/jour)
3
2
1
0
phase 2
phase 3

FIG.13

FIG.14

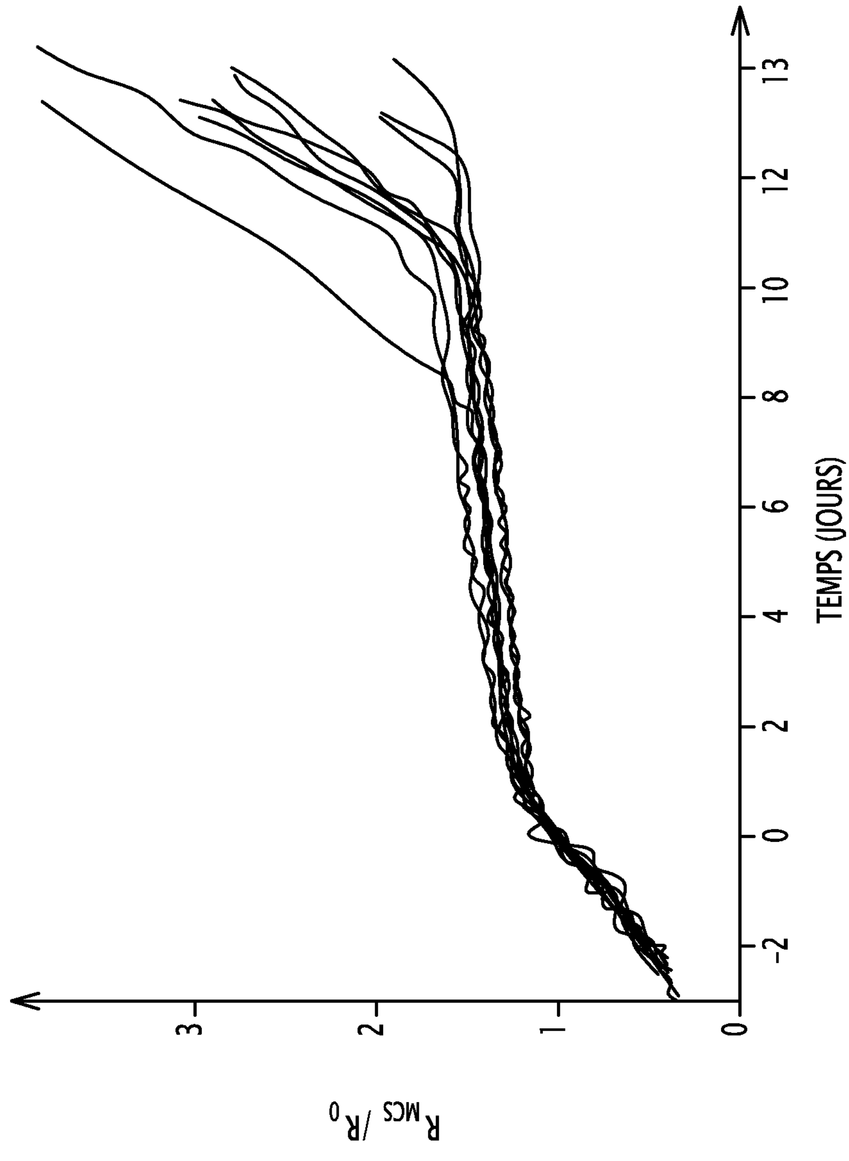

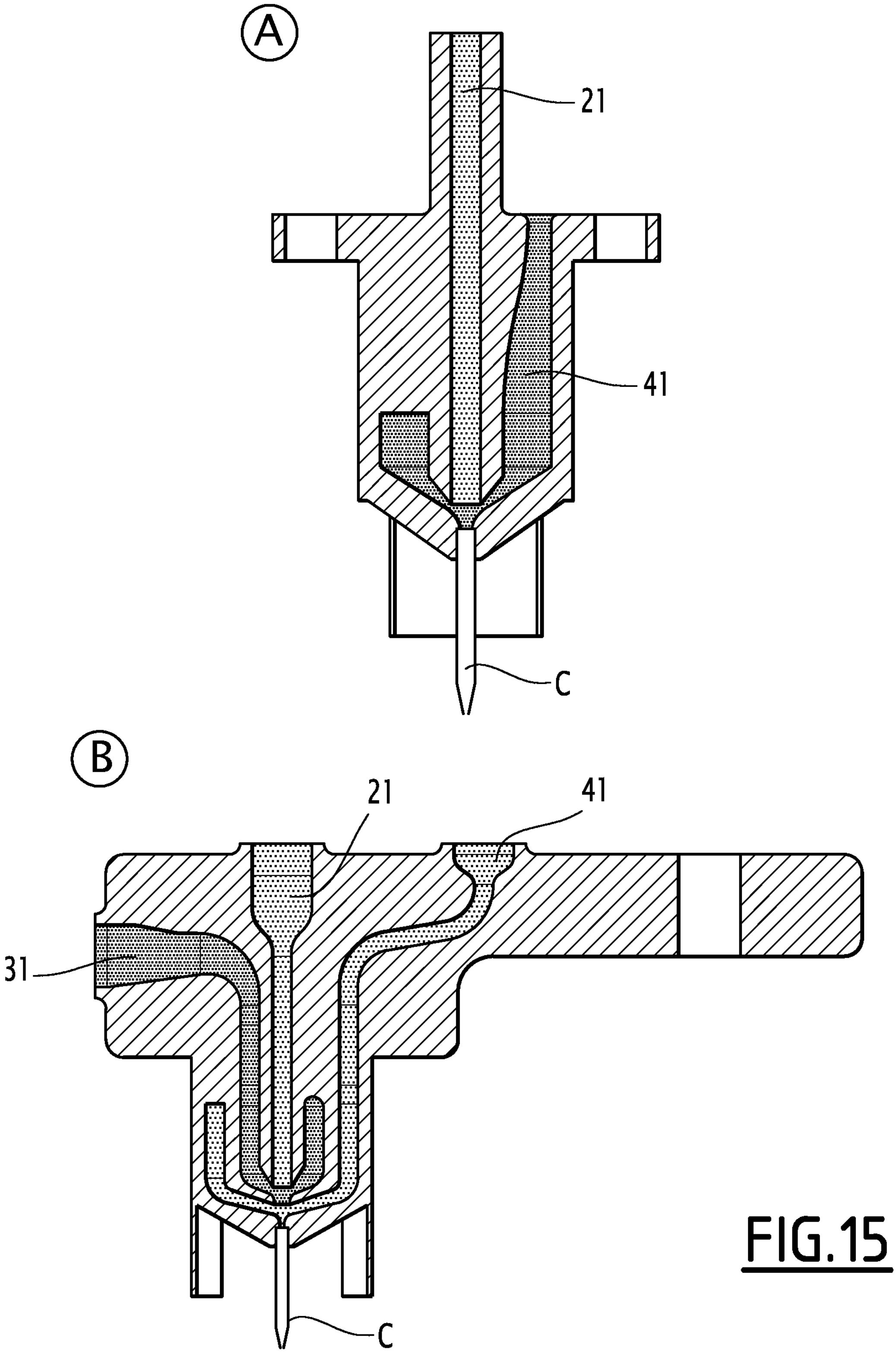


FIG.15

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2939012
- US 4409331 A
- FR 1061404
- WO 2010063937 A
- FR 1056925


### Littérature non-brevet citée dans la description


- **PERRET, E. et al.** *Langmuir,* 2002, vol. 18, 846, , 854
- **HIRSCHHAEUSER, F. et al.** *J. Biotechnol.,* 2010, vol. 148, 3, , 15
- **SCHNEIDER, C. A. et al.** *Nat. Methods,* 2012, vol. 9, 671, , 675
- **SCHINDELIN, J. et al.** *Nature Methods,* 2012, vol. 9, 676, , 682
- **MARMOTTANT, P. et al.** *Proc. Natl. Acad. Sci. U.S.A.,* 2009, vol. 106, 17271, , 17275
- **FERY, A. ; WEINKAMER, R.** *Polymer,* 2007, vol. 48, 7221, , 7235
- **LANDAU, L. D. et al.** Theory of Elasticity. Butterworth-Heinemann, 1986, vol. 7
- **REID, C. ; RAND, R. P.** *Biophys J,* 1997, vol. 73, 1692-1694
- **VERETOUT, F.** *Journal of molecular biology,* vol. 205, 713-728
- **BONNET-GONNET, C. et al.** *Langmuir,* 1994, vol. 10, 4012-4021
- **ZHANG, J. et al.** *Journal of Food Engineering,* 2007, vol. 80, 157-165
- **FUNG, Y. C.** Foundations of Solid Mechanics. Prentice Hall, 1965
- **DYM, C. L. ; WILLIAMS, H. E.** *International Journal of Mechanical Engineering Education,* 2007, vol. 35, 108-113